(19) Europäisches Patentamt
European Patent Office
Office européen des brevets

(11) EP 1 772 052 A1

(12) # EUROPÄISCHE PATENTANMELDUNG

(43) Veröffentlichungstag:
**11.04.2007 Patentblatt 2007/15**

(21) Anmeldenummer: **05090277.4**

(22) Anmeldetag: **05.10.2005**

(51) Int Cl.:
***A01H 5/00*** *(2006.01)* ***A01H 5/08*** *(2006.01)*
***C12N 5/14*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(71) Anmelder: **Bayer CropScience GmbH**
**65929 Frankfurt/Main (DE)**

(72) Erfinder:
- **Frohberg, Claus**
  **14532 Kleinmachnow (DE)**
- **Essigmann, Bernd**
  **12157 Berlin (DE)**

(74) Vertreter: **Kossmann, Jochen**
**Bayer BioScience GmbH**
**Hermannswerder 20a**
**14473 Potsdam (DE)**

(54) **Verbesserte Verfahren und Mittel zur Herstellung von Hyaluronan**

(57) Die vorliegende Erfindung betrifft Pflanzenzellen und Pflanzen, die eine erhöhte Menge an Hyaluronan synthetisieren, sowie Verfahren zur Herstellung solcher Pflanzen, als auch Verfahren zur Herstellung von Hyaluronan mit Hilfe dieser Pflanzenzellen oder Pflanzen. Erfindungsgemäße Pflanzenzellen oder genetisch modifizierte Pflanzen weisen dabei die Aktivität einer Hyaluronansynthase und zusätzlich eine erhöhte Aktivität einer Glutamin:Fructose-6-Phosphat Amidotransferase (GFAT) und eine erhöhte Aktivität einer UDP-Glucose Dehydrogenase (UDP-Glc-DH) im Vergleich zu Wildtyp Pflanzenzellen bzw. Wildtyp Pflanzen auf. Weiterhin betrifft die vorliegende Erfindung die Verwendung von Pflanzen mit gesteigerter Hyaluronansynthese zur Herstellung von Hyaluronan und Lebens- oder Futtermitteln, die Hyaluronan enthalten.

EP 1 772 052 A1

SEQUENCE LISTING

<110> Bayer CropScience GmbH

<120> Verbesserte Verfahren und Mittel zur Herstellung von Hyaluronan

<130> BCS 05-5010 EP

<160> 13

<170> PatentIn version 3.1

<210> 1
<211> 1707
<212> DNA
<213> Paramecium bursaria Chlorella Virus 1

<220>
<221> CDS
<222> (1)..(1707)
<223>

<300>
<308> PB42580
<309> 1995-12-24
<313> (50903)..(52609)

<400> 1
```
atg ggt aaa aat ata atc ata atg gtt tcg tgg tac acc atc ata act        48
Met Gly Lys Asn Ile Ile Ile Met Val Ser Trp Tyr Thr Ile Ile Thr
1               5                   10                  15

tca aat cta atc gcg gtt gga gga gcc tct cta atc ttg gct ccg gca        96
Ser Asn Leu Ile Ala Val Gly Gly Ala Ser Leu Ile Leu Ala Pro Ala
            20                  25                  30

att act ggg tat gtt cta cat tgg aat att gct ctc tcg aca atc tgg       144
Ile Thr Gly Tyr Val Leu His Trp Asn Ile Ala Leu Ser Thr Ile Trp
        35                  40                  45

gga gta tca gct tat ggt att ttc gtt ttt ggg ttt ttc ctt gca caa       192
Gly Val Ser Ala Tyr Gly Ile Phe Val Phe Gly Phe Phe Leu Ala Gln
```

```
        50                  55                  60

gtt tta ttt tca gaa ctg aac agg aaa cgt ctt cgc aag tgg att tct      240
Val Leu Phe Ser Glu Leu Asn Arg Lys Arg Leu Arg Lys Trp Ile Ser
65                  70                  75                  80

ctc aga cct aag ggt tgg aat gat gtt cgt ttg gct gtg atc att gct      288
Leu Arg Pro Lys Gly Trp Asn Asp Val Arg Leu Ala Val Ile Ile Ala
                85                  90                  95

gga tat cgc gag gat cct tat atg ttc cag aag tgc ctc gag tct gta      336
Gly Tyr Arg Glu Asp Pro Tyr Met Phe Gln Lys Cys Leu Glu Ser Val
            100                 105                 110

cgt gac tct gat tat ggc aac gtt gcc cgt ctg att tgt gtg att gac      384
Arg Asp Ser Asp Tyr Gly Asn Val Ala Arg Leu Ile Cys Val Ile Asp
        115                 120                 125

ggt gat gag gac gat gat atg agg atg gct gcc gtt tac aag gcg atc      432
Gly Asp Glu Asp Asp Asp Met Arg Met Ala Ala Val Tyr Lys Ala Ile
    130                 135                 140

tac aat gat aat atc aag aag ccc gag ttt gtt ctg tgt gag tca gac      480
Tyr Asn Asp Asn Ile Lys Lys Pro Glu Phe Val Leu Cys Glu Ser Asp
145                 150                 155                 160

gac aag gaa ggt gaa cgc atc gac tct gat ttc tct cgc gac att tgt      528
Asp Lys Glu Gly Glu Arg Ile Asp Ser Asp Phe Ser Arg Asp Ile Cys
                165                 170                 175

gtc ctc cag cct cat cgt gga aaa cgg gag tgt ctt tat act ggg ttt      576
Val Leu Gln Pro His Arg Gly Lys Arg Glu Cys Leu Tyr Thr Gly Phe
            180                 185                 190

caa ctt gca aag atg gac ccc agt gtc aat gct gtc gtt ctg att gac      624
Gln Leu Ala Lys Met Asp Pro Ser Val Asn Ala Val Val Leu Ile Asp
        195                 200                 205

agc gat acc gtt ctc gag aag gat gct att ctg gaa gtt gta tac cca      672
Ser Asp Thr Val Leu Glu Lys Asp Ala Ile Leu Glu Val Val Tyr Pro
    210                 215                 220

ctt gca tgc gat ccc gag atc caa gcc gtt gca ggt gag tgt aag att      720
Leu Ala Cys Asp Pro Glu Ile Gln Ala Val Ala Gly Glu Cys Lys Ile
225                 230                 235                 240

tgg aac aca gac act ctt ttg agt ctt ctc gtc gct tgg cgg tac tat      768
Trp Asn Thr Asp Thr Leu Leu Ser Leu Leu Val Ala Trp Arg Tyr Tyr
                245                 250                 255

tct gcg ttt tgt gtg gag agg agt gcc cag tct ttt ttc agg act gtt      816
Ser Ala Phe Cys Val Glu Arg Ser Ala Gln Ser Phe Phe Arg Thr Val
            260                 265                 270

cag tgc gtt ggg ggg cca ctg ggt gcc tac aag att gat atc att aag      864
Gln Cys Val Gly Gly Pro Leu Gly Ala Tyr Lys Ile Asp Ile Ile Lys
        275                 280                 285

gag att aag gac ccc tgg att tcc cag cgc ttt ctt ggt cag aag tgt      912
Glu Ile Lys Asp Pro Trp Ile Ser Gln Arg Phe Leu Gly Gln Lys Cys
    290                 295                 300

act tac ggt gac gac cgc cgg cta acc aac gag atc ttg atg cgt ggt      960
Thr Tyr Gly Asp Asp Arg Arg Leu Thr Asn Glu Ile Leu Met Arg Gly
305                 310                 315                 320

aaa aag gtt gtg ttc act cca ttt gct gtt ggt tgg tct gac agt ccg     1008
Lys Lys Val Val Phe Thr Pro Phe Ala Val Gly Trp Ser Asp Ser Pro
                325                 330                 335
```

```
acc aat gtg ttt cgg tac atc gtt cag cag acc cgc tgg agt aag tcg      1056
Thr Asn Val Phe Arg Tyr Ile Val Gln Gln Thr Arg Trp Ser Lys Ser
            340                 345                 350

tgg tgc cgc gaa att tgg tac acc ctc ttc gcc gcg tgg aag cac ggt      1104
Trp Cys Arg Glu Ile Trp Tyr Thr Leu Phe Ala Ala Trp Lys His Gly
        355                 360                 365

ttg tct gga att tgg ctg gcc ttt gaa tgt ttg tat caa att aca tac      1152
Leu Ser Gly Ile Trp Leu Ala Phe Glu Cys Leu Tyr Gln Ile Thr Tyr
    370                 375                 380

ttc ttc ctc gtg att tac ctc ttt tct cgc cta gcc gtt gag gcc gac      1200
Phe Phe Leu Val Ile Tyr Leu Phe Ser Arg Leu Ala Val Glu Ala Asp
385                 390                 395                 400

cct cgc gcc cag aca gcc acg gtg att gtg agc acc acg gtt gca ttg      1248
Pro Arg Ala Gln Thr Ala Thr Val Ile Val Ser Thr Thr Val Ala Leu
                405                 410                 415

att aag tgt ggg tat ttt tca ttc cga gcc aag gat att cgg gcg ttt      1296
Ile Lys Cys Gly Tyr Phe Ser Phe Arg Ala Lys Asp Ile Arg Ala Phe
            420                 425                 430

tac ttt gtg ctt tat aca ttt gtt tac ttt ttc tgt atg att ccg gcc      1344
Tyr Phe Val Leu Tyr Thr Phe Val Tyr Phe Phe Cys Met Ile Pro Ala
        435                 440                 445

agg att act gca atg atg acg ctt tgg gac att ggc tgg ggt act cgc      1392
Arg Ile Thr Ala Met Met Thr Leu Trp Asp Ile Gly Trp Gly Thr Arg
    450                 455                 460

ggt gga aac gag aag cct tcc gtt ggc acc cgg gtc gct ctg tgg gca      1440
Gly Gly Asn Glu Lys Pro Ser Val Gly Thr Arg Val Ala Leu Trp Ala
465                 470                 475                 480

aag caa tat ctc att gca tat atg tgg tgg gcc gcg gtt gtt ggc gct      1488
Lys Gln Tyr Leu Ile Ala Tyr Met Trp Trp Ala Ala Val Val Gly Ala
                485                 490                 495

gga gtt tac agc atc gtc cat aac tgg atg ttc gat tgg aat tct ctt      1536
Gly Val Tyr Ser Ile Val His Asn Trp Met Phe Asp Trp Asn Ser Leu
            500                 505                 510

tct tat cgt ttt gct ttg gtt ggt att tgt tct tac att gtt ttt att      1584
Ser Tyr Arg Phe Ala Leu Val Gly Ile Cys Ser Tyr Ile Val Phe Ile
        515                 520                 525

gtt att gtg ctg gtg gtt tat ttc acc ggc aaa att acg act tgg aat      1632
Val Ile Val Leu Val Val Tyr Phe Thr Gly Lys Ile Thr Thr Trp Asn
    530                 535                 540

ttc acg aag ctt cag aag gag cta atc gag gat cgc gtt ctg tac gat      1680
Phe Thr Lys Leu Gln Lys Glu Leu Ile Glu Asp Arg Val Leu Tyr Asp
545                 550                 555                 560

gca act acc aat gct cag tct gtg tga                                  1707
Ala Thr Thr Asn Ala Gln Ser Val
                565
```

<210> 2

<211> 568

<212> PRT

<213> Paramecium bursaria Chlorella Virus 1

<400> 2

Met Gly Lys Asn Ile Ile Ile Met Val Ser Trp Tyr Thr Ile Ile Thr
1 5 10 15

Ser Asn Leu Ile Ala Val Gly Gly Ala Ser Leu Ile Leu Ala Pro Ala
20 25 30

Ile Thr Gly Tyr Val Leu His Trp Asn Ile Ala Leu Ser Thr Ile Trp
35 40 45

Gly Val Ser Ala Tyr Gly Ile Phe Val Phe Gly Phe Phe Leu Ala Gln
50 55 60

Val Leu Phe Ser Glu Leu Asn Arg Lys Arg Leu Arg Lys Trp Ile Ser
65 70 75 80

Leu Arg Pro Lys Gly Trp Asn Asp Val Arg Leu Ala Val Ile Ile Ala
85 90 95

Gly Tyr Arg Glu Asp Pro Tyr Met Phe Gln Lys Cys Leu Glu Ser Val
100 105 110

Arg Asp Ser Asp Tyr Gly Asn Val Ala Arg Leu Ile Cys Val Ile Asp
115 120 125

Gly Asp Glu Asp Asp Asp Met Arg Met Ala Ala Val Tyr Lys Ala Ile
130 135 140

Tyr Asn Asp Asn Ile Lys Lys Pro Glu Phe Val Leu Cys Glu Ser Asp
145 150 155 160

Asp Lys Glu Gly Glu Arg Ile Asp Ser Asp Phe Ser Arg Asp Ile Cys
165 170 175

Val Leu Gln Pro His Arg Gly Lys Arg Glu Cys Leu Tyr Thr Gly Phe
180 185 190

Gln Leu Ala Lys Met Asp Pro Ser Val Asn Ala Val Val Leu Ile Asp
195 200 205

Ser Asp Thr Val Leu Glu Lys Asp Ala Ile Leu Glu Val Val Tyr Pro
210 215 220

Leu Ala Cys Asp Pro Glu Ile Gln Ala Val Ala Gly Glu Cys Lys Ile
225 230 235 240

Trp Asn Thr Asp Thr Leu Leu Ser Leu Leu Val Ala Trp Arg Tyr Tyr
245 250 255

Ser Ala Phe Cys Val Glu Arg Ser Ala Gln Ser Phe Phe Arg Thr Val
260 265 270

Gln Cys Val Gly Gly Pro Leu Gly Ala Tyr Lys Ile Asp Ile Ile Lys
275 280 285

Glu Ile Lys Asp Pro Trp Ile Ser Gln Arg Phe Leu Gly Gln Lys Cys
290 295 300

Thr Tyr Gly Asp Asp Arg Arg Leu Thr Asn Glu Ile Leu Met Arg Gly
305 310 315 320

Lys Lys Val Val Phe Thr Pro Phe Ala Val Gly Trp Ser Asp Ser Pro
325 330 335

Thr Asn Val Phe Arg Tyr Ile Val Gln Gln Thr Arg Trp Ser Lys Ser
340 345 350

Trp Cys Arg Glu Ile Trp Tyr Thr Leu Phe Ala Ala Trp Lys His Gly
355 360 365

Leu Ser Gly Ile Trp Leu Ala Phe Glu Cys Leu Tyr Gln Ile Thr Tyr
370 375 380

Phe Phe Leu Val Ile Tyr Leu Phe Ser Arg Leu Ala Val Glu Ala Asp
385 390 395 400

Pro Arg Ala Gln Thr Ala Thr Val Ile Val Ser Thr Thr Val Ala Leu
405 410 415

Ile Lys Cys Gly Tyr Phe Ser Phe Arg Ala Lys Asp Ile Arg Ala Phe
420 425 430

Tyr Phe Val Leu Tyr Thr Phe Val Tyr Phe Phe Cys Met Ile Pro Ala
435 440 445

Arg Ile Thr Ala Met Met Thr Leu Trp Asp Ile Gly Trp Gly Thr Arg
450 455 460

Gly Gly Asn Glu Lys Pro Ser Val Gly Thr Arg Val Ala Leu Trp Ala
465 470 475 480

Lys Gln Tyr Leu Ile Ala Tyr Met Trp Trp Ala Ala Val Val Gly Ala
485 490 495

Gly Val Tyr Ser Ile Val His Asn Trp Met Phe Asp Trp Asn Ser Leu
500 505 510

Ser Tyr Arg Phe Ala Leu Val Gly Ile Cys Ser Tyr Ile Val Phe Ile
515 520 525

Val Ile Val Leu Val Val Tyr Phe Thr Gly Lys Ile Thr Thr Trp Asn

530 535 540

Phe Thr Lys Leu Gln Lys Glu Leu Ile Glu Asp Arg Val Leu Tyr Asp
545 550 555 560

Ala Thr Thr Asn Ala Gln Ser Val
565

<210> 3

<211> 1707

<212> DNA

<213> Artificial Sequence

<220>

<223> Synthetic sequence encoding Paramecium bursaria Chlorella Virus H yaluronansynthase protein

<400> 3

```
atgggtaaga acattatcat tatggtgtcc tggtacacaa ttattacaag taatctcatc      60
gcagttggtg gtgcatctct tattctcgct ccagctatca ctggatatgt tcttcactgg     120
aacatcgccc tctcaactat ttggggagtt tccgcatatg gtatttttgt tttcgggttc     180
tttttggctc aggttctgtt ctcagagctc aatcgtaaga gactcaggaa gtggattagc     240
cttagaccaa aggggtggaa tgacgttcgt ctcgctgtca ttatcgctgg ctaccgtgaa     300
gatccttaca tgtttcaaaa gtgcttggaa tcagttaggg atagtgatta tggcaacgtc     360
gctagactga tctgtgtgat tgatggagat gaggacgacg atatgaggat ggcagctgtt     420
tataaggcta tctataatga taacattaag aagcctgaat ttgttctttg cgagtctgat     480
gacaaggaag gagaacggat tgattcagat ttctcacgtg atatctgcgt tctccaacct     540
catcgtggga agcgtgaatg tctttataca ggtttccaac tcgccaaaat ggacccatca     600
gtgaacgctg tggttcttat cgatagtgat actgtgctgg agaaagatgc tatcttggag     660
gttgtttacc ctcttgcctg tgatcctgaa attcaagctg tggctggaga gtgcaagatc     720
tggaacacag atactcttct ttctctgctt gtcgcatgga gatattactc cgcattctgt     780
gtggagagga gcgctcaatc ctttttccgt accgttcaat gcgttggtgg tcctttggga     840
gcttacaaaa ttgatatcat caaggagatt aaggacccat ggattagtca aaggtttctt     900
ggtcagaagt gcacttatgg cgatgatcgt agattgacta acgaaatcct tatgaggggc     960
aagaaagtcg tttttactcc atttgctgtc ggatggtctg attcacctac aaatgttttc    1020
cgttatattg tgcaacaaac acgttggagt aagagctggt gtagggagat ctggtacact    1080
ttgttcgctg cttggaagca cgggcttagc ggaatttggc ttgcttttga atgcctttac    1140
cagattacat actttttctt ggtgatctat ttgttttcac gtcttgccgt cgaggctgac    1200
cctagagcac agactgcaac tgtgattgtt tctactacag tcgcacttat taagtgtggc    1260
```

```
tatttcagtt ttagagcaaa agatattaga gccttctatt ttgttttgta cacatttgtt    1320
tatttctttt gcatgattcc agctcgtatt accgctatga tgaccttgtg ggacatcgga    1380
tggggaacta gaggtggtaa cgaaaagcct tctgtgggaa caagggtggc cctttgggca    1440
aaacaatatc tcatcgccta catgtggtgg gccgctgtcg ttggtgccgg agtgtactca    1500
atcgttcata actggatgtt tgactggaac tctttgagct atcgtttcgc tcttgtgggt    1560
atttgttctt acattgtttt catcgtgatt gtgctcgttg tgtatttcac tggtaaaatc    1620
acaacctgga atttcactaa acttcaaaag gaattgattg aagacagggt tctgtatgat    1680
gctactacca acgcccagtc agtttaa                                        1707

<210>  4
<211>  1260
<212>  DNA
<213>  Paramecium bursaria Chlorella Virus 1


<220>
<221>  CDS
<222>  (62)..(1228)
<223>


<300>
<308>  U42580.4
<309>  2004-09-20
<313>  (291749.)..(292918)


<400>  4
atcaacgtga tttatatttt aaacaaagac cattcacatc tttagtactt aattaattat      60

a atg tca cga atc gca gtc gtt ggt tgt ggt tac gtc gga acc gct tgt     109
  Met Ser Arg Ile Ala Val Val Gly Cys Gly Tyr Val Gly Thr Ala Cys
  1               5                   10                  15

gca gta ctt ctt gct caa aaa aac gaa gtc atc gtg ctt gat att agc      157
Ala Val Leu Leu Ala Gln Lys Asn Glu Val Ile Val Leu Asp Ile Ser
            20                  25                  30

gaa gac cgt gtt caa cta atc aag aac aag aag agt cca atc gag gac      205
Glu Asp Arg Val Gln Leu Ile Lys Asn Lys Lys Ser Pro Ile Glu Asp
        35                  40                  45

aag gaa atc gaa gag ttt ctc gaa acg aaa gac ctg aac ctg acc gcg      253
Lys Glu Ile Glu Glu Phe Leu Glu Thr Lys Asp Leu Asn Leu Thr Ala
    50                  55                  60

acg act gac aag gtt ctt gca tac gaa aac gcc gaa ttt gtc atc atc      301
Thr Thr Asp Lys Val Leu Ala Tyr Glu Asn Ala Glu Phe Val Ile Ile
65                  70                  75                  80
```

```
gca acc ccg act gac tat gac gtg gtt act agg tat ttt aac acg aaa      349
Ala Thr Pro Thr Asp Tyr Asp Val Val Thr Arg Tyr Phe Asn Thr Lys
                85                  90                  95

tct gtg gaa aac gtc att ggg gac gtg atc aaa aat aca cag acc cat      397
Ser Val Glu Asn Val Ile Gly Asp Val Ile Lys Asn Thr Gln Thr His
            100                 105                 110

cca act atc gtg att aaa tct acc atc ccc att gga ttt gtt gat aag      445
Pro Thr Ile Val Ile Lys Ser Thr Ile Pro Ile Gly Phe Val Asp Lys
        115                 120                 125

gtt cgt gag caa ttc gac tac caa aat atc att ttc tcc cca gaa ttt      493
Val Arg Glu Gln Phe Asp Tyr Gln Asn Ile Ile Phe Ser Pro Glu Phe
    130                 135                 140

ctg cgt gaa ggt aga gcc ttg tat gat aat ctc tac cca tcc cgt atc      541
Leu Arg Glu Gly Arg Ala Leu Tyr Asp Asn Leu Tyr Pro Ser Arg Ile
145                 150                 155                 160

atc gta gga gat gat tcc ccc att gcg ctt aag ttc gca aac ctt ctc      589
Ile Val Gly Asp Asp Ser Pro Ile Ala Leu Lys Phe Ala Asn Leu Leu
                165                 170                 175

gtt gaa ggt tct aaa act ccg ctt gcc cct gtc ctg acg atg gga act      637
Val Glu Gly Ser Lys Thr Pro Leu Ala Pro Val Leu Thr Met Gly Thr
            180                 185                 190

cgc gaa gcc gag gcc gtc aaa cta ttc tct aac acg tat ctt gca atg      685
Arg Glu Ala Glu Ala Val Lys Leu Phe Ser Asn Thr Tyr Leu Ala Met
        195                 200                 205

cga gtt gca tac ttc aac gaa cta gat aca ttc gca atg tct cac ggt      733
Arg Val Ala Tyr Phe Asn Glu Leu Asp Thr Phe Ala Met Ser His Gly
    210                 215                 220

atg aat gcg aaa gaa atc att gat ggt gtg act ctg gag cct cgc att      781
Met Asn Ala Lys Glu Ile Ile Asp Gly Val Thr Leu Glu Pro Arg Ile
225                 230                 235                 240

ggt cag ggg tac tca aac cct tcg ttc ggt tat gga gct tat tgc ttt      829
Gly Gln Gly Tyr Ser Asn Pro Ser Phe Gly Tyr Gly Ala Tyr Cys Phe
                245                 250                 255

cca aag gat acg aag caa ctg ctg gct aat ttc gag gga gtg cct caa      877
Pro Lys Asp Thr Lys Gln Leu Leu Ala Asn Phe Glu Gly Val Pro Gln
            260                 265                 270

gat atc atc gga gca att gta gaa tca aat gag act cgc aag gaa gtg      925
Asp Ile Ile Gly Ala Ile Val Glu Ser Asn Glu Thr Arg Lys Glu Val
        275                 280                 285

att gtg agt gaa gta gaa aat cgt ttc ccc acg act gtt ggt gtg tat      973
Ile Val Ser Glu Val Glu Asn Arg Phe Pro Thr Thr Val Gly Val Tyr
    290                 295                 300

aag ctc gcc gct aaa gcg ggt tct gat aat ttt cgg agt tct gca att     1021
Lys Leu Ala Ala Lys Ala Gly Ser Asp Asn Phe Arg Ser Ser Ala Ile
305                 310                 315                 320

gta gac ata atg gag cga ctt gca aac aag ggt tat cac att aag att     1069
Val Asp Ile Met Glu Arg Leu Ala Asn Lys Gly Tyr His Ile Lys Ile
                325                 330                 335

ttc gaa cca act gtg gaa caa ttc gaa aac ttt gaa gtt gat aac aac     1117
Phe Glu Pro Thr Val Glu Gln Phe Glu Asn Phe Glu Val Asp Asn Asn
            340                 345                 350
```

```
ctg aca aca ttt gcg act gag agc gat gta att atc gca aac aga gtt      1165
Leu Thr Thr Phe Ala Thr Glu Ser Asp Val Ile Ile Ala Asn Arg Val
        355                 360                 365

ccc gtt gaa cat cgc att ctc ttt ggt aaa aaa tta atc aca cgt gat      1213
Pro Val Glu His Arg Ile Leu Phe Gly Lys Lys Leu Ile Thr Arg Asp
    370                 375                 380

gta tat ggc gat aac taaaatgttt tcaatatgat gttgttaatg at              1260
Val Tyr Gly Asp Asn
385
```

<210> 5

<211> 389

<212> PRT

<213> Paramecium bursaria Chlorella Virus 1

<400> 5

```
Met Ser Arg Ile Ala Val Val Gly Cys Gly Tyr Val Gly Thr Ala Cys
1               5                   10                  15

Ala Val Leu Leu Ala Gln Lys Asn Glu Val Ile Val Leu Asp Ile Ser
            20                  25                  30

Glu Asp Arg Val Gln Leu Ile Lys Asn Lys Lys Ser Pro Ile Glu Asp
        35                  40                  45

Lys Glu Ile Glu Glu Phe Leu Glu Thr Lys Asp Leu Asn Leu Thr Ala
    50                  55                  60

Thr Thr Asp Lys Val Leu Ala Tyr Glu Asn Ala Glu Phe Val Ile Ile
65                  70                  75                  80

Ala Thr Pro Thr Asp Tyr Asp Val Val Thr Arg Tyr Phe Asn Thr Lys
                85                  90                  95

Ser Val Glu Asn Val Ile Gly Asp Val Ile Lys Asn Thr Gln Thr His
            100                 105                 110

Pro Thr Ile Val Ile Lys Ser Thr Ile Pro Ile Gly Phe Val Asp Lys
        115                 120                 125

Val Arg Glu Gln Phe Asp Tyr Gln Asn Ile Ile Phe Ser Pro Glu Phe
    130                 135                 140

Leu Arg Glu Gly Arg Ala Leu Tyr Asp Asn Leu Tyr Pro Ser Arg Ile
145                 150                 155                 160

Ile Val Gly Asp Asp Ser Pro Ile Ala Leu Lys Phe Ala Asn Leu Leu
                165                 170                 175
```

```
Val Glu Gly Ser Lys Thr Pro Leu Ala Pro Val Leu Thr Met Gly Thr
            180                 185                 190

Arg Glu Ala Glu Ala Val Lys Leu Phe Ser Asn Thr Tyr Leu Ala Met
        195                 200                 205

Arg Val Ala Tyr Phe Asn Glu Leu Asp Thr Phe Ala Met Ser His Gly
    210                 215                 220

Met Asn Ala Lys Glu Ile Ile Asp Gly Val Thr Leu Glu Pro Arg Ile
225                 230                 235                 240

Gly Gln Gly Tyr Ser Asn Pro Ser Phe Gly Tyr Gly Ala Tyr Cys Phe
                245                 250                 255

Pro Lys Asp Thr Lys Gln Leu Leu Ala Asn Phe Glu Gly Val Pro Gln
            260                 265                 270

Asp Ile Ile Gly Ala Ile Val Glu Ser Asn Glu Thr Arg Lys Glu Val
        275                 280                 285

Ile Val Ser Glu Val Glu Asn Arg Phe Pro Thr Thr Val Gly Val Tyr
    290                 295                 300

Lys Leu Ala Ala Lys Ala Gly Ser Asp Asn Phe Arg Ser Ser Ala Ile
305                 310                 315                 320

Val Asp Ile Met Glu Arg Leu Ala Asn Lys Gly Tyr His Ile Lys Ile
                325                 330                 335

Phe Glu Pro Thr Val Glu Gln Phe Glu Asn Phe Glu Val Asp Asn Asn
            340                 345                 350

Leu Thr Thr Phe Ala Thr Glu Ser Asp Val Ile Ile Ala Asn Arg Val
        355                 360                 365

Pro Val Glu His Arg Ile Leu Phe Gly Lys Lys Leu Ile Thr Arg Asp
    370                 375                 380

Val Tyr Gly Asp Asn
385
```

<210> 6

<211> 1170

<212> DNA

<213> Artificial Sequence

<220>

<223> Synthetic sequence encoding a Paramecium bursaria Chlorella Virus

protein having the activity of a UDP-Glc-DH

<400> 6
atgtctcgca tagctgttgt aggatgtggc tatgtgggaa ctgcatgtgc ggttctactt 60

gctcaaaaga acgaagttat tgtgcttgat attagtgaag accgtgttca acttattaag 120

aacaagaagt ctcctattga ggataaggaa atcgaagagt tcttggaaac aaaggatctt 180

aatcttactg cgactacaga taaggttctt gcctacgaga acgctgagtt tgtgataatc 240

gctacaccaa ccgattacga cgttgtgact cgatatttca ataccaaatc cgtggaaaac 300

gttataggag atgttatcaa gaacactcaa acccacccta ctatcgtcat caagtccaca 360

attcccatcg gtttcgttga taaggtcaga gagcagtttg attatcaaaa cattatcttc 420

tcacctgagt tcttaaggga gggtcgtgct ctctacgata atttgtatcc gtcccgtatt 480

atcgttggcg acgattctcc tatcgctctc aagttcgcaa atctcttagt tgagggtagt 540

aagacccctt tggctcctgt tttgacaatg ggaaccagag aagcagaagc tgtcaagcta 600

ttctctaata cctaccttgc catgagggta gcatacttta acgaacttga tacatttgct 660

atgtcgcatg gtatgaatgc caaggagatt atagatggtg tcactttaga gcccaggatc 720

ggtcaaggat attctaaccc atcattcggc tatggagctt actgctttcc taaggacact 780

aagcagttgc tggcaaactt cgagggagtt cctcaagaca tcataggcgc tattgtggag 840

tcaaacgaaa caaggaaaga ggtgatagtt agtgaggtag agaatcgttt cccaacgaca 900

gtcggtgttt acaaactggc agctaaagct ggtagcgata acttcaggtc aagtgctatt 960

gtcgacatca tggaacgcct ggctaacaaa ggttaccaca ttaagatctt tgagccaact 1020

gtagagcagt tcgaaaattt cgaagttgac aataacttga caacgtttgc tactgagtca 1080

gacgttatta tcgcaaatcg tgtccctgtg gaacatagaa tcctatttgg aaagaagctc 1140

attaccagag atgtttacgg tgataattaa 1170

<210> 7

<211> 2298

<212> DNA

<213> Mus musculus

<220>

<221> CDS

<222> (150)..(2192)

<223>

<220>

<221> mutation

<222> (1060)..(1060)

<223> sequnce inserted in plasmid IC 370-256 contains an exchnage from G to A at position 1060

<220>

<221> mutation

<222> (1245)..(1245)

<223> sequnce inserted in plasmid IC 370-256 contains an exchnage from T to C at position 1245

<220>

<221> allele

<222> (1190)..(1190)

<223> sequnce inserted in plasmid IC 365-256 contains a base exchnage f rom T to C at position 1190

<220>

<221> allele

<222> (2027)..(2027)

<223> sequnce inserted in plasmid IC 365-256 contains a base exchnage f rom G to A at position 2027

<300>

<308> BC050762.1

<309> 2005-03-08

<313> (150)..(2195)

<400> 7
```
gagagcgaag cgagcgctga gtcggactgt cgggtctgag ctgtcgcatc ccagagtcct      60

ctcattgcca ccaccccggc ccgagctcac cctcgcttct gaagctctcc gcgcgcccga     120

cagctcagcc ctcgcccgtg accaacatc atg tgc ggt ata ttt gct tat tta       173
                                Met Cys Gly Ile Phe Ala Tyr Leu
                                1               5

aat tac cat gtt cct cga aca aga cga gaa atc ttg gag aca cta atc       221
Asn Tyr His Val Pro Arg Thr Arg Arg Glu Ile Leu Glu Thr Leu Ile
    10                  15                  20

aaa ggc ctt cag aga ctg gaa tac aga gga tat gat tct gct ggt gtg       269
Lys Gly Leu Gln Arg Leu Glu Tyr Arg Gly Tyr Asp Ser Ala Gly Val
25                  30                  35                  40

gga ctt gac gga ggc aat gac aaa gac tgg gaa gcc aac gcc tgc aaa       317
Gly Leu Asp Gly Gly Asn Asp Lys Asp Trp Glu Ala Asn Ala Cys Lys
                45                  50                  55
```

```
atc cag ctc att aag aag aaa gga aaa gtt aag gca ctg gat gaa gaa     365
Ile Gln Leu Ile Lys Lys Lys Gly Lys Val Lys Ala Leu Asp Glu Glu
            60                  65                  70

gtt cac aaa caa caa gat atg gac ttg gat ata gaa ttt gat gtg cat     413
Val His Lys Gln Gln Asp Met Asp Leu Asp Ile Glu Phe Asp Val His
        75                  80                  85

ctt gga ata gct cat acc cgt tgg gcg aca cat gga gaa ccc aat cct     461
Leu Gly Ile Ala His Thr Arg Trp Ala Thr His Gly Glu Pro Asn Pro
    90                  95                  100

gtc aat agt cac ccc cag cgc tct gat aaa aat aat gaa ttc att gtt     509
Val Asn Ser His Pro Gln Arg Ser Asp Lys Asn Asn Glu Phe Ile Val
105                 110                 115                 120

att cat aat gga atc atc acc aac tac aaa gac ttg aaa aag ttt ctg     557
Ile His Asn Gly Ile Ile Thr Asn Tyr Lys Asp Leu Lys Lys Phe Leu
                125                 130                 135

gaa agc aaa ggc tat gac ttt gaa tct gaa aca gac aca gaa acc att     605
Glu Ser Lys Gly Tyr Asp Phe Glu Ser Glu Thr Asp Thr Glu Thr Ile
            140                 145                 150

gcc aag ctc gtc aag tac atg tat gac aac tgg gag agc cag gac gtc     653
Ala Lys Leu Val Lys Tyr Met Tyr Asp Asn Trp Glu Ser Gln Asp Val
        155                 160                 165

agt ttt acc acc ttg gtg gag aga gtt atc caa caa ttg gaa ggc gcc     701
Ser Phe Thr Thr Leu Val Glu Arg Val Ile Gln Gln Leu Glu Gly Ala
    170                 175                 180

ttt gct ctt gtg ttt aaa agt gtc cat ttt ccc ggg caa gca gtt ggc     749
Phe Ala Leu Val Phe Lys Ser Val His Phe Pro Gly Gln Ala Val Gly
185                 190                 195                 200

aca agg cga ggt agc cct ctc ttg att ggt gtg cgg agt gaa cat aag     797
Thr Arg Arg Gly Ser Pro Leu Leu Ile Gly Val Arg Ser Glu His Lys
                205                 210                 215

ctt tct aca gat cac att ccg att ctg tac aga aca ggc aaa gac aag     845
Leu Ser Thr Asp His Ile Pro Ile Leu Tyr Arg Thr Gly Lys Asp Lys
            220                 225                 230

aaa gga agc tgc ggt ctt tcc cgt gtg gac agc acg aca tgc ctg ttc     893
Lys Gly Ser Cys Gly Leu Ser Arg Val Asp Ser Thr Thr Cys Leu Phe
        235                 240                 245

cct gtt gag gaa aag gca gtt gaa tat tac ttt gct tct gat gca agt     941
Pro Val Glu Glu Lys Ala Val Glu Tyr Tyr Phe Ala Ser Asp Ala Ser
    250                 255                 260

gcc gtg ata gag cac acc aat cgt gtc atc ttt ctg gaa gat gat gat     989
Ala Val Ile Glu His Thr Asn Arg Val Ile Phe Leu Glu Asp Asp Asp
265                 270                 275                 280

gtt gca gca gtg gtg gat ggc cgt ctc tct atc cac cga att aaa cga    1037
Val Ala Ala Val Val Asp Gly Arg Leu Ser Ile His Arg Ile Lys Arg
                285                 290                 295

act gca gga gac cat cct ggc cga gct gtg caa act ctc cag atg gag    1085
Thr Ala Gly Asp His Pro Gly Arg Ala Val Gln Thr Leu Gln Met Glu
            300                 305                 310

ctc cag cag atc atg aag ggc aac ttt agt tca ttt atg cag aag gaa    1133
Leu Gln Gln Ile Met Lys Gly Asn Phe Ser Ser Phe Met Gln Lys Glu
        315                 320                 325
```

```
att ttt gag cag cca gaa tct gtt gtg aac aca atg aga gga aga gtc      1181
Ile Phe Glu Gln Pro Glu Ser Val Val Asn Thr Met Arg Gly Arg Val
    330                 335                 340

aat ttt gat gac tac act gtg aat ttg gga ggt ttg aaa gat cac att      1229
Asn Phe Asp Asp Tyr Thr Val Asn Leu Gly Gly Leu Lys Asp His Ile
345                 350                 355                 360

aag gag atc cag cgg tgt cgg cgg ttg att ctt att gct tgt ggc aca      1277
Lys Glu Ile Gln Arg Cys Arg Arg Leu Ile Leu Ile Ala Cys Gly Thr
                365                 370                 375

agt tac cac gct ggt gtg gca acc cgt cag gtc ctg gag gag ctg acc      1325
Ser Tyr His Ala Gly Val Ala Thr Arg Gln Val Leu Glu Glu Leu Thr
            380                 385                 390

gag ctg ccc gtg atg gtg gag ctt gcc agt gac ttc ttg gat aga aac      1373
Glu Leu Pro Val Met Val Glu Leu Ala Ser Asp Phe Leu Asp Arg Asn
        395                 400                 405

act cca gtc ttt cga gat gat gtt tgc ttt ttc att agt caa tca ggc      1421
Thr Pro Val Phe Arg Asp Asp Val Cys Phe Phe Ile Ser Gln Ser Gly
    410                 415                 420

gag aca gct gac acc ctg atg gga ctt cgt tac tgt aag gag aga gga      1469
Glu Thr Ala Asp Thr Leu Met Gly Leu Arg Tyr Cys Lys Glu Arg Gly
425                 430                 435                 440

gcc tta act gtg ggg atc aca aat aca gtc ggc agt tct ata tca agg      1517
Ala Leu Thr Val Gly Ile Thr Asn Thr Val Gly Ser Ser Ile Ser Arg
                445                 450                 455

gag aca gat tgc ggg gtt cat att aat gct ggt cct gag att ggc gtg      1565
Glu Thr Asp Cys Gly Val His Ile Asn Ala Gly Pro Glu Ile Gly Val
            460                 465                 470

gcc agt aca aag gca tac acc agc cag ttt gtg tcc ctc gtg atg ttt      1613
Ala Ser Thr Lys Ala Tyr Thr Ser Gln Phe Val Ser Leu Val Met Phe
        475                 480                 485

gct ctc atg atg tgt gat gac agg atc tcc atg caa gag aga cgc aaa      1661
Ala Leu Met Met Cys Asp Asp Arg Ile Ser Met Gln Glu Arg Arg Lys
    490                 495                 500

gag atc atg ctc gga ctg aag cga ctg ccg gac ttg att aag gaa gtg      1709
Glu Ile Met Leu Gly Leu Lys Arg Leu Pro Asp Leu Ile Lys Glu Val
505                 510                 515                 520

ctg agc atg gat gat gaa atc cag aag ctg gcg acg gag ctt tac cac      1757
Leu Ser Met Asp Asp Glu Ile Gln Lys Leu Ala Thr Glu Leu Tyr His
                525                 530                 535

cag aag tcg gtc ctg ata atg ggg cgg ggc tac cat tat gct aca tgc      1805
Gln Lys Ser Val Leu Ile Met Gly Arg Gly Tyr His Tyr Ala Thr Cys
            540                 545                 550

ctt gaa ggg gct ctg aaa atc aag gag att act tat atg cat tcg gaa      1853
Leu Glu Gly Ala Leu Lys Ile Lys Glu Ile Thr Tyr Met His Ser Glu
        555                 560                 565

ggc atc ctt gct ggt gag ctc aag cac ggc cct ctg gcc ttg gtg gac      1901
Gly Ile Leu Ala Gly Glu Leu Lys His Gly Pro Leu Ala Leu Val Asp
    570                 575                 580

aag ttg atg cct gtc atc atg atc atc atg cga gac cac act tat gcc      1949
Lys Leu Met Pro Val Ile Met Ile Ile Met Arg Asp His Thr Tyr Ala
585                 590                 595                 600

aag tgc cag aac gct ctt cag cag gtg gtt gca cgg cag ggg cgt cca      1997
```

```
Lys Cys Gln Asn Ala Leu Gln Gln Val Val Ala Arg Gln Gly Arg Pro
                605                 610                 615

gtc gtg atc tgt gat aag gag gat act gag acc att aag aat aca aaa      2045
Val Val Ile Cys Asp Lys Glu Asp Thr Glu Thr Ile Lys Asn Thr Lys
            620                 625                 630

agg aca atc aag gtg ccc cac tca gtg gac tgc ttg cag ggc att ctc      2093
Arg Thr Ile Lys Val Pro His Ser Val Asp Cys Leu Gln Gly Ile Leu
        635                 640                 645

agt gtg att ccc ctg cag ctg ctg gct ttc cac ctg gct gtg ctg aga      2141
Ser Val Ile Pro Leu Gln Leu Leu Ala Phe His Leu Ala Val Leu Arg
    650                 655                 660

ggc tac gat gtt gat ttt cca cgg aat ctt gcc aaa tct gta aca gta      2189
Gly Tyr Asp Val Asp Phe Pro Arg Asn Leu Ala Lys Ser Val Thr Val
665                 670                 675                 680

gag taacagacac ctgaaactta agacagttaa gcaacacgag ataccttttg           2242
Glu

tatttaaatt tttgatttaa actatcaaaa aaaaaaaaaa aaaaaaaaaa aaaaaa        2298
```

<210> 8

<211> 681

<212> PRT

<213> Mus musculus

<400> 8

```
Met Cys Gly Ile Phe Ala Tyr Leu Asn Tyr His Val Pro Arg Thr Arg
1               5                   10                  15

Arg Glu Ile Leu Glu Thr Leu Ile Lys Gly Leu Gln Arg Leu Glu Tyr
            20                  25                  30

Arg Gly Tyr Asp Ser Ala Gly Val Gly Leu Asp Gly Gly Asn Asp Lys
        35                  40                  45

Asp Trp Glu Ala Asn Ala Cys Lys Ile Gln Leu Ile Lys Lys Lys Gly
    50                  55                  60

Lys Val Lys Ala Leu Asp Glu Glu Val His Lys Gln Gln Asp Met Asp
65                  70                  75                  80

Leu Asp Ile Glu Phe Asp Val His Leu Gly Ile Ala His Thr Arg Trp
                85                  90                  95

Ala Thr His Gly Glu Pro Asn Pro Val Asn Ser His Pro Gln Arg Ser
            100                 105                 110

Asp Lys Asn Asn Glu Phe Ile Val Ile His Asn Gly Ile Ile Thr Asn
        115                 120                 125
```

Tyr Lys Asp Leu Lys Lys Phe Leu Glu Ser Lys Gly Tyr Asp Phe Glu
130 135 140

Ser Glu Thr Asp Thr Glu Thr Ile Ala Lys Leu Val Lys Tyr Met Tyr
145 150 155 160

Asp Asn Trp Glu Ser Gln Asp Val Ser Phe Thr Thr Leu Val Glu Arg
165 170 175

Val Ile Gln Gln Leu Glu Gly Ala Phe Ala Leu Val Phe Lys Ser Val
180 185 190

His Phe Pro Gly Gln Ala Val Gly Thr Arg Arg Gly Ser Pro Leu Leu
195 200 205

Ile Gly Val Arg Ser Glu His Lys Leu Ser Thr Asp His Ile Pro Ile
210 215 220

Leu Tyr Arg Thr Gly Lys Asp Lys Lys Gly Ser Cys Gly Leu Ser Arg
225 230 235 240

Val Asp Ser Thr Thr Cys Leu Phe Pro Val Glu Glu Lys Ala Val Glu
245 250 255

Tyr Tyr Phe Ala Ser Asp Ala Ser Ala Val Ile Glu His Thr Asn Arg
260 265 270

Val Ile Phe Leu Glu Asp Asp Asp Val Ala Ala Val Val Asp Gly Arg
275 280 285

Leu Ser Ile His Arg Ile Lys Arg Thr Ala Gly Asp His Pro Gly Arg
290 295 300

Ala Val Gln Thr Leu Gln Met Glu Leu Gln Gln Ile Met Lys Gly Asn
305 310 315 320

Phe Ser Ser Phe Met Gln Lys Glu Ile Phe Glu Gln Pro Glu Ser Val
325 330 335

Val Asn Thr Met Arg Gly Arg Val Asn Phe Asp Asp Tyr Thr Val Asn
340 345 350

Leu Gly Gly Leu Lys Asp His Ile Lys Glu Ile Gln Arg Cys Arg Arg
355 360 365

Leu Ile Leu Ile Ala Cys Gly Thr Ser Tyr His Ala Gly Val Ala Thr
370 375 380

Arg Gln Val Leu Glu Glu Leu Thr Glu Leu Pro Val Met Val Glu Leu
385 390 395 400

Ala Ser Asp Phe Leu Asp Arg Asn Thr Pro Val Phe Arg Asp Asp Val
405 410 415

Cys Phe Phe Ile Ser Gln Ser Gly Glu Thr Ala Asp Thr Leu Met Gly
420 425 430

Leu Arg Tyr Cys Lys Glu Arg Gly Ala Leu Thr Val Gly Ile Thr Asn
435 440 445

Thr Val Gly Ser Ser Ile Ser Arg Glu Thr Asp Cys Gly Val His Ile
450 455 460

Asn Ala Gly Pro Glu Ile Gly Val Ala Ser Thr Lys Ala Tyr Thr Ser
465 470 475 480

Gln Phe Val Ser Leu Val Met Phe Ala Leu Met Met Cys Asp Asp Arg
485 490 495

Ile Ser Met Gln Glu Arg Arg Lys Glu Ile Met Leu Gly Leu Lys Arg
500 505 510

Leu Pro Asp Leu Ile Lys Glu Val Leu Ser Met Asp Asp Glu Ile Gln
515 520 525

Lys Leu Ala Thr Glu Leu Tyr His Gln Lys Ser Val Leu Ile Met Gly
530 535 540

Arg Gly Tyr His Tyr Ala Thr Cys Leu Glu Gly Ala Leu Lys Ile Lys
545 550 555 560

Glu Ile Thr Tyr Met His Ser Glu Gly Ile Leu Ala Gly Glu Leu Lys
565 570 575

His Gly Pro Leu Ala Leu Val Asp Lys Leu Met Pro Val Ile Met Ile
580 585 590

Ile Met Arg Asp His Thr Tyr Ala Lys Cys Gln Asn Ala Leu Gln Gln
595 600 605

Val Val Ala Arg Gln Gly Arg Pro Val Val Ile Cys Asp Lys Glu Asp
610 615 620

Thr Glu Thr Ile Lys Asn Thr Lys Arg Thr Ile Lys Val Pro His Ser
625 630 635 640

Val Asp Cys Leu Gln Gly Ile Leu Ser Val Ile Pro Leu Gln Leu Leu
645 650 655

Ala Phe His Leu Ala Val Leu Arg Gly Tyr Asp Val Asp Phe Pro Arg
660 665 670

Asn Leu Ala Lys Ser Val Thr Val Glu

675 680

<210> 9

<211> 2049

<212> DNA

<213> Mus musculus

<220>

<221> CDS

<222> (1)..(2046)

<223>

<300>

<308> BC031928.1

<309> 2003-10-07

<313> (51)..(299)

<400> 9
```
atg tgc gga atc ttt gcc tac atg aat tac aga gtt ccc aag aca agg      48
Met Cys Gly Ile Phe Ala Tyr Met Asn Tyr Arg Val Pro Lys Thr Arg
1               5                   10                  15

aaa gag att ttc gaa acc ctt atc agg ggt ctg cag cgg ctg gag tac      96
Lys Glu Ile Phe Glu Thr Leu Ile Arg Gly Leu Gln Arg Leu Glu Tyr
            20                  25                  30

cgg ggc tat gac tct gcg ggg gtt gcc att gat ggg aat aac cac gaa     144
Arg Gly Tyr Asp Ser Ala Gly Val Ala Ile Asp Gly Asn Asn His Glu
        35                  40                  45

gtc aaa gaa aga cac atc cat ctt gtg aag aaa agg ggg aaa gta aag     192
Val Lys Glu Arg His Ile His Leu Val Lys Lys Arg Gly Lys Val Lys
    50                  55                  60

gct ctg gat gaa gaa ctt tac aag caa gat agc atg gac ttg aag gtg     240
Ala Leu Asp Glu Glu Leu Tyr Lys Gln Asp Ser Met Asp Leu Lys Val
65                  70                  75                  80

gag ttt gag aca cac ttc ggc att gcc cac aca cgt tgg gcc acc cac     288
Glu Phe Glu Thr His Phe Gly Ile Ala His Thr Arg Trp Ala Thr His
                85                  90                  95

ggg gtt ccc aat gct gtc aac agt cac ccg cag cgt tcg gac aaa gac     336
Gly Val Pro Asn Ala Val Asn Ser His Pro Gln Arg Ser Asp Lys Asp
            100                 105                 110

aat gaa ttt gtt gtc atc cac aac ggg atc atc act aat tac aag gat     384
Asn Glu Phe Val Val Ile His Asn Gly Ile Ile Thr Asn Tyr Lys Asp
        115                 120                 125

cta agg aag ttt ctg gaa agc aaa ggc tac gag ttt gag tca gaa aca     432
Leu Arg Lys Phe Leu Glu Ser Lys Gly Tyr Glu Phe Glu Ser Glu Thr
    130                 135                 140
```

```
gac acg gag acc atc gcc aag ctg att aaa tat gta ttt gac aac aga       480
Asp Thr Glu Thr Ile Ala Lys Leu Ile Lys Tyr Val Phe Asp Asn Arg
145                 150                 155                 160

gag act gag gac ata acg ttt tcc aca ttg gtc gaa aga gtc att cag       528
Glu Thr Glu Asp Ile Thr Phe Ser Thr Leu Val Glu Arg Val Ile Gln
                165                 170                 175

cag ttg gaa ggc gcc ttt gca ctg gtt ttc aag agt att cac tac ccg       576
Gln Leu Glu Gly Ala Phe Ala Leu Val Phe Lys Ser Ile His Tyr Pro
            180                 185                 190

gga gaa gct gtc gcc acg agg aga ggc agc ccc ttg ctc atc ggg gta       624
Gly Glu Ala Val Ala Thr Arg Arg Gly Ser Pro Leu Leu Ile Gly Val
        195                 200                 205

cga agc aaa tac aaa ctc tcc aca gag cag atc ccc gtc tta tat ccg       672
Arg Ser Lys Tyr Lys Leu Ser Thr Glu Gln Ile Pro Val Leu Tyr Pro
    210                 215                 220

aca tgc aat atc gag aat gtg aag aat atc tgc aag act agg atg aag       720
Thr Cys Asn Ile Glu Asn Val Lys Asn Ile Cys Lys Thr Arg Met Lys
225                 230                 235                 240

aga ctg gac agc tcc acc tgc ctg cac gct gtg ggc gat aaa gct gtg       768
Arg Leu Asp Ser Ser Thr Cys Leu His Ala Val Gly Asp Lys Ala Val
                245                 250                 255

gaa ttc ttc ttt gct tct gat gca agt gcc atc ata gaa cac acc aac       816
Glu Phe Phe Phe Ala Ser Asp Ala Ser Ala Ile Ile Glu His Thr Asn
            260                 265                 270

cgg gtc atc ttc tta gaa gat gat gat atc gct gca gtg gct gat ggg       864
Arg Val Ile Phe Leu Glu Asp Asp Asp Ile Ala Ala Val Ala Asp Gly
        275                 280                 285

aaa ctc tcc att cac cga gtc aag cgc tca gct act gat gac ccc tcc       912
Lys Leu Ser Ile His Arg Val Lys Arg Ser Ala Thr Asp Asp Pro Ser
    290                 295                 300

cga gcc atc cag acc ttg cag atg gaa ctg cag caa ata atg aaa ggt       960
Arg Ala Ile Gln Thr Leu Gln Met Glu Leu Gln Gln Ile Met Lys Gly
305                 310                 315                 320

aac ttc agc gca ttt atg cag aag gag atc ttc gag cag cca gaa tca      1008
Asn Phe Ser Ala Phe Met Gln Lys Glu Ile Phe Glu Gln Pro Glu Ser
                325                 330                 335

gtt ttt aat acc atg aga ggt cgg gtg aat ttt gag acc aac aca gtg      1056
Val Phe Asn Thr Met Arg Gly Arg Val Asn Phe Glu Thr Asn Thr Val
            340                 345                 350

ctc ctg ggt ggc ttg aag gac cat ttg aaa gag atc cga cga tgc cga      1104
Leu Leu Gly Gly Leu Lys Asp His Leu Lys Glu Ile Arg Arg Cys Arg
        355                 360                 365

agg ctc att gtg att ggc tgt gga acc agc tac cat gcc gct gtg gct      1152
Arg Leu Ile Val Ile Gly Cys Gly Thr Ser Tyr His Ala Ala Val Ala
    370                 375                 380

aca cgg caa gtc tta gag gaa ctg acc gag ctg cct gtg atg gtt gaa      1200
Thr Arg Gln Val Leu Glu Glu Leu Thr Glu Leu Pro Val Met Val Glu
385                 390                 395                 400

ctt gcc agt gac ttt ctg gac agg aac aca cct gtg ttc agg gat gac      1248
Leu Ala Ser Asp Phe Leu Asp Arg Asn Thr Pro Val Phe Arg Asp Asp
                405                 410                 415
```

```
gtt tgc ttt ttc ata agc caa tca ggt gag act gca gac acg ctc ctg      1296
Val Cys Phe Phe Ile Ser Gln Ser Gly Glu Thr Ala Asp Thr Leu Leu
            420                 425                 430

gcg ctg cga tac tgt aag gat cga ggt gcg ctg acc gtg ggc atc acc      1344
Ala Leu Arg Tyr Cys Lys Asp Arg Gly Ala Leu Thr Val Gly Ile Thr
        435                 440                 445

aac acc gtg ggt agc tcc atc tcc cgg gag act gac tgt ggc gtc cac      1392
Asn Thr Val Gly Ser Ser Ile Ser Arg Glu Thr Asp Cys Gly Val His
    450                 455                 460

atc aac gca ggg ccc gag att ggg gtg gcc agc acc aag gcg tac acc      1440
Ile Asn Ala Gly Pro Glu Ile Gly Val Ala Ser Thr Lys Ala Tyr Thr
465                 470                 475                 480

agc cag ttc atc tct ctg gtg atg ttt ggt ttg atg atg tct gaa gat      1488
Ser Gln Phe Ile Ser Leu Val Met Phe Gly Leu Met Met Ser Glu Asp
                485                 490                 495

cga att tct cta cag aac agg aga caa gag atc atc cgt ggc ctc aga      1536
Arg Ile Ser Leu Gln Asn Arg Arg Gln Glu Ile Ile Arg Gly Leu Arg
            500                 505                 510

tct tta ccg gag ctg atc aaa gaa gtg ctg tcc ctg gat gag aag atc      1584
Ser Leu Pro Glu Leu Ile Lys Glu Val Leu Ser Leu Asp Glu Lys Ile
        515                 520                 525

cat gac ttg gcc ctg gag ctc tac aca caa agg tct ctc ctc gtg atg      1632
His Asp Leu Ala Leu Glu Leu Tyr Thr Gln Arg Ser Leu Leu Val Met
    530                 535                 540

gga cgg gga tat aac tat gcc aca tgt ctg gaa ggt gcc ttg aaa att      1680
Gly Arg Gly Tyr Asn Tyr Ala Thr Cys Leu Glu Gly Ala Leu Lys Ile
545                 550                 555                 560

aag gag ata acc tac atg cat tca gaa ggt atc cta gcc gga gag ctg      1728
Lys Glu Ile Thr Tyr Met His Ser Glu Gly Ile Leu Ala Gly Glu Leu
                565                 570                 575

aag cac ggg ccc ctt gct ctc gtc gac aag cag atg cca gtc atc atg      1776
Lys His Gly Pro Leu Ala Leu Val Asp Lys Gln Met Pro Val Ile Met
            580                 585                 590

gtc atc atg aag gat cct tgc ttt gcc aag tgc cag aat gcc ctg cag      1824
Val Ile Met Lys Asp Pro Cys Phe Ala Lys Cys Gln Asn Ala Leu Gln
        595                 600                 605

cag gtc act gcc cgc cag ggt cgc cca atc ata ctg tgt tcc aag gat      1872
Gln Val Thr Ala Arg Gln Gly Arg Pro Ile Ile Leu Cys Ser Lys Asp
    610                 615                 620

gac acc gag agc tcc aag ttt gca tat aaa acc att gaa ctt ccc cac      1920
Asp Thr Glu Ser Ser Lys Phe Ala Tyr Lys Thr Ile Glu Leu Pro His
625                 630                 635                 640

aca gtg gac tgt ctc cag ggt atc ctg agc gtg att cca ctc cag ctt      1968
Thr Val Asp Cys Leu Gln Gly Ile Leu Ser Val Ile Pro Leu Gln Leu
                645                 650                 655

ctg tcc ttc cac ctg gct gtc ctc cga ggt tat gat gtt gac ttc ccc      2016
Leu Ser Phe His Leu Ala Val Leu Arg Gly Tyr Asp Val Asp Phe Pro
            660                 665                 670

aga aac cta gcc aag tct gtc act gtg gaa tga                          2049
Arg Asn Leu Ala Lys Ser Val Thr Val Glu
        675                 680
```

<210> 10

<211> 682

<212> PRT

<213> Mus musculus

<400> 10

```
Met Cys Gly Ile Phe Ala Tyr Met Asn Tyr Arg Val Pro Lys Thr Arg
1               5                   10                  15

Lys Glu Ile Phe Glu Thr Leu Ile Arg Gly Leu Gln Arg Leu Glu Tyr
            20                  25                  30

Arg Gly Tyr Asp Ser Ala Gly Val Ala Ile Asp Gly Asn Asn His Glu
        35                  40                  45

Val Lys Glu Arg His Ile His Leu Val Lys Lys Arg Gly Lys Val Lys
    50                  55                  60

Ala Leu Asp Glu Glu Leu Tyr Lys Gln Asp Ser Met Asp Leu Lys Val
65                  70                  75                  80

Glu Phe Glu Thr His Phe Gly Ile Ala His Thr Arg Trp Ala Thr His
                85                  90                  95

Gly Val Pro Asn Ala Val Asn Ser His Pro Gln Arg Ser Asp Lys Asp
            100                 105                 110

Asn Glu Phe Val Val Ile His Asn Gly Ile Ile Thr Asn Tyr Lys Asp
        115                 120                 125

Leu Arg Lys Phe Leu Glu Ser Lys Gly Tyr Glu Phe Glu Ser Glu Thr
    130                 135                 140

Asp Thr Glu Thr Ile Ala Lys Leu Ile Lys Tyr Val Phe Asp Asn Arg
145                 150                 155                 160

Glu Thr Glu Asp Ile Thr Phe Ser Thr Leu Val Glu Arg Val Ile Gln
                165                 170                 175

Gln Leu Glu Gly Ala Phe Ala Leu Val Phe Lys Ser Ile His Tyr Pro
            180                 185                 190

Gly Glu Ala Val Ala Thr Arg Arg Gly Ser Pro Leu Leu Ile Gly Val
        195                 200                 205

Arg Ser Lys Tyr Lys Leu Ser Thr Glu Gln Ile Pro Val Leu Tyr Pro
    210                 215                 220

Thr Cys Asn Ile Glu Asn Val Lys Asn Ile Cys Lys Thr Arg Met Lys
```

225 230 235 240

Arg Leu Asp Ser Ser Thr Cys Leu His Ala Val Gly Asp Lys Ala Val
245 250 255

Glu Phe Phe Phe Ala Ser Asp Ala Ser Ala Ile Ile Glu His Thr Asn
260 265 270

Arg Val Ile Phe Leu Glu Asp Asp Asp Ile Ala Ala Val Ala Asp Gly
275 280 285

Lys Leu Ser Ile His Arg Val Lys Arg Ser Ala Thr Asp Asp Pro Ser
290 295 300

Arg Ala Ile Gln Thr Leu Gln Met Glu Leu Gln Gln Ile Met Lys Gly
305 310 315 320

Asn Phe Ser Ala Phe Met Gln Lys Glu Ile Phe Glu Gln Pro Glu Ser
325 330 335

Val Phe Asn Thr Met Arg Gly Arg Val Asn Phe Glu Thr Asn Thr Val
340 345 350

Leu Leu Gly Gly Leu Lys Asp His Leu Lys Glu Ile Arg Arg Cys Arg
355 360 365

Arg Leu Ile Val Ile Gly Cys Gly Thr Ser Tyr His Ala Ala Val Ala
370 375 380

Thr Arg Gln Val Leu Glu Glu Leu Thr Glu Leu Pro Val Met Val Glu
385 390 395 400

Leu Ala Ser Asp Phe Leu Asp Arg Asn Thr Pro Val Phe Arg Asp Asp
405 410 415

Val Cys Phe Phe Ile Ser Gln Ser Gly Glu Thr Ala Asp Thr Leu Leu
420 425 430

Ala Leu Arg Tyr Cys Lys Asp Arg Gly Ala Leu Thr Val Gly Ile Thr
435 440 445

Asn Thr Val Gly Ser Ser Ile Ser Arg Glu Thr Asp Cys Gly Val His
450 455 460

Ile Asn Ala Gly Pro Glu Ile Gly Val Ala Ser Thr Lys Ala Tyr Thr
465 470 475 480

Ser Gln Phe Ile Ser Leu Val Met Phe Gly Leu Met Met Ser Glu Asp
485 490 495

Arg Ile Ser Leu Gln Asn Arg Arg Gln Glu Ile Ile Arg Gly Leu Arg
500 505 510

```
Ser Leu Pro Glu Leu Ile Lys Glu Val Leu Ser Leu Asp Glu Lys Ile
        515                 520                 525

His Asp Leu Ala Leu Glu Leu Tyr Thr Gln Arg Ser Leu Leu Val Met
    530                 535                 540

Gly Arg Gly Tyr Asn Tyr Ala Thr Cys Leu Glu Gly Ala Leu Lys Ile
545                 550                 555                 560

Lys Glu Ile Thr Tyr Met His Ser Glu Gly Ile Leu Ala Gly Glu Leu
                565                 570                 575

Lys His Gly Pro Leu Ala Leu Val Asp Lys Gln Met Pro Val Ile Met
            580                 585                 590

Val Ile Met Lys Asp Pro Cys Phe Ala Lys Cys Gln Asn Ala Leu Gln
        595                 600                 605

Gln Val Thr Ala Arg Gln Gly Arg Pro Ile Ile Leu Cys Ser Lys Asp
    610                 615                 620

Asp Thr Glu Ser Ser Lys Phe Ala Tyr Lys Thr Ile Glu Leu Pro His
625                 630                 635                 640

Thr Val Asp Cys Leu Gln Gly Ile Leu Ser Val Ile Pro Leu Gln Leu
                645                 650                 655

Leu Ser Phe His Leu Ala Val Leu Arg Gly Tyr Asp Val Asp Phe Pro
            660                 665                 670

Arg Asn Leu Ala Lys Ser Val Thr Val Glu
        675                 680
```

<210> 11

<211> 1830

<212> DNA

<213> Escherichia coli

<220>

<221> CDS

<222> (1)..(1827)

<223>

<300>

<308> U00096.2

<309> 2005-09-08

<313> (3909862)..(3911691)

<400> 11

```
atg tgt gga att gtt ggc gcg atc gcg caa cgt gat gta gca gaa atc       48
Met Cys Gly Ile Val Gly Ala Ile Ala Gln Arg Asp Val Ala Glu Ile
1               5                   10                  15

ctt ctt gaa ggt tta cgt cgt ctg gaa tac cgc gga tat gac tct gcc       96
Leu Leu Glu Gly Leu Arg Arg Leu Glu Tyr Arg Gly Tyr Asp Ser Ala
            20                  25                  30

ggt ctg gcc gtt gtt gat gca gaa ggt cat atg acc cgc ctg cgt cgc      144
Gly Leu Ala Val Val Asp Ala Glu Gly His Met Thr Arg Leu Arg Arg
        35                  40                  45

ctc ggt aaa gtc cag atg ctg gca cag gca gcg gaa gaa cat cct ctg      192
Leu Gly Lys Val Gln Met Leu Ala Gln Ala Ala Glu Glu His Pro Leu
    50                  55                  60

cat ggc ggc act ggt att gct cac act cgc tgg gcg acc cac ggt gaa      240
His Gly Gly Thr Gly Ile Ala His Thr Arg Trp Ala Thr His Gly Glu
65                  70                  75                  80

cct tca gaa gtg aat gcg cat ccg cat gtt tct gaa cac att gtg gtg      288
Pro Ser Glu Val Asn Ala His Pro His Val Ser Glu His Ile Val Val
                85                  90                  95

gtg cat aac ggc atc atc gaa aac cat gaa ccg ctg cgt gaa gag cta      336
Val His Asn Gly Ile Ile Glu Asn His Glu Pro Leu Arg Glu Glu Leu
            100                 105                 110

aaa gcg cgt ggc tat acc ttc gtt tct gaa acc gac acc gaa gtg att      384
Lys Ala Arg Gly Tyr Thr Phe Val Ser Glu Thr Asp Thr Glu Val Ile
        115                 120                 125

gcc cat ctg gtg aac tgg gag ctg aaa caa ggc ggg act ctg cgt gag      432
Ala His Leu Val Asn Trp Glu Leu Lys Gln Gly Gly Thr Leu Arg Glu
    130                 135                 140

gcc gtt ctg cgt gct atc ccg cag ctg cgt ggt gcg tac ggt aca gtg      480
Ala Val Leu Arg Ala Ile Pro Gln Leu Arg Gly Ala Tyr Gly Thr Val
145                 150                 155                 160

atc atg gac tcc cgt cac ccg gat acc ctg ctg gcg gca cgt tct ggt      528
Ile Met Asp Ser Arg His Pro Asp Thr Leu Leu Ala Ala Arg Ser Gly
                165                 170                 175

agt ccg ctg gtg att ggc ctg ggg atg ggc gaa aac ttt atc gct tct      576
Ser Pro Leu Val Ile Gly Leu Gly Met Gly Glu Asn Phe Ile Ala Ser
            180                 185                 190

gac cag ctg gcg ctg ttg ccg gtg acc cgt cgc ttt atc ttc ctt gaa      624
Asp Gln Leu Ala Leu Leu Pro Val Thr Arg Arg Phe Ile Phe Leu Glu
        195                 200                 205

gag ggc gat att gcg gaa atc act cgc cgt tcg gta aac atc ttc gat      672
Glu Gly Asp Ile Ala Glu Ile Thr Arg Arg Ser Val Asn Ile Phe Asp
    210                 215                 220

aaa act ggc gcg gaa gta aaa cgt cag gat atc gaa tcc aat ctg caa      720
Lys Thr Gly Ala Glu Val Lys Arg Gln Asp Ile Glu Ser Asn Leu Gln
225                 230                 235                 240

tat gac gcg ggc gat aaa ggc att tac cgt cac tac atg cag aaa gag      768
```

```
Tyr Asp Ala Gly Asp Lys Gly Ile Tyr Arg His Tyr Met Gln Lys Glu
                245                 250                 255

atc tac gaa cag ccg aac gcg atc aaa aac acc ctt acc gga cgc atc      816
Ile Tyr Glu Gln Pro Asn Ala Ile Lys Asn Thr Leu Thr Gly Arg Ile
            260                 265                 270

agc cac ggt cag gtt gat tta agc gag ctg gga ccg aac gcc gac gaa      864
Ser His Gly Gln Val Asp Leu Ser Glu Leu Gly Pro Asn Ala Asp Glu
        275                 280                 285

ctg ctg tcg aag gtt gag cat att cag atc ctc gcc tgt ggt act tct      912
Leu Leu Ser Lys Val Glu His Ile Gln Ile Leu Ala Cys Gly Thr Ser
    290                 295                 300

tat aac tcc ggt atg gtt tcc cgc tac tgg ttt gaa tcg cta gca ggt      960
Tyr Asn Ser Gly Met Val Ser Arg Tyr Trp Phe Glu Ser Leu Ala Gly
305                 310                 315                 320

att ccg tgc gac gtc gaa atc gcc tct gaa ttc cgc tat cgc aaa tct     1008
Ile Pro Cys Asp Val Glu Ile Ala Ser Glu Phe Arg Tyr Arg Lys Ser
                325                 330                 335

gcc gtg cgt cgt aac agc ctg atg atc acc ttg tca cag tct ggc gaa     1056
Ala Val Arg Arg Asn Ser Leu Met Ile Thr Leu Ser Gln Ser Gly Glu
            340                 345                 350

acc gcg gat acc ctg gct ggc ctg cgt ctg tcg aaa gag ctg ggt tac     1104
Thr Ala Asp Thr Leu Ala Gly Leu Arg Leu Ser Lys Glu Leu Gly Tyr
        355                 360                 365

ctt ggt tca ctg gca atc tgt aac gtt ccg ggt tct tct ctg gtg cgc     1152
Leu Gly Ser Leu Ala Ile Cys Asn Val Pro Gly Ser Ser Leu Val Arg
    370                 375                 380

gaa tcc gat ctg gcg cta atg acc aac gcg ggt aca gaa atc ggc gtg     1200
Glu Ser Asp Leu Ala Leu Met Thr Asn Ala Gly Thr Glu Ile Gly Val
385                 390                 395                 400

gca tcc act aaa gca ttc acc act cag tta act gtg ctg ttg atg ctg     1248
Ala Ser Thr Lys Ala Phe Thr Thr Gln Leu Thr Val Leu Leu Met Leu
                405                 410                 415

gtg gcg aag ctg tct cgc ctg aaa ggt ctg gat gcc tcc att gaa cat     1296
Val Ala Lys Leu Ser Arg Leu Lys Gly Leu Asp Ala Ser Ile Glu His
            420                 425                 430

gac atc gtg cat ggt ctg cag gcg ctg ccg agc cgt att gag cag atg     1344
Asp Ile Val His Gly Leu Gln Ala Leu Pro Ser Arg Ile Glu Gln Met
        435                 440                 445

ctg tct cag gac aaa cgc att gaa gcg ctg gca gaa gat ttc tct gac     1392
Leu Ser Gln Asp Lys Arg Ile Glu Ala Leu Ala Glu Asp Phe Ser Asp
    450                 455                 460

aaa cat cac gcg ctg ttc ctg ggc cgt ggc gat cag tac cca atc gcg     1440
Lys His His Ala Leu Phe Leu Gly Arg Gly Asp Gln Tyr Pro Ile Ala
465                 470                 475                 480

ctg gaa ggc gca ttg aag ttg aaa gag atc tct tac att cac gct gaa     1488
Leu Glu Gly Ala Leu Lys Leu Lys Glu Ile Ser Tyr Ile His Ala Glu
                485                 490                 495

gcc tac gct gct ggc gaa ctg aaa cac ggt ccg ctg gcg cta att gat     1536
Ala Tyr Ala Ala Gly Glu Leu Lys His Gly Pro Leu Ala Leu Ile Asp
            500                 505                 510

gcc gat atg ccg gtt att gtt gtt gca ccg aac aac gaa ttg ctg gaa     1584
Ala Asp Met Pro Val Ile Val Val Ala Pro Asn Asn Glu Leu Leu Glu
```

```
        515                 520                 525

aaa ctg aaa tcc aac att gaa gaa gtt cgc gcg cgt ggc ggt cag ttg      1632
Lys Leu Lys Ser Asn Ile Glu Glu Val Arg Ala Arg Gly Gly Gln Leu
    530                 535                 540

tat gtc ttc gcc gat cag gat gcg ggt ttt gta agt agc gat aac atg      1680
Tyr Val Phe Ala Asp Gln Asp Ala Gly Phe Val Ser Ser Asp Asn Met
545                 550                 555                 560

cac atc atc gag atg ccg cat gtg gaa gag gtg att gca ccg atc ttc      1728
His Ile Ile Glu Met Pro His Val Glu Glu Val Ile Ala Pro Ile Phe
                565                 570                 575

tac acc gtt ccg ctg cag ctg ctg gct tac cat gtc gcg ctg atc aaa      1776
Tyr Thr Val Pro Leu Gln Leu Leu Ala Tyr His Val Ala Leu Ile Lys
            580                 585                 590

ggc acc gac gtt gac cag ccg cgt aac ctg gca aaa tcg gtt acg gtt      1824
Gly Thr Asp Val Asp Gln Pro Arg Asn Leu Ala Lys Ser Val Thr Val
        595                 600                 605

gag taa                                                              1830
Glu
```

<210> 12

<211> 609

<212> PRT

<213> Escherichia coli

<400> 12

```
Met Cys Gly Ile Val Gly Ala Ile Ala Gln Arg Asp Val Ala Glu Ile
1               5                   10                  15

Leu Leu Glu Gly Leu Arg Arg Leu Glu Tyr Arg Gly Tyr Asp Ser Ala
            20                  25                  30

Gly Leu Ala Val Val Asp Ala Glu Gly His Met Thr Arg Leu Arg Arg
        35                  40                  45

Leu Gly Lys Val Gln Met Leu Ala Gln Ala Ala Glu Glu His Pro Leu
    50                  55                  60

His Gly Gly Thr Gly Ile Ala His Thr Arg Trp Ala Thr His Gly Glu
65                  70                  75                  80

Pro Ser Glu Val Asn Ala His Pro His Val Ser Glu His Ile Val Val
                85                  90                  95

Val His Asn Gly Ile Ile Glu Asn His Glu Pro Leu Arg Glu Glu Leu
            100                 105                 110

Lys Ala Arg Gly Tyr Thr Phe Val Ser Glu Thr Asp Thr Glu Val Ile
        115                 120                 125
```

```
Ala His Leu Val Asn Trp Glu Leu Lys Gln Gly Gly Thr Leu Arg Glu
    130                 135                 140

Ala Val Leu Arg Ala Ile Pro Gln Leu Arg Gly Ala Tyr Gly Thr Val
145                 150                 155                 160

Ile Met Asp Ser Arg His Pro Asp Thr Leu Leu Ala Ala Arg Ser Gly
                165                 170                 175

Ser Pro Leu Val Ile Gly Leu Gly Met Gly Glu Asn Phe Ile Ala Ser
            180                 185                 190

Asp Gln Leu Ala Leu Leu Pro Val Thr Arg Arg Phe Ile Phe Leu Glu
        195                 200                 205

Glu Gly Asp Ile Ala Glu Ile Thr Arg Arg Ser Val Asn Ile Phe Asp
    210                 215                 220

Lys Thr Gly Ala Glu Val Lys Arg Gln Asp Ile Glu Ser Asn Leu Gln
225                 230                 235                 240

Tyr Asp Ala Gly Asp Lys Gly Ile Tyr Arg His Tyr Met Gln Lys Glu
                245                 250                 255

Ile Tyr Glu Gln Pro Asn Ala Ile Lys Asn Thr Leu Thr Gly Arg Ile
            260                 265                 270

Ser His Gly Gln Val Asp Leu Ser Glu Leu Gly Pro Asn Ala Asp Glu
        275                 280                 285

Leu Leu Ser Lys Val Glu His Ile Gln Ile Leu Ala Cys Gly Thr Ser
    290                 295                 300

Tyr Asn Ser Gly Met Val Ser Arg Tyr Trp Phe Glu Ser Leu Ala Gly
305                 310                 315                 320

Ile Pro Cys Asp Val Glu Ile Ala Ser Glu Phe Arg Tyr Arg Lys Ser
                325                 330                 335

Ala Val Arg Arg Asn Ser Leu Met Ile Thr Leu Ser Gln Ser Gly Glu
            340                 345                 350

Thr Ala Asp Thr Leu Ala Gly Leu Arg Leu Ser Lys Glu Leu Gly Tyr
        355                 360                 365

Leu Gly Ser Leu Ala Ile Cys Asn Val Pro Gly Ser Ser Leu Val Arg
    370                 375                 380

Glu Ser Asp Leu Ala Leu Met Thr Asn Ala Gly Thr Glu Ile Gly Val
385                 390                 395                 400
```

Ala Ser Thr Lys Ala Phe Thr Thr Gln Leu Thr Val Leu Leu Met Leu
405 410 415

Val Ala Lys Leu Ser Arg Leu Lys Gly Leu Asp Ala Ser Ile Glu His
420 425 430

Asp Ile Val His Gly Leu Gln Ala Leu Pro Ser Arg Ile Glu Gln Met
435 440 445

Leu Ser Gln Asp Lys Arg Ile Glu Ala Leu Ala Glu Asp Phe Ser Asp
450 455 460

Lys His His Ala Leu Phe Leu Gly Arg Gly Asp Gln Tyr Pro Ile Ala
465 470 475 480

Leu Glu Gly Ala Leu Lys Leu Lys Glu Ile Ser Tyr Ile His Ala Glu
485 490 495

Ala Tyr Ala Ala Gly Glu Leu Lys His Gly Pro Leu Ala Leu Ile Asp
500 505 510

Ala Asp Met Pro Val Ile Val Val Ala Pro Asn Asn Glu Leu Leu Glu
515 520 525

Lys Leu Lys Ser Asn Ile Glu Glu Val Arg Ala Arg Gly Gly Gln Leu
530 535 540

Tyr Val Phe Ala Asp Gln Asp Ala Gly Phe Val Ser Ser Asp Asn Met
545 550 555 560

His Ile Ile Glu Met Pro His Val Glu Glu Val Ile Ala Pro Ile Phe
565 570 575

Tyr Thr Val Pro Leu Gln Leu Leu Ala Tyr His Val Ala Leu Ile Lys
580 585 590

Gly Thr Asp Val Asp Gln Pro Arg Asn Leu Ala Lys Ser Val Thr Val
595 600 605

Glu

<210> 13

<211> 1830

<212> DNA

<213> Artificial Sequence

<220>

<223> Synthetic sequence encoding an Escherichia coli protein having th e activity of a GFAT

<400> 13

```
atgtgcggaa ttgttggtgc tatcgcccaa agagacgttg ctgagatttt gttagagggt     60
ctgcgaaggc tagagtatag aggatatgac tccgctggtc tggctgtcgt tgatgctgag    120
ggtcatatga caaggctaag aaggttagga aaggttcaga tgcttgctca ggcagctgag    180
gaacatccat tgcatggagg tactggtatt gcacatacca ggtgggctac tcatggggag    240
ccatcagaag ttaatgctca tccacatgtg agtgagcata tcgttgtagt tcacaatggg    300
ataattgaaa accacgaacc attgagggaa gagttaaagg caagaggata tacttttgtg    360
agtgagactg acactgaggt tattgcacat ttagtgaact gggaactcaa acaggggggc    420
acattgcgtg aggctgtgtt aagagctatt cctcaactta gaggtgcata cggtactgtt    480
attatggatt caagacaccc agatactctc cttgcagcta gatcaggtag tcccttggtc    540
ataggacttg gaatgggtga aaattttatc gctagcgacc aattggcctt attgccagtt    600
acaagacgat ttattttcct tgaagagggc gatattgctg agattactag aaggtctgtg    660
aacatctttg ataagactgg cgctgaggtt aaacgtcagg atatcgagtc taaccttcaa    720
tacgatgctg gtgataaagg aatttacagg cattatatgc aaaaggaaat ttatgaacaa    780
ccaaatgcta tcaaaaacac acttactggc cgtatttctc atggacaggt cgatttaagc    840
gagcttggtc ctaatgcaga cgaactgcta tcaaaagttg agcacataca gatactggca    900
tgcggaacta gttataattc aggaatggtc tctagatact ggttcgaaag cttggcaggt    960
ataccttgtg atgtagagat cgcttctgag tttaggtata gaaagtctgc tgtgcgtaga   1020
aattcattaa tgattacatt atctcaatcc ggagaaacag cagatacact ggctggattg   1080
aggctttcta aggaactcgg atatctgggt tcacttgcta tttgtaatgt accaggttcc   1140
tcattggttc gtgaatcaga tctagcactt atgacaaatg caggaactga aataggtgtg   1200
gcaagtacca aggctttcac aacccaactg accgtacttt taatgttggt agcaaaactc   1260
agtcgattaa aggggctaga tgcatctatc gaacatgata ttgttcacgg gcttcaagct   1320
ctcccttcaa gaattgaaca aatgctttca caagataaga gaatagaggc attggctgaa   1380
gatttttccg acaaacatca cgcattgttt cttggacgtg gcgatcaata tccaattgca   1440
ttggaaggag ctttgaagtt gaaagaaata agttacattc acgcagaagc atatgcagct   1500
ggagaactca agcatggtcc tttggcactc atcgacgctg acatgcccgt gatcgtagtg   1560
gctcctaata acgaactgct cgaaaagctt aaatcaaata tcgaagaggt tcgagctaga   1620
ggaggtcagc tttacgtttt cgctgaacaa gatgctggat tcgtgtcaag cgataatatg   1680
catataattg aaatgcctca cgttgaagaa gtgattgcac ctatatttta tacagtccca   1740
ttgcaacttc tagcttacca tgttgcactt attaaaggaa ctgatgttga tcagcctaga   1800
aacctagcaa aatctgtaac agtcgaataa                                    1830
```

## Beschreibung

Die vorliegende Erfindung betrifft Pflanzenzellen und Pflanzen, die eine erhöhte Menge an Hyaluronan synthetisieren, sowie Verfahren zur Herstellung solcher Pflanzen, als auch Verfahren zur Herstellung von Hyaluronan mit Hilfe dieser Pflanzenzellen oder Pflanzen. Erfindungsgemäße Pflanzenzellen oder genetisch modifizierte Pflanzen weisen dabei die Aktivität einer Hyaluronansynthase und zusätzlich eine erhöhte Aktivität einer Glutamin:Fructose-6-Phosphat Amidotransferase (GFAT) und eine erhöhte Aktivität einer UDP-Glucose Dehydrogenase (UDP-Glc-DH) im Vergleich zu Wildtyp Pflanzenzellen bzw. Wildtyp Pflanzen auf. Weiterhin betrifft die vorliegende Erfindung die Verwendung von Pflanzen mit gesteigerter Hyaluronansynthese zur Herstellung von Hyaluronan und Lebens- oder Futtermitteln, die Hyaluronan enthalten.

Hyaluronan ist ein in der Natur vorkommendes, unverzweigtes, lineares Mucopolysaccharid (Glucosaminoglucan), welches aus alternierenden Molekülen von Glucuronsäure und N-Acetyl-Glucosamin zusammengesetzt ist. Der Grundbaustein von Hyaluronan besteht aus dem Disaccharid Glucuronsäure-beta-1,3-N-Acetyl-Glucosamin. Diese Wiederholungseinheit ist im Hyaluronan durch beta-1,4-Verknüpfungen miteinander verbunden.
Im Bereich der Pharmazie wird häufig der Begriff Hyaluronsäure verwendet. Da Hyaluronan meist als Polyanion und nicht als freie Säure vorliegt, wird im Folgenden der Begriff Hyaluronan bevorzugt verwendet, wobei beide Molekülformen von der jeweiligen Bezeichnung als umfasst gelten.

Hyaluronan besitzt außergewöhnliche physico-chemische Eigenschaften, wie z.B. Eigenschaften von Polyelektrolyten, viskoelastische Eigenschaften, eine hohe Wasserbindekapazität, Eigenschaften der Gelbildung, die, neben weiteren Eigenschaften von Hyaluronan, in einem Übersichtsartikel von Lapcik et al. (1998, Chemical Reviews 98(8), 2663-2684) beschrieben sind.

Hyaluronan ist Bestandteil von extrazellulärem Bindegewebe und Körperflüssigkeiten von Vertebraten. Beim Menschen wird Hyaluronsäure von der Zellmembran aller Körperzellen, vor allem der mesenchymalen Zellen, synthetisiert und kommt ubiquitär im Körper vor, mit einer besonders hohen Konzentration in den Bindegeweben, der extrazellulären Matrix, der Nabelschnur, der Gelenkflüssigkeit, dem Knorpelgewebe, der Haut und dem Glaskörper des Auges (Bernhard Gebauer, 1998, Inaugural-Dissertation, Virchow-Klinikum Medizinische Fakultät Charité der Humboldt Universität zu Berlin; Fraser et al., 1997, Journal of Internal Medicine 242, 27-33). Kürzlich konnte Hyaluronan auch in tierischen nicht-Vertebraten Organismen (Mollusken) nachgewiesen werden (Volpi und Maccari, 2003, Biochimie 85, 619-625).
Weiterhin synthetisieren einige human pathogene gram-positive Bakterien (Gruppe A und C *Streptococcus*) und gram-negative Bakterien (*Pasteurella*) Hyaluronan als Exopolysaccharide, die diese Bakterien vor dem Zugriff des Immunsystems ihres Wirtes bewahren, da Hyaluronan eine nicht immunogene Substanz darstellt.
Viren, welche einzellige und teilweise als Endosymbionten in *Paramecium* Spezies vorkommende Grünalgen der Gattung *Chlorella* infizieren, vermitteln den einzelligen Grünalgen nach Virusinfektion die Fähigkeit, Hyaluronan zu synthetisieren (Graves et al., 1999, Virology 257, 15-23). Jedoch ist die Fähigkeit zur Hyaluronsynthese kein Merkmal, dass den betreffenden Algen zuzuordnen ist. Die Fähigkeit der Algen, Hyaluronan zu synthetisieren wird durch eine Infektion eines Virus, dessen Genom eine Hyaluronansynthase codierende Sequenz aufweist, vermittelt (DeAngelis, 1997, Science 278, 1800-1803). Weiterhin enthält das Virus Genom Sequenzen, codierend für eine UDP-Glucose-Dehydrogenase (UDP-Glc-DH) und eine Glutamine:Fructose-6-Phosphat-Amidotransferase (GFAT). UDP-Glc-DH katalysiert die Synthese von UDP-Glucuronsäure, das von der Hyaluronansynthase als Substrat verwendet wird. GFAT konvertiert Fructose-6-Phosphat zu Glucosamin-6-Phosphat, das ein Metabolit im Stoffelwechselweg für die Hyaluronansynthese in z.B. Bakterien darstellt. Beide Gene codieren Proteine, die, wie auch die Hyaluronansynthase des Virus, in der frühen Phase der Virusinfektion transkribiert werden. (DeAngelis et al., 1997, Science 278, 1800-1803, Graves et al., 1999, Virology 257, 15-23). Die Aktivität eines Proteins mit der Aktivität einer Glutamin:Fructose-6-Phosphat Amidotransferase (GFAT) konnte weder in Extrakten, die aus nicht mit einem Virus infizierten Zellen, noch aus mit Virus infizierten Zellen nachgewiesen werden (Landstein et al., 1998, Virology 250, 388-396). Welche Rolle die Expression von UDP-Glc-DH und GFAT in Virus infizierten Chlorella Zellen für die Hyaluronansynthese spielt und ob sie für die Hyaluronansynthese benötigt werden, ist daher nicht bekannt.
Natürlich vorkommende Pflanzen selbst weisen in ihrem Genom keine Nucleinsäuren auf, die Proteine codieren, welche die Synthese von Hyaluronan katalysieren und, obwohl eine Vielzahl an pflanzlichen Kohlenhydraten beschrieben und charakterisiert wurden, konnten bisher weder Hyaluronan noch dem Hyaluronan verwandte Moleküle in nicht infizierten, natürlich vorkommenden Pflanzen nachgewiesen werden (Graves et al., 1999, Virology 257, 15-23).

Die Katalyse der Hyaluronansynthese wird durch ein einziges, Membran integriertes oder Membran assoziiertes Enzym, der Hyaluronansynthase bewerkstelligt. Die bisher untersuchten Hyaluronansynthasen können in zwei Gruppen eingeteilt werden: Hyaluronansynthasen der Klasse I und Hyaluronansynthasen der Klasse II (DeAngelis, 1999, CMLS, Cellular and Molecular Life Sciences 56, 670-682). Eine weitere Unterscheidung der Hyaluronansynthasen aus Vertebraten erfolgt an Hand der identifizierten Isoenzyme. Die verschiedenen Isoenzyme werden in der Reihenfolge ihrer Identifizierung mit arabischen Nummern bezeichnet (z.B. hsHAS1, hsHAS2, hsHAS3).

Der Mechanismus des Transfers von synthetisierten Hyaluronanmolekülen über die Cytoplasmamembran hinweg in das die Zelle umgebende Medium, ist noch nicht vollständig geklärt. Frühere Hypothesen, gingen davon aus, dass der Transport über die Zellmembran hinweg von der Hyaluronansynthase selbst bewerkstelligt würde. Neuere Ergebnisse deuten jedoch darauf hin, dass der Transport von Hyaluronanmolekülen über die Cytoplasmamembran durch einen energieabhängigen Transport mittels dafür zuständiger Transportproteine stattfindet. So wurden durch Mutagenese *Streptococcus* Stämme erzeugt, bei welchen die Synthese eines aktiven Transportproteins inhibiert war. Diese Stämme synthetisierten weniger Hyaluronan als entsprechende Wildtyp Bakterienstämme (Ouskova et al., 2004, Glycobiology 14(10), 931-938). In humanen Fibroblastenzellen konnte mit Hilfe von auf bekannte Transportproteine spezifisch inhibierend wirkenden Agentien gezeigt werden, dass sowohl die Menge an produziertem Hyaluronan, als auch die Aktivität von Hyaluronansynthasen reduziert werden kann (Prehm und Schumacher, 2004, Biochemical Pharmacology 68, 1401-1410). Ob und wenn überhaupt, in welcher Menge Transportproteine, die Hyaluronan transportieren können, in Pflanzen vorliegen, ist nicht bekannt.

Die außergewöhnlichen Eigenschaften des Hyaluronans eröffnen eine Vielfalt an Möglichkeiten für die Anwendung auf verschiedensten Gebieten, wie z.B. der Pharmazie, der Kosmetikindustrie, in der Lebens- und Futtermittel Herstellung, bei technischen Anwendungen (z.B: als Schmierstoffe) etc.. Die wichtigsten Anwendungen, bei denen Hyaluronan zurzeit verwendet wird, liegen im medizinischen und kosmetischen Bereich (siehe z.B. Lapcik et al., 1998, Chemical Reviews 98(8), 2663-2684, Goa und Benfield, 1994, Drugs 47(3), 536-566).
Im medizinischen Bereich werden Hyaluronan enthaltende Produkte derzeit zur intraartikulären Arthrosebehandlung sowie bei den Ophthalmika, die bei Operationen am Auge zum Einsatz kommen, verwendet. Auch für die Behandlung von Gelenkerkrankungen bei Rennpferden wird Hyaluronan eingesetzt. Daneben ist Hyaluronsäure Bestandteil einiger Rhinologica, die z.B. in Form von Augentropfen und Nasalia der Befeuchtung trockener Schleimhäute dienen. Hyaluronan enthaltende Injektionslösungen werden als Analgetika und Antirheumatica verwendet. Hyaluronan oder derivatisiertes Hyaluronan enthaltenden Auflagen werden bei der Wundheilung eingesetzt. Als Dermatika werden Hyaluronan enthaltende Gelimplantate zur Korrektur von Hautdeformationen in der plastischen Chirugie verwendet.
Für pharmakologische Anwendungen wird Hyaluronan mit einem hohen Molekulargewicht bevorzugt.
In der Schönheitsmedizin zählen Hyaluronpräparate zu den geeigneten Hautfüllmaterialien. Durch Einspritzen von Hyaluronan erreicht man für eine begrenzte Zeit die Glättung von Falten oder ein vergrößertes Lippenvolumen.
In kosmetischen Produkten, insbesondere in Hautcremes und Lotionen findet Hyaluronan wegen seiner hohen Wasserbindungskapazität häufig Anwendung als Feuchtigkeitsspender.

Weiterhin werden Hyaluronan enthaltende Präparate als so genannte Nutraceuticals (Nahrungsergänzungsmittel) vertrieben, die auch bei Tieren (z.B. Hunde, Pferde) zur Vorbeugung und Linderung von Arthrose angewandt werden.

Für gewerbliche Zwecke verwendetes Hyaluronan wird zurzeit aus tierischen Geweben (Hahnenkämmen) isoliert oder fermentativ mittels Bakterienkulturen hergestellt.
Ein Verfahren zur Isolierung von Hyaluronan aus Hahnenkämmen oder alternativ aus Nabelschnüren ist beschrieben in US 4,141,973. Neben Hyaluronan kommen in tierischen Geweben (z.B. Hahnenkämme, Nabelschnüre) noch weitere, mit Hyaluronan verwandte Mucopolysaccharide, wie Chondrotinsulfat, Dermatansulfat, Keratansulfat, Heparansulfat und Heparin vor. Tierische Organismen weisen weiterhin Proteine (Hyaladherine) auf, die spezifisch an Hyaluronan binden und für verschiedenste Funktionen im Organismus, wie z.B. dem Abbau von Hyaluronan in der Leber, der Funktion von Hyaluronan als Leitstruktur für die Zellwanderung, die Regulierung der Endocytose, die Verankerung von Hyaluronan an der Zelloberfläche oder die Ausbildung von Hyaluronan Netzwerken notwendig sind (Turley, 1991, Adv Drug Delivery Rev 7, 257 ff.; Laurent und Fraser, 1992, FASEB J. 6, 183 ff.; Stamenkovic und Aruffo, 1993, Methods Enzymol. 245, 195 ff; Knudson und Knudson, 1993, FASEB 7, 1233 ff.).

Die zur bakteriellen Produktion von Hyaluronan verwendeten *Streptococcus* Stämme gehören ausschließlich zu den human pathogenen Bakterien. Diese Bakterien produzieren auch beim Kultivieren (pyrogene) Exotoxine und Haemolysine (Streptolysin, (insbesondere alpha- und beta-Haemolysin) (Kilian, M.: Streptococcus and Enterococcus. In: Medical Microbiology. Greenwood, D.; Slack, RCA; Peutherer, J.F. (Eds.). Kapitel 16. Churchill Livingstone, Edinburgh, UK: pp. 174-188, 2002, ISBN 0443070776), die ins Kulturmedium abgegeben werden. Dieses erschwert die Reinigung und Isolierung des Hyaluronans, welches mit Hilfe von *Streptococcus* Stämmen hergestellt wurde. Besonders für phamazeutische Anwendungen stellt das Vorliegen von Exotoxinen und Haemolysinen in den Präparaten ein Problem dar.

US 4,801,539 beschreibt die Herstellung von Hyaluronan durch Fermentation eines mutagenisierten Bakterienstammes *(Streptococcus zooedemicus).* Der verwendete mutagenisierte Bakterienstamm synthetisiert kein beta-Haemolysin mehr. Es wurde dabei eine Ausbeute von 3,6 g Hyaluronan pro Liter Kultur erreicht.
EP 0694616 beschreibt ein Verfahren zur Kultivierung von *Streptococcus zooedemicus* oder *Streptococcus equi,* worin unter den verwendeten Kulturbedingungen kein Streptolysin, aber erhöhte Mengen an Hyaluronan synthetisiert werden. Es wurde dabei eine Ausbeute von 3,5 g Hyaluronan pro Liter Kultur erreicht.
*Streptococcus* Stämme geben während der Kultur das Enzym Hyaluronidase in das Kulturmedium ab, was dazu führt, dass auch bei diesem Produktionssystem das Molekulargewicht während der Aufreinigung reduziert wird. Die Verwendung von Hyaluronidase negativen *Streptococcus* Stämmen oder von Produktionsverfahren, bei welchen die Produktion von Hyaluronidase während der Kultur inhibiert wird, zur Produktion von Hyaluronan, sind in US 4,782,046 beschrieben. Es wurde dabei eine Ausbeute von bis zu 2,5 g Hyaluronan pro Liter Kultur erreicht, wobei ein maximales mittleres Molekulargewicht von $3,8x10^6$ Da bei einer Molekulargewichtsverteilung von $2,4x10^6$ bis $4,0x10^6$ erhalten wurde.
US 20030175902 und WO 03 054163 beschreiben die Herstellung von Hyaluronan mit Hilfe der heterologen Expression einer Hyaluronansynthase aus *Streptococcus equisimilis* in *Bacillus subtilis.* Um die Produktion von ausreichenden Mengen an Hyaluronan zu erlangen ist neben der heterologen Expression einer Hyaluronansynthase auch die gleichzeitige Expression einer UDP-Glucose-Dehydrogenase in den *Bacillus* Zellen notwendig. Die absolute Menge an Hyaluronan, die bei der Produktion mit Hilfe von *Bacillus subtilis* erhalten wird, ist in US 20030175902 und WO 03 054163 nicht angegeben. Es wurde ein maximales mittleres Molekulargewicht von ca. $4,2x10^6$ Da erhalten. Dieses mittlere Molekulargewicht wurde jedoch nur für den rekombinanten *Bacillus* Stamm erhalten, bei welchem ein Gen codierend das Hyaluronansynthase Gen aus *Streptococcus equisimilis* und das Gen codierend die UDP-Glucose-Dehydrogenase aus *Bacillus subtilis* unter Kontrolle des *amy*Q Promotors in das *Bacillus subtilis* Genom integriert war und gleichzeitig das *Bacillus subtilis* endogene cxpY Gen (codierend eine P450 Cytochromoxidase) inaktiviert war.

WO 05 012529 beschreibt die Herstellung von transgenen Tabakpflanzen, die mit Nucleinsäuremolekülen, codierend Hyaluronansynthasen aus *Chlorella* infizierenden Viren transformiert wurden. In WO 05 012529 wurden zum einen Nucleinsäuresequenzen, codierend Hyaluronansynthase des Chlorellavirus CVHI1-Stammes und zum anderen des Chlorellavirus CVKA1-Stammes für die Transformation von Tabakpflanzen verwendet. Die Synthese von Hyaluronan konnte nur für eine Pflanze, die nach Transformation mit einer Nucleinsäuresequenz, codierend eine Hyaluronansynthase, die aus dem Chlorellavirus CVKA1-Stamm isoliert wurde, nachgewiesen werden. Für Tabakpflanzen, die mit einer Nucleinsäuresequenz, codierend eine Hyaluronansynthase, die aus dem Chlorellavirus CVH11-Stamm isoliert wurde, konnte keine Hyaluronansynthese in den entsprechenden transgenen Pflanzen nachgewiesen werden. Die Menge an Hyaluronan, die von der einzigen in WO 05 012529 erhaltenen, Hyaluronan produzierenden transgenen Tabakpflanze synthetisiert wurde, ist mit ca. 4,2 μg Hyaluronan pro ml Messvolumen angegeben was unter Berücksichtigung der Beschreibung zur Durchführung des betreffenden Experiments etwa einer Menge von maximal 12 μg an produziertem Hyaluronan pro Gramm Frischgewicht an Pflanzenmaterial entspricht.

Hyaluronansynthase katalysiert die Synthese von Hyaluronan ausgehend von den Edukten UDP-N-Acetyl Glucosamin und UDP-Glucuronsäure. Beide genannten Edukte kommen in pflanzlichen Zellen vor.
UDP-Glucuronsäure dient in pflanzlichen Zellen als Metabolit für einen von mehreren möglichen Synthesewegen der Ascorbinsäure (Lorence et al., 2004, Plant Physiol 134, 1200-1205) und als zentrales Metabolit für die Synthese der Zellwandbestandteile Pektin und Hemicellulose, die im Endoplasmatischen Reticulum der Pflanzenzelle synthetisiert werden (Reiter, 1998, Plant Physiol Biochem 36(1), 167-176). Das wichtigste und am häufigsten vorkommende Monomer des Pektins stellt die D-Galacturonsäure dar (2004, H. W. Heldt in "Plant Biochemistry", 3rd Edition, Academic Press, ISBN 0120883910), welche unter Verwendung von UDP-Glucuronsäure synthetisiert wird. Weiterhin können u.a. auch UDP-Xylose, UDP-Arabinose, UDP-Galacturonsäure und UDP-Apiose, Metabolite für die Synthese von Hemicellulose und Pektin, unter Verwendung von UDP-Glucuronsäure synthetisiert werden (Seitz et al., 2000, Plant Journal, 21(6), 537-546). UDP-Glucuronsäure kann in pflanzlichen Zellen entweder mittels des Hexosephosphat Stoffwechsels, umfassend u.a. die Umsetzung von UDP-Glucose zu UDP-Glucuronsäure durch UDP-Glc-DH oder mittels des oxidativen *myo*-Inositol Stoffwechselweges, umfassend die Umsetzung von Glucuronat-1-Phosphat zu UDP-Glucuronsäure mittels Glucuronat-1-Phosphat Uridilyltransferase, synthetisiert werden. Beide Stoffwechselwege zur Synthese von Glucuronsäure scheinen unabhängig voneinander, alternativ in unterschiedlichen Geweben/Entwicklungsstadien von *Arabidopsis* Pflanzen zu existieren (Seitz et al., 2000, Plant Journal 21(6), 537-546). Welchen Beitrag die beiden genannten Stoffwechselwege (Hexosephosphat- oder oxidativer *myo*-Inositol-Stoffwechselweg) jeweils bezüglich der Synthese von UDP-Glucuronsäure leisten, ist noch nicht geklärt (Kärkönen, 2005, Plant Biosystems 139(1), 46-49).
Das Enzym UDP-Glc-DH katalysiert die Umsetzung von UDP-Glucose zu UDP-Glucuronsäure. Samac et al. (2004, Applied Biochemistry and Biotechnology 113-116, Humana Press, Edittor Ashok Mulehandani, 1167-1182) beschreiben die gewebespezifische Überexpression einer UDP-Glc-DH aus Sojabohne in Phloemzellen von *Alfalfa* mit dem Ziel, den Pektingehalt in Stengeln dieser Pflanzen zu erhöhen. Obwohl die Aktivität der UDP-Glc-DH um mehr als 200% gegenüber entsprechenden Wildtyp Pflanzen gesteigert werden konnte, war die Menge an Pektin die von entsprechenden Pflanzen produziert wurde, geringer, als die Menge an Pektin, die von entsprechenden Wildtyp Pflanzen produziert wurde. Die Menge an Xylose und Rhamnose Monomeren in der Zellwandfraktion der betreffenden transgenen Pflanzen war erhöht, wohingegen die Menge an Mannose Monomeren in der Zellwandfraktion verringert war.
Die konstitutive Überexpression einer UDP-Glc-DH in *Arabidosis* Pflanzen führte dazu, dass die betreffenden Pflanzen im Vergleich mit entsprechenden Wildtyp Pflanzen ein aberrantes Wachstum zeigten und einen Zwerg (dwarf) Phänotyp aufwiesen. Die Zellwandfraktion der entsprechenden Pflanzen wies eine erhöhte Menge an Mannose und Galactose und eine verringerte Menge an Xylose, Arabinose und Uronsäuren im Vergleich zu entsprechenden Wildtyp Pflanzen auf ( Roman, 2004, "Studies on The Role of UDP-Glc-DH in Polysaccharide Biosynthesis", Dissertation, Acta Universitatis Upsaliensis, ISBN 91-554-6088-7, ISSN 0282-7476). Diese Ergebnisse stehen also zumindest teilweise im Widerspruch zu den Ergebnissen von Samac et al. (2004, Applied Biochemistry and Biotechnology 113-116, Humana Press, Edittor Ashok Mulehandani, 1167-1182), die eine verringerte Menge an Mannose und eine erhöhte Menge an Xyolse in der Zellwandfraktion entsprechender transgener Pflanzen nachgewiesen hatten.

WO 98 35047 beschreibt für die Synthese von UDP-N-Acetylglucosamin in Pflanzenzellen einen Stoffwechselweg, wobei Glucosamin, welches durch mehrere aufeinander folgende enzymatisch katalysierte Reaktionsschritte unter Bildung der Metabolite N-Acetylglucosamin, N-Acetyl-Glucosamin-6-Phosphat, N-Acetyl-Glucosamin-1-Phosphat zu UDP-N-Acetylglucosamin umgesetzt wird. Ein alternativer Stoffwechselweg beinhaltet die Umsetzung von Fructose-6-Phosphat und Glutamin zu Glucosamin-6-Phosphat, welches anschließend durch mehrere aufeinander folgende enenzymatisch katalysierte Reaktionsschritte unter Bildung der Metabolite Glucosamin-1-Phosphat und N-Acetylglucosamin-1-Phosphat zu UDP-N-Acetylglucosamin umgesetzt wird. Die Umsetzung von Fructose-6-Phosphat und Glutamin zu Glucosamin-6-Phosphat wird durch ein Protein mit der Aktivität einer Glutamin:Fructose-6-Phosphat Amidotransferase (GFAT) katalysiert (Mayer et al., 1968, Plant Physiol. 43, 1097-1107).

WO 00 11192 beschreibt die endosperm-spezifische Überexpression eines Nucleinsäuremoleküls aus Mais codierend ein Protein mit der enzymatischen Aktivität einer GFAT in transgenen Maispflanzen mit dem Ziel, eine kationische Stärke in Pflanzen zu synthetisieren, die 2-Amino Anhydroglucose Moleküle aufweist. Der beschriebene Stoffwechselweg, welcher gemäß der Beschreibung von WO 00 11192 zum Einbau von 2-Amino-Anhydroglucose in die Stärke führen soll, umfasst u.a. den Einbau von UDP-Glucosamin mittels Stärke- und/oder Glycogensynthasen in die Stärke. In Mehl aus Endosperm der betreffenden transgenen Maispflanzen, die ein Nucleinsäuremolekül codierend ein Protein mit der enzymatischen Aktivität einer GFAT translational fusioniert mit einem plastidären Signalpeptid überexprimierten, konnten angeblich erhöhte Mengen an UDP-Glucosamin nachgewiesen werden. Wurde das Protein mit der enzymatischen Aktivität einer GFAT ohne Signalpeptid exprimiert, so konnten in den entsprechenden Mehlen aus Mais Endosperm Gewebe eine erhöhte Menge an Glucosamin-1-Phosphat nachgewiesen werden. Kationische Stärke konnte in den transgenen Pflanzen nicht nachgewiesen werden.

Die Produktion von Hyaluronan mit Hilfe der Fermentation von Bakterien Stämmen ist mit hohen Kosten verbunden, da die Bakterien in geschlossenen, sterilen Behältnissen, unter aufwendigen, kontrollierten Kulturbedingungen (siehe z.B. US 4,897,349) fermentiert werden müssen. Weiterhin ist die Menge an Hyaluronan, die mittels Fermentation von Bakterien Stämmen produziert werden kann, beschränkt durch die jeweils bestehenden Produktionsanlagen. Dabei ist auch zu berücksichtigen, dass Fermenter wegen physikalischer Gesetzmäßigkeiten nicht für übermäßig große Kulturvolumen gebaut werden können. Insbesondere ist hier die für eine effiziente Produktion gleichmäßige Durchmischung von durch von außen zugeführten Substanzen (z.B. essentielle Nährstoffquellen für Bakterien, Reagenzien zur pH Regulierung, Sauerstoff) mit dem Kulturmedium zu erwähnen, die in großen Fermentern, wenn überhaupt, nur mit großem technischen Aufwand gewährleistet werden kann.

Das Vorhandensein von anderen Mucopolysacchariden und von spezifisch an Hyaluronan bindenden Proteinen in tierischen Geweben macht die Aufreinigung von Hyaluronan aus tierischen Organismen aufwendig. Die Verwendung von Hyaluronan haltigen medizinischen Präparaten, die mit tierischen Proteinen verunreinigt sind, kann bei Patienten zu unerwünschten immunologischen Reaktionen des Körpers führen (US 4,141,973), insbesondere dann, wenn der Patient eine Allergie gegen tierische Proteine (z.B. Hühnereiweiß) aufweist. Weiterhin sind die Mengen (Ausbeuten) an Hyaluronan, die aus tierischen Geweben in ausreichender Qualität und Reinheit gewonnen werden können, gering (Hahnenkamm: 0,079% w/w, EP 0144019, US 4,782,046), was dazu führt, dass große Mengen an tierischen Geweben verarbeitet werden müssen. Ein weiteres Problem bei der Isolierung von Hyaluronan aus tierischen Geweben besteht darin, dass das Molekulargewicht des Hyaluronans während der Aufreinigung verringert wird, weil tierische Gewebe auch ein Hyaluronan abbauendes Enzym (Hyaluronidase) aufweisen.

*Streptococcus* Stämme produzieren neben den bereits erwähnten Hyaluronidasen und Exotoxinen auch Endotoxine, die, wenn in phamakologischen Produkten vorhanden, Risiken für die Gesundheit des Patienten darstellen. In einer wissenschaftlichen Studie wurde gezeigt, dass selbst auf dem Markt befindliche Hyaluronan enthaltende medizinische Präparate noch nachweisbare Mengen an bakteriellen Endotoxinen aufweisen (Dick et al., 2003, Eur J Opthalmol. 13(2), 176-184). Ein weiterer Nachteil des mit Hilfe von *Streptococcus* Stämmen produzierten Hyaluronans besteht darin, dass das isolierte Hyaluronan ein geringeres Molekulargewicht aufweist, als Hyaluronan, welches aus Hahnenkämmen isoliert wurde (Lapcik et al. 1998, Chemical Reviews 98(8), 2663-2684). US 20030134393 beschreibt die Verwendung eines *Streptococcus* Stammes zur Produktion von Hyaluronan, welcher eine besonders ausgeprägte Hyaluronan Kapsel (supercapsulated) synthetisiert. Das nach Fermentation isolierte Hyaluronan wies ein Molekulargewicht von $9{,}1\times10^{6}$ Da auf. Jedoch betrug die Ausbeute lediglich 350 mg pro Liter.

Einige der Nachteile der Produktion von Hyaluronan mittels bakterieller Fermentation oder durch Isolierung aus tierischen Geweben könnten durch die Produktion von Hyaluronan mittels transgener Pflanzen zwar ausgeschlossen werden, jedoch würden die bisher erreichten Mengen an Hyaluronan, die mittels transgener Pflanzen hergestellt werden können, eine relativ große Anbaufläche benötigen, um größere Mengen an Hyaluronan zu produzieren. Weiterhin ist die Isolierung oder Aufreinigung von Hyaluronan aus Pflanzen, die einen geringen Hyaluronangehalt aufweisen wesentlich aufwendiger und kostenintensiver als aus Pflanzen, die einen höheren Hyaluronangehalt aufweisen.

Obwohl Hyaluronan außergewöhnliche Eigenschaften aufweist, wird es für technische Anwendungen wegen seiner geringen Verfügbarkeit und des hohen Preises bisher, wenn überhaupt, selten eingesetzt.

Es ist daher Aufgabe der vorliegenden Erfindung, Mittel und Verfahren zur Verfügung zu stellen, die es erlauben, Hyaluronan in ausreichender Menge und Qualität zur Verfügung zu stellen, sowie die Möglichkeit zu eröffnen, Hyaluronan auch für technische Anwendungen und Anwendungen im Lebensmittel- und Futtermittelbereich zur Verfügung zu stellen.

Diese Aufgabe wird durch die in den Ansprüchen bezeichneten Ausführungsformen gelöst.

Somit betrifft die vorliegende Erfindung genetisch modifizierte Pflanzenzellen oder genetisch modifizierte Pflanzen, die ein in ihr Genom stabil integriertes Nudeinsäuremolekül, codierend eine Hyaluronansynthase aufweisen, dadurch gekennzeichnet, dass besagte Pflanzenzellen oder besagte Pflanzen zusätzlich eine erhöhte Aktivität eines Proteins mit der (enzymatischen) Aktivität einer Glutamin:Fructose-6-Phosphat Amidotransferase (GFAT) und eine erhöhte Aktivität eines Proteins mit der (enzymatischen) Aktivität einer UDP-Glucose Dehydrogenase (UDP-Glc-DH) aufweisen im Vergleich zu entsprechenden nicht genetisch modifizierten Wildtyp Pflanzenzellen bzw. nicht genetisch modifizierten Wildtyp Pflanzen.

Die genetische Modifikation erfindungsgemäßer genetisch modifizierter Pflanzenzellen oder erfindungsgemäßer genetisch modifizierter Pflanzen kann dabei jede genetische Modifikation sein, die eine stabile Intergration eines Nucleinsäuremoleküls, codierend eine Hyaluronansynthase in eine Pflanzenzelle oder eine Pflanze bewirkt und die zur Erhöhung der Aktivität eines Proteins mit der (enzymatischen) Aktivität einer GFAT und die zur Erhöhung der Aktivität eines Proteins mit der (enzymatischen) Aktivität einer UDP-Glc-DH in genetisch modifizierten Pflanzenzellen oder genetisch modifizierten Pflanzen führt im Vergleich zu entsprechenden nicht genetisch modifizierten Wildtyp Pflanzenzellen bzw. nicht genetisch modifizierten Wildtyp Pflanzen.

Der Begriff "Wildtyp-Pflanzenzelle" bedeutet im Zusammenhang mit der vorliegenden Erfindung, dass es sich um Pflanzenzellen handelt, die als Ausgangsmaterial für die Herstellung der erfindungsgemäßen genetisch modifizierten Pflanzenzellen dienten, d.h. deren genetische Information, abgesehen von den eingeführten genetischen Modifikationen, die zu einer stabilen Integration eines Nucleinsäuremoleküls, codierend eine Hyaluronansynthase und zur Erhöhung der Aktivität eines Proteins mit der Aktivität einer GFAT und zur Erhöhung der Aktivität eines Proteins mit der Aktivität einer UDP-Glc-DH führen, der einer erfindungsgemäßen genetisch modifizierten Pflanzenzelle entspricht.

Der Begriff "Wildtyp-Pflanze" bedeutet im Zusammenhang mit der vorliegenden Erfindung, dass es sich um Pflanzen handelt, die als Ausgangsmaterial für die Herstellung der erfindungsgemäßen genetisch modifizierten Pflanzen dienten, d.h. deren genetische Information, abgesehen von den eingeführten genetischen Modifikationen, die zu einer stabilen Integration eines Nucleinsäuremoleküls, codierend eine Hyaluronansynthase und zur Erhöhung der Aktivität eines Proteins mit der Aktivität einer GFAT und zur Erhöhung der Aktivität eines Proteins mit der Aktivität einer UDP-Glc-DH führen, der einer erfindungsgemäßen genetisch modifizierten Pflanze entspricht.

Der Begriff "entsprechend" bedeutet im Zusammenhang mit der vorliegenden Erfindung, dass beim Vergleich von mehreren Gegenständen die betreffenden Gegenstände, die miteinander verglichen werden, unter gleichen Bedingungen gehalten wurden. Im Zusammenhang mit der vorliegenden Erfindung bedeutet der Begriff "entsprechend" im Zusammenhang mit Wildtyp-Pflanzenzelle oder Wildtyp-Pflanze, dass die Pflanzenzellen oder Pflanzen, die miteinander verglichen werden, unter gleichen Kulturbedingungen aufgezogen wurden und dass sie ein gleiches (Kultur-) Alter aufweisen.

Unter dem Begriff "Hyaluronansynthase" (EC 2.4.1.212) soll im Zusammenhang mit der vorliegenden Erfindung ein Protein verstanden werden, das ausgehend von den Substraten UDP-Glucuronsäure (UDP-GlcA) und N-Acetyl-Glucosamin (UDP-GlcNAc) Hyaluronan synthetisiert. Die Katalyse der Hyaluronsynthese folgt dabei folgenden Reaktionsschemen:

$$\text{nUDP-GlcA + nUDP-GlcNAc} \rightarrow \text{beta-1,4-[GlcA-beta-1,3-GlcNAc]}_n \text{ + 2 nUDP}$$

Nucleinsäuremoleküle und korrespondierende Proteinsequenzen, codierend Hyaluronansynthasen sind u.a. für folgende Organismen beschrieben: Kaninchen (*Oryctolagus cuniculus*) ocHas2 (EMBL AB055978.1, US 20030235893), ocHas3 (EMBL AB055979.1, US 20030235893); Pavian (*Papio anubis*) paHas1 (EMBL AY463695.1); Frosch (*Xenopus laevis*) xlHas1 (EMBL M22249.1, US 20030235893), xlHas2 (DG42) (EMBL AF168465.1), xlHas3 (EMBL AY302252.1); Mensch *(Homo sapiens)* hsHAS1 (EMBL D84424.1, US 20030235893), hsHAS2 (EMBL U54804.1, US 20030235893), hsHAS3 (EMBL AF232772.1, US 20030235893); Maus *(Mus musculus*), mmHas1 (EMBL D82964.1, US 20030235893), mmHAS2 (EMBL U52524.2, US 20030235893), mmHas3 (EMBL U86408.2, US 20030235893); Rind (*Bos taurus*) btHas2 (EMBL AJ004951.1, US 20030235893); Huhn *(Gallus gallus)* ggHas2 (EMBL AF106940.1, US 20030235893); Ratte (*Rattus norvegicus*) rnHas 1 (EMBL AB097568.1, Itano et al., 2004, J. Biol. Chem. 279(18) 18679-18678), rnHas2 (EMBL AF008201.1); rnHas 3 (NCBI NM_172319.1, Itano et al., 2004, J. Biol. Chem. 279(18) 18679-18678), Pferd *(Equus caballus)* ecHAS2 (EMBL AY056582.1, GI:23428486), Schwein (*Sus scrofa*) sscHAS2 (NCBI NM_214053.1, GI:47522921), sscHas 3 (EMBLAB159675), Zebrafisch (*Danio rerio*) brHas1 (EMBL AY437407), brHas2 (EMBL AF190742.1) brHas3 (EMBL AF190743.1); *Pasteurella multocida* pmHas (EMBL AF036004.2); *Streptococcus pyogenes* spHas (EMBL, L20853.1, L21187.1, US 6,455,304, US 20030235893); *Streptococcus equis* seHas (EMBL AF347022.1, AY173078.1), *Streptococcus uberis* suHasA (EMBL AJ242946.2, US 20030235893), *Streptococcus equisimilis* seqHas (EMBL AF023876.1, US 20030235893); *Sulfolobus solfataricus* ssHAS (US 20030235893), *Sulfolobus tokodaii* stHas (AP000988.1), *Paramecium bursaria Chlorella* Virus 1, cvHAS (EMBL U42580.3, PB42580, US 20030235893).

Unter dem Begriff "UDP-Glucose Dehydrogenase (UDP-Glc-DH)" (E.C. 1.1.1.22) soll im Zusammenhang mit der vorliegenden Erfindung ein Protein verstanden werden, das aus UDP-Glucose (UDP-Glc) und $NAD^+$ UDP-Glucuronsäure (UDP-GlcA) und NADH synthetisiert. Diese Katalyse folgt dabei folgendem Reaktionsschema:

$$\text{UDP-Glc} + 2\ NAD^+ \rightarrow \text{UDP-GlcA} + 2\ \text{NADH}$$

Unter dem Begriff "Glutamin:Fructose-6-Phosphat Amidotransferase (GFAT)" (E.C. 2.6.1.16), in der Fachliteratur auch als Glucosamin Synthase bezeichnet, soll im Zusammenhang mit der vorliegenden Erfindung ein Protein verstanden werden, das aus den Edukten Glutamin und Fructose-6-Phosphat (Fruc-6-P) Glucosamin-6-Phosphat (GlcN-6-P) synthetisiert. Diese Katalyse folgt dabei folgendem Reaktionsschema:

$$\text{Glutamin} + \text{Fruc-6-P} \rightarrow \text{GlcN-6-P} + \text{Glutamat}$$

Insbesondere in tierischen Organismen konnten zwei unterschiedliche Isoformen von Proteinen mit der enzymatischen Aktivität einer GFAT nachgewiesen werden (in der Literatur bezeichnet als GFAT-1 bzw. GFAT-2). Hu et al. (2004), J. Biol. Chem. 279(29), 29988-29993) beschreiben Unterschiede der betreffenden Proteine aus Maus: Neben Unterschieden der gewebespezifischen Expression der betreffenden Proteine, mit der enzymatischen Aktivität einer Glutamin:Fructose-6-Phosphat Amidotransferase 1 (GFAT-1) und einer Glutamin:Fructose-6-Phosphat Amidotransferase 2 (GFAT-2), konnte gezeigt werden, dass beide Isoformen durch Phosphorylierung mittels einer cAMP abhängigen Proteinkinase reguliert werden. Die Aktivität eines Proteins mit der enzymatischen Aktivität einer GFAT-1 wird durch Phosphorylierung eines konservierten Serinrestes (Serin 205 in der GFAT-1 aus Maus, GenBank Acc No.: AF334736.1) der betreffenden Aminosäuresequenz inhibiert, wohingegen die Aktivität eines Proteins mit der Aktivität einer GFAT-2 durch Phosphorylierung eines konservierten Serinrestes (Serin 202 in der GFAT-2 aus Maus, GenBank Acc No.: NM_013529) der betreffenden Aminosäuresequenz gesteigert wird. Sowohl Proteine mit der Aktivität einer GFAT-1, als auch Proteine mit der Aktivität einer GFAT-2 werden durch UDP-N-Acetylglucosamin konzentrationsabhängig inhibiert, jedoch ist die Inhibition durch UDP-N-Acetylglucosamin für ein Protein mit der Aktivität einer GFAT-2 geringer (maximale Verringerung der Aktivität durch UDP-N-Acetylglucosamin um ca. 15%), im Vergleich zu einem Protein mit der Aktivität einer GFAT-1 (maximale Verringerung der Aktivität durch UDP-N-Acetylglucosamin um ca. 51% bzw. 80%). Es gibt Hinweise darauf, dass die Inhibition eines Proteins mit der Aktivität einer GFAT-1 in tierischen Organismen darauf zurückzuführen ist, dass bei erhöhten UDP-N-Acetylglucosamin Konzentrationen eine O-Glucose-N-Acetylglucosamin Glycosylierung der betreffenden Proteine erfolgt. Ob eine Regulation der Aktivität von Proteinen mittels O-Glycosylierung auch in pflanzlichen Zellen vorkommt, ist zurzeit nicht eindeutig geklärt (Huber und Hardin, 2004, Current Opinion in Plant Biotechnology 7, 318-322).

Unter dem Begriff "Glutamin:Fructose-6-Phosphat Amidotransferase-1 (GFAT-1)" soll im Zusammenhang mit der vorliegenden Erfindung ein Protein verstanden werden, das die Aktivität einer GFAT aufweist und dessen Aktivität durch Phosphorylierung mittels einer cAMP abhängigen Proteinkinase inhibiert wird.

Unter dem Begriff "Glutamin:Fructose-6-Phosphat Amidotransferase-2 (GFAT-2)" soll im Zusammenhang mit der vorliegenden Erfindung ein Protein verstanden werden, das die Aktivität einer GFAT aufweist und das durch Phosphorylierung mittels einer cAMP abhängigen Proteinkinase aktiviert wird.

Im Zusammenhang mit der vorliegenden Erfindung wird der Begriff "Glutamin:Fructose-6-Phosphat Amidotransferase (GFAT)" als übergeordneter Begriff verwendet, der alle Proteine umfasst, die die Aktivität einer GFAT aufweisen. Er umfasst daher auch Proteine, die in der Literatur als "Glutamin:Fructose-6-Phosphat Amidotransferase-1 (GFAT-1)" oder als "Glutamin:Fructose-6-Phosphat Amidotransferase-2 (GFAT-2)" bezeichnet werden, ist jedoch nicht auf diese beschränkt.

Der Begriff "erhöhte Aktivität eines Proteins mit der (enzymatischen) Aktivität einer GFAT" bedeutet im Rahmen der vorliegenden Erfindung eine Erhöhung der Expression endogener Gene, die Proteine mit der Aktivität einer GFAT codieren und/oder eine Erhöhung der Menge an Transkripten codierend Proteine mit der Aktivität einer GFAT und/oder eine Erhöhung der Menge an Protein mit der Aktivität einer GFAT in den Zellen und/oder eine Erhöhung der enzymatischen Aktivität von Proteinen mit der Aktivität einer GFAT in den Zellen.

Der Begriff "erhöhte Aktivität eines Proteins mit der (enzymatischen) Aktivität einer UDP-Glc-DH" bedeutet im Rahmen der vorliegenden Erfindung eine Erhöhung der Expression endogener Gene, die Proteine mit der Aktivität einer UDP-Glc-DH codieren und/oder eine Erhöhung der Menge an Transkripten codierend Proteine mit der Aktivität einer UDP-Glc-DH und/oder eine Erhöhung der Menge an Protein mit der Aktivität einer UDP-Glc-DH in den Zellen und/oder eine Erhöhung der enzymatischen Aktivität von Proteinen mit der Aktivität einer UDP-Glc-DH in den Zellen.

In erfindungsgemäßen genetisch modifizierten Pflanzenzellen oder erfindungsgemäßen genetisch modifizierten Pflanzen ist jeweils mindestens eine der zuvor genannten Bedingungen, die eine Erhöhung einer enzymatischen Aktivität eines Proteins bedeuten, für Proteine mit der (enzymatischen) Aktivität einer GFAT und für Proteine mit der (enzymatischen) Aktivität einer UDP-Glc-DH erfüllt.

Eine Erhöhung der Expression kann beispielsweise bestimmt werden durch Messung der Menge an Transkripten, codierend ein Protein mit der Aktivität einer GFAT bzw. codierend ein Protein mit der Aktivität einer UDP-Glc-DH, z.B. durch Northern-Blot-Analyse oder RT-PCR. Eine Erhöhung bedeutet dabei vorzugsweise eine Erhöhung der Menge an Transkripten im Vergleich zu entsprechenden nicht genetisch modifizierten Wildtyp Pflanzenzellen oder nicht genetisch modifizierten Wildtyp Pflanzen um mindestens 50%, insbesondere um mindestens 70%, bevorzugt um mindestens 85% und besonders bevorzugt um mindestens 100%. Eine Erhöhung der Menge an Transkripten, codierend ein Protein mit der Aktivität einer GFAT bzw. codierend ein Protein mit der Aktivität einer UDP-Glc-DH bedeutet auch, dass Pflanzen oder Pflanzenzellen, die keine nachweisbaren Mengen an Transkripten, codierend ein Protein mit der Aktivität einer GFAT und/oder codierend ein Protein mit der Aktivität einer UDP-Glc-DH, aufweisen, nach erfindungsgemäßer genetischer Modifikation nachweisbare Mengen an Transkripten, codierend ein Protein mit der Aktivität einer GFAT und/oder codierend ein Protein mit der Aktivität einer UDP-Glc-DH, aufweisen.

Die Erhöhung der Menge an Protein mit der Aktivität einer GFAT bzw. von Proteinen mit der Aktivität einer von UDP-Glc-DH, die eine erhöhte Aktivität dieser Proteine in den betreffenden Pflanzenzellen zur Folge hat, kann beispielsweise bestimmt werden durch immunologische Methoden wie Western-Blot-Analyse, ELISA (Enzyme Linked Immuno Sorbent Assay) oder RIA (Radio Immune Assay). Methoden zur Herstellung von Antikörpern, die spezifisch mit einem bestimmten Protein reagieren, d.h. die spezifisch an besagtes Protein binden, sind dem Fachmann bekannt (siehe z.B. Lottspeich und Zorbas (Eds.), 1998, Bioanalytik, Spektrum akad, Verlag, Heidelberg, Berlin, ISBN 3-8274-0041-4). Die Herstellung solcher Antikörper wird von einigen Firmen (z.B. Eurogentec, Belgien) als Auftragsservice angeboten. Eine Erhöhung der Menge an Protein bedeutet dabei vorzugsweise eine Erhöhung der Menge an Protein mit der Aktivität einer GFAT und/oder von Proteinen mit der Aktivität einer von UDP-Glc-DH im Vergleich zu entsprechenden nicht genetisch modifizierten Wildtyp Pflanzenzellen oder nicht genetisch modifizierten Wildtyp Pflanzen um mindestens 50%, insbesondere um mindestens 70%, bevorzugt um mindestens 85% und besonders bevorzugt um mindestens 100%. Eine Erhöhung der Menge an Protein mit der Aktivität einer GFAT und/oder an Protein mit der Aktivität einer UDP-Glc-DH bedeutet auch, dass Pflanzen oder Pflanzenzellen, die keine nachweisbare Menge eines Proteins mit der Aktivität einer GFAT und/oder die keine nachweisbare Aktivität eines Proteins mit der Aktivität einer UDP-Glc-DH aufweisen, nach erfindungsgemäßer genetischer Modifikation eine nachweisbare Menge eines Proteins mit der Aktivität einer GFAT und/oder eine nachweisbare Menge eines Proteins mit der Aktivität einer UDP-Glc-DH Proteins aufweisen.

Die Erhöhung der Aktivität eines Proteins mit der Aktivität einer GFAT in pflanzlichen Extrakten kann mit dem Fachmann bekannten Methoden wie z.B. beschrieben in Samac et al. (2004, Applied Biochemistry and Biotechnology 113-116, Humana Press, Edittor Ashok Mulehandani, 1167-1182, ISSN 0273-2289) bestimmt werden. Eine bevorzugte Methode zur Ermittleung der Menge der Aktivität eines Proteins mit der Aktivität einer GFAT ist in Allgemeine Methoden, Punkt 6 aufgeführt.

Die Erhöhung der Aktivität eines Proteins mit der Aktivität einer UDP-Glc-DH in pflanzlichen Extrakten kann mit dem Fachmann bekannten Methoden wie z.B. beschrieben in WO 00 11192 beschrieben werden. Eine bevorzugte Methode zur Ermittlung der Menge der Aktivität eines Proteins mit der Aktivität einer UDP-Glc-DH ist in Allgemeine Methoden, Punkt 7 aufgeführt.

Eine Erhöhung der Menge an (enzymatischer) Aktivität von Proteinen mit der Aktivität einer GFAT bzw. von Proteinen mit der Aktivität einer von UDP-Glc-DH, bedeutet vorzugsweise eine Erhöhung der Aktivität solcher Proteine um mindestens 50%, bevorzugt um mindestens 70%, insbesondere bevorzugt um mindestens 85% und besonders bevorzugt um mindestens 100% im Vergleich zu entsprechenden nicht genetisch modifizierten Wildtyp Pflanzenzellen oder nicht genetisch modifizierten Wildtyp Pflanzen. Eine Erhöhung der Menge an (enzymatischer) Aktivität von Proteinen mit der Aktivität einer GFAT und/oder an Protein mit der Aktivität einer UDP-Glc-DH bedeutet auch, dass Pflanzen oder Pflanzenzellen, die keine nachweisbare Menge eines Proteins mit der Aktivität einer GFAT und/oder die keine nachweisbare Aktivität eines Proteins mit der Aktivität einer UDP-Glc-DH aufweisen, nach erfindungsgemäßer genetischer Modifikation eine nachweisbare Menge eines Proteins mit der Aktivität einer GFAT und/oder eine nachweisbare Menge eines Proteins mit der Aktivität einer UDP-Glc-DH aufweisen.

Unter dem Begriff "Genom" soll im Zusammenhang mit der vorliegenden Erfindung die Gesamtheit des in einer pflanzlichen Zelle vorliegenden Erbmaterials verstanden werden. Dem Fachmann ist bekannt, dass neben dem Zellkern auch andere Kompartimente (z.B. Plastiden, Mitochondrien) Erbmaterial enthalten.

Unter dem Begriff "stabil integriertes Nucleinsäuremolekül" soll im Zusammenhang mit der vorliegenden Erfindung die Integration eines Nukleinsäuremoleküls in das Genom der Pflanze verstanden werden. Ein stabil integriertes Nucleinsäuremolekül zeichnet sich dadurch aus, dass es bei der Replikation des entsprechenden Integrationsortes zusammen mit den wirtseigenen, den Integrationsort flankierenden Nucleinsäuresequenzen vervielfältigt wird, so dass der Integrationsort in dem replizierten Tochter DNA Strang von den selben Nucleinsäuresequenzen umgeben ist, wie auf dem abgelesenen Mutter Strang, der als Matrize für die Replikation dient.

Für die stabile Integration von Nucleinsäuremolekülen in eine pflanzliche Wirtszelle steht eine Vielzahl von Techniken zur Verfügung. Diese Techniken umfassen die Transformation pflanzlicher Zellen mit T-DNA unter Verwendung von *Agrobacterium tumefaciens* oder *Agrobacterium rhizogenes* als Transformationsmittel, die Fusion von Protoplasten, die Injektion, die Elektroporation von DNA, die Einbringung der DNA mittels des biolistischen Ansatzes sowie weitere Möglichkeiten (Übersicht in "Transgenic Plants", Leandro ed., Humana Press 2004, ISBN 1-59259-827-7).

Die Verwendung der Agrobakterien-vermittelten Transformation von Pflanzenzellen ist intensiv untersucht und ausreichend in EP 120516; Hoekema, IN: The Binary Plant Vector System Offsetdrukkerij Kanters B.V. Alblasserdam (1985), Chapter V; Fraley et al., Crit. Rev. Plant Sci. 4, 1-46 und bei An et al. EMBO J. 4, (1985), 277-287 beschrieben worden. Für die Transformation von Kartoffel, siehe z.B. Rocha-Sosa et al., EMBO J. 8, (1989), 29-33), für die Transformation von Tomatenpflanzen z.B. US 5,565,347.

Auch die Transformation monokotyler Pflanzen mittels auf *Agrobacterium* Transformation basierender Vektoren wurde beschrieben (Chan et al., Plant Mol. Biol. 22, (1993), 491-506; Hiei et al., Plant J. 6, (1994) 271-282; Deng et al, Science in China 33, (1990), 28-34; Wilmink et al., Plant Cell Reports 11, (1992), 76-80; May et al., Bio/Technology 13, (1995), 486-492; Conner und Domisse, Int. J. Plant Sci. 153 (1992), 550-555; Ritchie et al, Transgenic Res. 2, (1993), 252-265). Ein alternatives System zur Transformation von monokotylen Pflanzen ist die Transformation mittels des biolistischen Ansatzes (Wan und Lemaux, Plant Physiol. 104, (1994), 37-48; Vasil et al., Bio/Technology 11 (1993), 1553-1558; Ritala et al., Plant Mol. Biol. 24, (1994), 317-325; Spencer et al., Theor. Appl. Genet. 79, (1990), 625-631), die Protoplastentransformation, die Elektroporation von partiell permeabilisierten Zellen, die Einbringung von DNA mittels Glasfasern. Insbesondere die Transformation von Mais wird in der Literatur mehrfach beschrieben (vgl. z. B. WO95/06128, EP0513849, EP0465875, EP0292435; Fromm et al., Biotechnology 8, (1990), 833-844; Gordon-Kamm et al., Plant Cell 2, (1990), 603-618; Koziel et al., Biotechnology 11 (1993), 194-200; Moroc et al., Theor. Appl. Genet. 80, (1990), 721-726). Die Transformation von anderen Gräsern, wie z.B. der Rutenhirse (switchgrass, *Panicum virgatum*) ist ebenfalls beschrieben (Richards et al., 2001, Plant Cell Reporters 20, 48-54).

Auch die erfolgreiche Transformation anderer Getreidearten wurde bereits beschrieben, z.B. für Gerste (Wan und Lemaux, s.o.; Ritala et al., s.o.; Krens et al., Nature 296, (1982), 72-74) und für Weizen (Nehra et al., Plant J. 5, (1994), 285-297; Becker et al., 1994, Plant Journal 5, 299-307). Alle vorstehenden Methoden sind im Rahmen der vorliegenden Erfindung geeignet.

Erfindungsgemäße genetisch modifizierte Pflanzenzellen oder erfindungsgemäße genetisch modifizierte Pflanzen bieten gegenüber dem Stand der Technik den Vorteil, dass sie höhere Mengen an Hyaluronan produzieren als Pflanzen, die nur die Aktivität einer Hyaluronansynthase aufweisen. Dieses ermöglicht die Hyaluronan Produktion mit geringem Aufwand, da die Isolierung von Hyaluronan aus Pflanzen mit höherem Hyaluronangehalt weniger aufwendig und kostengünstiger ist. Weiterhin sind geringere Anbauflächen notwendig, um mit erfindungsgemäßen genetisch modifizierten Pflanzen Hyaluronan zu produzieren im Vergleich zu im Stand der Technik beschriebenen Pflanzen. Dieses führt zu der Möglichkeit, Hyaluronan in ausreichender Menge auch für technische Anwendungen zur Verfügung zu stellen, bei welchen es wegen der geringen Verfügbarkeit und des hohen Preises zurzeit keine Verwendung findet.

Pflanzlichen Organismen der Gattung *Chlorella,* die mit einem Virus infiziert sind, sind nicht geeignet für die Produktion von größeren Mengen von Hyaluronan. Virus infizierte Algen weisen für die Produktion von Hyaluronan den Nachteil auf, dass sie die Gene, die für die Hyaluronansynthase notwendig sind, nicht stabil in ihr Genom integriert haben (Van Etten und Meints, 1999, Annu. Rev. Microbiol. 53, 447-494), so dass für die Produktion von Hyaluronan immer wieder eine erneute Virusinfektion erfolgen muss. Daher ist es nicht möglich, einzelne *Chlorella* Zellen zu isolieren, die kontinuierlich die gewünschte Qualität und Quantität an Hyaluronan synthetisieren. Weiterhin findet die Produktion von Hyaluronan in Virus infizierten *Chlorella* Algen nur für eine begrenzte Zeit statt und die Algen werden durch von dem Virus bewirkte Lyse bereits etwa 8 Stunden nach der Infektion abgetötet (Van Etten et al., 2002, Arch Virol 147, 1479-1516). Demgegenüber bietet die vorliegende Erfindung den Vorteil, dass erfindungsgemäße genetisch modifizierte Pflanzenzellen und erfindungsgemäße genetisch modifizierte Pflanzen unbegrenzt vegetativ oder sexuell vermehrt werden können und dass sie kontinuierlich Hyaluronan produzieren.

Die in WO 05 012529 beschriebenen transgenen Pflanzen, die ein Nucleinsäuremolekül, codierend eine Hyaluronansynthase, aufweisen, synthetisieren eine relativ geringe Menge an Hyaluronan. Demgegenüber bietet die vorliegende Erfindung den Vorteil, dass erfindungsgemäße genetisch modifizierte Pflanzenzellen und erfindungsgemäße genetisch modifizierte Pflanzen deutlich höhere Mengen an Hyaluronan synthetisieren.

Gegenstand der vorliegenden Erfindung sind daher auch erfindungsgemäße genetisch modifizierte Pflanzenzellen oder erfindungsgemäße genetisch modifizierte Pflanzen, die Hyaluronan synthetisieren. Vorzugsweise synthetisieren erfindungsgemäße genetisch modifizierte Pflanzenzellen oder erfindungsgemäße genetisch modifizierte Pflanzen mindestens 100 bevorzugt mindestens 600 besonders bevorzugt mindestens 1000 insbesondere bevorzugt mindestens 1500 μg Hyaluronan pro g Frischgewicht (FG) Pflanzenmaterial. Zur Bestimmung des Hyaluronangehaltes bezüglich des Frischgeweichtes in erfindungsgemäßen genetisch modifizierten Pflanzenzellen oder in erfindungsgemäßen genetisch modifizierten Pflanzen wird bevorzugt die unter Allgemeine Methoden Punkt 2 beschriebene Methode zur Aufarbeitung des Pflanzenmaterials und die unter Allgemeine Methoden Punkt 4 beschriebene Methode zur Bestimmung der Menge an Hyaluronan verwendet.

Gegenstand der vorliegenden Erfindung sind auch erfindungsgemäße genetisch modifizierte Pflanzenzellen oder erfindungsgemäße genetisch modifizierte Pflanzen, die mindestens 1000 bevorzugt mindestens 2000 besonders bevorzugt mindestens 4000 insbesondere bevorzugt mindestens 5000 µg Hyaluronan pro g Trockengewicht (TG) Pflanzenmaterial. Zur Bestimmung des Hyaluronangehaltes bezüglich des Trockengewichtes in erfindungsgemäßen genetisch modifizierten Pflanzenzellen oder in erfindungsgemäßen genetisch modifizierten Pflanzen wird bevorzugt die in Beispiel 13 k) beschriebene Methode zur Aufarbeitung des Pflanzenmaterials und die unter Allgemeine Methoden Punkt 4 beschriebene Methode zur Bestimmung der Menge an Hyaluronan verwendet.

Da beobachtet wurde, dass Hyaluronan über die Entwicklungszeit in pflanzlichem Gewebe akkumuliert, soll die Menge an Hyaluronan bezüglich der Frischgewichtes bzw. bezüglich des Trockengewichtes in erfindungsgemäßen genetisch modifizierten Pflanzenzellen oder in erfindungsgemäßen genetisch modifizierten Pflanzen besonders bevorzugt zum Zeitpunkt der Ernte oder wenige (ein bis zwei) Tage vor der Ernte betreffender Pflanzenzellen bzw. betreffender Pflanzen bestimmt werden. Insbesondere wird dabei solches Pflanzenmaterial (z.B. Knollen, Samen, Blätter) bezüglich der Menge an Hyaluronan eingesetzt, das zur weiteren Verarbeitung herangezogen werden soll.

Erfindungsgemäße genetisch modifizierte Pflanzenzellen oder erfindungsgemäße genetisch modifizierte Pflanzen, die Hyaluronan synthetisieren, lassen sich dadurch nachweisen, dass man das von ihnen synthetisierte Hyaluronan isoliert und dessen Struktur nachweist.
Da pflanzliches Gewebe den Vorteil aufweist, dass es keine Hyaluronidasen enthält, kann zum Nachweis des Vorliegens von Hyaluronan in erfindungsgemäßen genetisch modifizierten Pflanzenzellen oder erfindungsgemäßen genetisch modifizierten Pflanzen eine einfache und schnelle Isolierungsmethode verwendet werden. Dazu wird zu dem zu untersuchenden Pflanzengewebe Wasser hinzu gegeben, bevor das Pflanzengewebe mechanisch (z.B. unter zu Hilfenahme einer Kugelmühle, Schlagmühle, eines Warring Blendors, eines Entsafters etc.) zerkleinert wird. Anschließend kann bei Bedarf erneut Wasser zu der Suspension hinzu gegeben werden, bevor Zelltrümmer und Wasser unlösliche Bestandteile durch Zentrifugation oder Sieben abgetrennt werden. Der Nachweis des Vorliegens von Hyaluronan kann anschließend im nach Zentrifugation erhaltenen Überstand z.B. mittels eines spezifisch an Hyaluronan bindenden Proteins erfolgen. Ein Verfahren zum Nachweis von Hyaluronan mit Hilfe eines spezifisch an Hyaluronan bindenden Proteins ist z.B. in US 5,019,498 beschrieben. Testkits, (z.B. das Hyaluronic Acid (HA) Test Kit der Firma Corgenix, Inc., Colorado, USA, Prod. Nr. 029-001) zur Durchführung der in US 5,019,498 beschrieben Methode, sind käuflich erwerbbar (z.B. das Hyaluronic Acid (HA) Test Kit der Firma Corgenix, Inc., Colorado, USA, Prod. Nr. 029-001; siehe auch Allgemeine Methoden Punkt 4). Parallel dazu kann ein Aliquot des erhaltenen Überstandes der Zentrifugation zunächst mit einer Hyaluronidase verdaut werden, bevor der Nachweis des Vorliegens von Hyaluronan mit Hilfe des spezifisch an Hyaluronan bindenden Proteins, wie oben beschrieben, erfolgt. Durch die Einwirkung der Hyaluronidase in dem Parallelansatz wird das darin vorliegende Hyaluronan abgebaut, so dass nach vollständigem Verdau keine signifikanten Mengen an Hyaluronan mehr nachweisbar sind. Weiterhin kann der Nachweis des Vorliegens von Hyaluronan im Überstand der Zentrifugation auch mit anderen Analysemethoden, wie z.B. der IR-, NMR- oder Massen-Spektroskopie, erfolgen.

Wie bereits oben ausgeführt, ist zur Zeit nicht geklärt, welcher Stoffwechselweg (Hexosephosphat- oder oxidativer *myo*-Inositol-Stoffinrechselweg) in pflanzlichen Zellen hauptsächlich für die Synthese von UDP-Glucuronsäure verwendet wird und ob beide Stoffwechselwege einen unterschiedlichen quantitativen Beitrag je nach Gewebe und/oder Entwicklungsstadium der Pflanze zur Synthese von UDP-Glucuronsäure leisten. Weiterhin führte die Überexpression einer UDP-Glc-DH in transgenen Pflanzen zu nicht konsistenten Ergebnissen und das Ziel mittels eines solchen Ansatzes den Pektingehalt der Zellwand zu erhöhen konnte nicht erreicht werden. Zusätzlich unterliegt die Regulation der Aktivität von Proteinen mit der Aktivität einer UDP-Glc-DH einer Inhibierung durch UDP-Xylose. Dieses wurde sowohl für betreffende Proteine, die aus Prokaryonten (Campbell et al., 1997, J. Biol. Chem. 272(6), 3416-3422; Schiller et al., 1973, Biochim. Biophys Acta 293(1), 1-10), aus tierischen Organismen (Balduini et al., 1970, Biochem. J. 120(4), 719-724) und aus Pflanzen (Hinterberg, 2002, Plant Physiol. Biochem. 40, 1011-1017) stammen, gezeigt. Zudem stellen die aus der von einem Protein mit der Aktivität einer UDP-Glc-DH katalysierten Reaktion hervorgehenden Reaktionsprodukte Glucuronsäure und NADH die Aktivität eines Proteins mit der Aktivität einer GFAT regulierende Inhibitoren dar (Campbell et al., 1997, J. Biol. Chem. 272(6), 3416-3422, Ordman und Kirkwood, 1977, Biochim Biophys Acta 482(1) 25-32; Turner und Botha, 2002, Archives of Biochem. Biophys..407, 209-216).
Die Überexpression eines mit einem plastidären Signalpeptid translational fusionierten Proteins mit der enzymatischen Aktivität einer GFAT in Mais führte zur Erhöhung des UDP-Glucosamin Gehaltes und die cytosolische Überexpression eines Proteins mit der enzymatischen Aktivität einer GFAT in Mais führte zur Erhöhung des Glucosamin-1-Phosphat Gehaltes in gemahlenem Endosperm Gewebe. UDP-Glucosamin und Glucosamin-1-Phosphat stellen jedoch kein Edukt für die Synthese von Hyaluronan mittels Hyaluronansynthase dar. Weiterhin ist bekannt, dass Glucosamin cytotoxisch auf Pflanzenzellen wirkt (Roberts et al., 1971, Plant Physiol. 48, 36-42) und dass es, wenn es in höheren Konzentrationen in pflanzlichen Zellen vorliegt, zu Glucosamin-6-Phosphat umgewandelt wird. Glucosamin-6-Phosphat ist ebenfalls toxisch für Pflanzenzellen (WO 98 35047, US 6,444,878). Weiterhin ist bekannt, dass Proteine mit der Aktivität einer (GFAT durch Metabolite, die im weiteren Stoffwechselweg für die Synthese von UDP-N-Acetyl-Glucosamin auftreten, inhibitorisch reguliert werden. Proteine mit der Aktivität einer GFAT, isoliert aus Eukaryonten (sowohl tierische als auch pflanzliche Organismen) werden z.B. durch UDP-N-Acetyl-Glucosamin, welches eines der beiden Substrate für Hyaluronansynthase darstellt, inhibiert (Kornfeld, 1967, J. Biol. Chem. 242(13), 3135-3141; Graack et al., 2001, Biochem. J. 360, 401-412; Mayer et al., 1968, Plant Physiol. 43, 1097-1107). Bakterielle Proteine mit der Aktivität einer GFAT werden durch Glucosamin-6-Phosphat, eines direkten Reaktionsprodukts der durch GFAT katalysierten Reaktion, inhibiert (Deng et al., 2005, Metabolic Engeneering 7, 201-214).

Die Literatur enthält keine Hinweise darauf, wodurch die Menge an synthetisiertem Hyaluronan in Pflanzenzellen begrenzt sein könnte. Es wurde daher überraschenderweise gefunden, dass genetisch modifizierte Pflanzenzellen oder genetisch modifizierte Pflanzen, die ein Nucleinsäuremolekül, codierend eine Hyaluronansynthase aufweisen und die zusätzlich eine erhöhte Aktivität einer GFAT und eine erhöhte Aktivität einer UDP-Glc-DH im Vergleich zu genetisch modifizierten Pflanzenzellen bzw. genetisch modifizierten Pflanzen, die (nur) die Aktivität einer Hyaluronansynthase aufweisen, signifikant höhere Mengen an Hyaluronan produzieren.

In einer bevorzugten Ausführungsform betrifft die vorliegende Erfindung erfindungsgemäße genetisch modifizierte Pflanzenzellen oder erfindungsgemäße genetisch modifizierte Pflanzen, dadurch gekennzeichnet, dass sie eine erhöhte Menge an Hyaluronan produzieren im Vergleich zu genetisch modifizierten Pflanzenzellen bzw. im Vergleich zu genetisch modifizierten Pflanzen, die (nur) die Aktivität einer Hyaluronansynthase aufweisen oder im Vergleich zu genetisch modifizierten Pflanzenzellen bzw. im Vergleich zu genetisch modifizierten Pflanzen, die die Aktivität einer Hyaluronansynthase aufweisen und keine erhöhte Aktivität eines Proteins mit der Aktivität einer GFAT und keine erhöhte Aktivität eines Proteins mit der Aktivität einer UDP-Glc-DH aufweisen. Vorzugsweise ist die Menge an produziertem Hyaluronan bezüglich des Frischgewichtes des Pflanzematerials in erfindungsgemäßen genetisch modifizierten Pflanzenzellen bzw. in erfindungsgemäßen genetisch modifizierten Pflanzen um mindestens das 1,5 fache, bevorzugt um mindestens das 5 fache, besonders bevorzugt um mindestens das 7,5 fache und insbesondere bevorzugt um mindestens 10 fache erhöht, im Vergleich zu entsprechenden genetisch modifizierten Pflanzenzellen bzw. im Vergleich zu entsprechenden genetisch modifizierten Pflanzen, die (nur) die Aktivität einer Hyaluronansynthase aufweisen. Zur Feststellung der Erhöhung des Hyaluronangehaltes bezüglich des Frischgewichtes des Pflanzematerials in erfindungsgemäßen genetisch modifizierten Pflanzenzellen bzw. in erfindungsgemäßen genetisch modifizierten Pflanzen ist vorzugsweise ein Vergleich zwischen erfindungsgemäßen genetisch modifizierten Pflanzenzellen bzw. erfindungsgemäßen genetisch modifizierten Pflanzen mit entsprechenden Pflanzenzellen bzw. Pflanzen, die (nur) die Aktivität Hyaluronansynthase aufweisen heranzuziehen, wobei äquivalentes Material (z.B. Blatt, Knolle) von Pflanzenzellen bzw. Pflanzen miteinander verglichen werden soll, die Pflanzenzellen bzw. Pflanzen, von denen diese Material entnommen wird, unter gleichen Bedingungen kultiviert wurden und wobei der Hyaluronangehalt von Pflanzenmaterial miteinander verglichen werden soll, das ein vergleichbares Alter (Entwicklungsstadium) aufweist. Es sollen z.B. nicht junge Blätter einer Pflanze mit alten Blättern einer anderen Pflanze oder Pflanzen verglichen werden.

Unter dem Begriff. "Pflanzenzelle oder Pflanze, die (nur) die Aktivität einer Hyaluronansynthase aufweist", soll im Zusammenhang mit der vorliegenden Erfindung eine genetisch modifizierte Pflanzenzelle oder eine genetisch modifizierte Pflanze verstanden werden, wobei die genetische Modifikation darin besteht, dass sie ein Nucleinsäuremolekül, codierend eine Hyaluronansynthase im Vergleich zu entsprechenden nicht genetisch modifizierten Wildtyp Pflanzenzellen bzw. nicht genetisch modifizierten Wildtyp Pflanzen aufweist.

Insbesondere zeichnen sich "Pflanzenzellen oder Pflanzen, die (nur) die Aktivität einer Hyaluronansynthase aufweisen, dadurch aus, dass sie Hyaluronan synthetisieren und dass sie keine zusätzlichen genetischen Modifikationen, die über das Einführen eines Nucleinsäuremoleküls, codierend eine Hyaluronansynthase, in nicht genetisch modifizierte Wildtyp Pflanzenzellen bzw. nicht genetisch modifizierte Wildtyp Pflanzen hinausgeht, aufweisen. Bevorzugt weisen solche Pflanzen keine erhöhte Aktivität eines Proteins mit der Aktivität einer GFAT und keine erhöhte Aktivität eines Proteins mit der Aktivität einer UDP-Glc-DH auf.

Die Menge an Hyaluronan, die von Pflanzenzellen oder Pflanzen produziert wird, kann mit Hilfe der bereits oben beschriebenen Methoden, z.B. unter Verwendung eines käuflichen Testkits, (z.B. das Hyaluronic Acid (HA) Test Kit der Firma Corgenix, Inc., Colorado, USA, Prod. Nr. 029-001) bestimmt werden. Eine im Zusammenhang mit der vorliegenden Erfindung bevorzugte Methode zur Bestimmung des Hyaluronangehaltes in Pflanzenzellen oder Pflanzen ist unter Allgemeine Methoden, Punkt 4 beschrieben.

In einer weiteren Ausführungsform der vorliegenden Erfindung handelt es sich bei erfindungsgemäßen genetisch modifizierten Pflanzenzellen oder erfindungsgemäßen genetisch modifizierten Pflanzen um Pflanzenzellen einer grünen Landpflanze bzw. um grüne Landpflanzen, die Hyaluronan synthetisieren.

Der Begriff "grüne Landpflanze (Embryophyta)" ist im Zusammenhang mit der vorliegenden Erfindung so zu verstehen, wie er in Strasburger, "Lehrbuch der Botanik", 34. Aufl., Spektrum Akad. Verl., 1999, (ISBN 3-8274-0779-6) definiert ist.

Eine bevorzugte Ausführungsform der vorliegenden Erfindung betrifft erfindungsgemäße genetisch modifizierte Pflanzenzellen von mehrzelligen Pflanzen oder erfindungsgemäße genetisch modifizierte Pflanzen, die mehrzellige Organismen darstellen. Es handelt sich bei dieser Ausführungsform daher um Pflanzenzellen oder Pflanzen die nicht von einzelligen Pflanzen (Protisten) stammen oder keine Protisten sind.

Bei den erfindungsgemäßen genetisch modifizierten Pflanzenzellen oder erfindungsgemäßen genetisch modifizierten Pflanzen kann es sich prinzipiell um Pflanzenzellen bzw. um Pflanzen jeder beliebigen Pflanzenspezies handeln, d.h. sowohl um monokotyle als auch dikotyle Pflanzen. Vorzugsweise handelt es sich um Nutzpflanzen, d.h. Pflanzen, die vom Menschen kultiviert werden für Zwecke der Ernährung von Mensch und Tier oder für die Produktion von Biomasse bzw. die Herstellung von Substanzen für technische, industrielle Zwecke (z.B. Mais, Reis, Weizen, Luzerne, Roggen, Hafer, Gerste, Maniok, Kartoffel, Tomate, Rutenhirse (*Panicum virgatum*), Sago, Mungbohne, Erbse, Sorghum, Möhre, Aubergine, Rettich, Raps, Sojabohne, Erdnuss, Gurke, Kürbis, Melone, Lauch, Knoblauch, Kohl, Spinat, Süßkartoffel, Spargel, Zucchini, Salat, Artischocke, Süßmais, Pastinak, Schwarzwurzel, Topinamubur, Banane, Zuckerrübe, Zuckerrohr, Rote Bete, Brokkoli, Kohl, Zwiebel, gelbe Rübe, Löwenzahn, Erdbeere, Apfel, Aprikose, Pflaume, Pfirsich, Weintrauben, Blumenkohl, Sellerie, Paprika, Steckrübe, Rhabarber). Besonders bevorzugt handelt es sich um Tomaten- oder Kartoffelpflanzen.

In einer vorzugsweisen Ausführungsform betrifft die vorliegende Erfindung erfindungsgemäße genetisch modifizierte Pflanzenzellen oder erfindungsgemäße genetisch modifizierte Pflanzen, wobei das Hyaluronansynthase codierende Nucleinsäuremolekül dadurch gekennzeichnet ist, dass eine virale Hyaluronansynthase codiert. Bevorzugt codiert das Hyaluronansynthase codierende Nucleinsäuremolekül eine Hyaluronansynthase eines Virus, der Algen infiziert.

Bezüglich eines Virus, der Algen infiziert, codiert das Hyaluronansynthase codierende Nucleinsäuremolekül vorzugsweise eine Hyaluronansynthase eines *Chlorella* infizierenden Virus, besonders bevorzugt eine Hyaluronansynthase eines *Paramecium bursaria Chlorella* Virus 1 und insbesondere bevorzugt eine Hyaluronansynthase eines *Paramecium bursaria Chlorella* Virus eines H1 Stammes.

In einer weiteren bevorzugten Ausführungsform betrifft die vorliegende Erfindung erfindungsgemäße genetisch modifizierte Pflanzenzellen oder erfindungsgemäße genetisch modifizierte Pflanzen, wobei das Hyaluronansynthase codierende Nucleinsäuremolekül dadurch gekennzeichnet, dass die Codons des Nucleinsäuremoleküls codierend eine Hyaluronansynthase verändert sind, im Vergleich zu den Codons des Nucleinsäuremoleküls, welches die Hyaluronansynthase des Ursprungsorganismus der Hyaluronansynthase codieren.

Besonders bevorzugt sind die Codons der Hyaluronansynthase dahingehend verändert, dass sie an die Häufigkeit der Verwendung der Codons der Pflanzenzelle oder der Pflanze, in dessen Genom sie integriert sind oder werden, angepasst sind.

Aminosäuren können auf Grund der Degeneration des genetischen Codes durch ein oder mehrere Codons codiert werden. Unterschiedliche Organismen verwenden die jeweils für eine Aminosäure codierenden Codons mit unterschiedlicher Häufigkeit. Das Anpassen der Codons einer codierenden Nucleinsäuresequenz an die Häufigkeit ihrer Verwendung in der Pflanzenzelle oder in der Pflanze, in dessen Genom die zu exprimierende Sequenz integriert werden soll, kann zu einer erhöhten Menge an translatiertem Protein und/oder zur Stabilität der betreffenden mRNA in den betreffenden Pflanzenzellen oder Pflanzen beitragen. Der Fachmann kann die Häufigkeit der Verwendung von Codons in betreffenden Pflanzenzellen oder Pflanzen ermitteln, indem er möglichst viele codierende Nucleinsäuresequenzen des betreffenden Organismus dahingehend untersucht, wie häufig bestimmte Codons für die Codierung einer bestimmten Aminosäure verwendet werden. Die Häufigkeit der Verwendung von Codons bestimmter Organismen ist dem Fachmann geläufig und kann mit Hilfe von Computerprogrammen einfach und schnell durchgeführt werden. Entsprechende Computerprogramme sind öffentlich zugänglich und werden u.a. im Internet frei zur Verfügung gestellt (z.B. http://gcua.schoedl.de/; http://www.kazusa.or.jp/codon/; http://www.entelechon.com/eng/cutanalysis.html). Das Anpassen der Codons einer codierenden Nucleinsäuresequenz an die Häufigkeit ihrer Verwendung in der Pflanzenzelle oder in der Pflanze, in dessen Genom die zu exprimierende Sequenz integriert werden soll, kann durch *in vitro* Mutagenese oder bevorzugt durch Neusynthese der Gensequenz erfolgen. Methoden zur Neusynthese von Nucleinsäuresequenzen sind dem Fachmann bekannt. Eine Neusynthese kann z.B. dadurch erfolgen, dass zunächst einzelne Nucleinsäureoligonucleotide synthetisiert werden, diese mit zu ihnen komplementären Oligonucleotiden hybridisiert werden, so dass sie einen DNA-Doppelstrang ausbilden, bevor die einzelnen doppelsträngigen Oligonucleotide so miteinander ligiert werden, dass die gewünschte Nucleinsäuresequenz erhalten wird. Die Neusynthese von Nucleinsäuresequenzen inklusive der Anpassung der Häufigkeit der Verwendung der Codons an einen bestimmten Zielorganismus kann auch als Auftrag an Firmen, die dieses als Dienstleistung anbieten, vergeben werden (z.B. Entelechon GmbH, Regensburg, Germany).

Bevorzugt ist das Hyaluronansynthase codierende Nucleinsäuremolekül dadurch gekennzeichnet, dass es eine Hyaluronansynthase codiert, dessen Aminosäuresequenz mit der unter SEQ ID NO 2 dargestellten Aminosäuresequenz eine Identität von mindestens 70%, vorzugsweise von mindestens 80%, bevorzugt von mindestens 90% und insbesondere bevorzugt von mindestens 95% aufweist. In einer besonders bevorzugten Ausführungsform ist das Hyaluronansynthase codierende Nucleinsäuremolekül dadurch gekennzeichnet, dass es eine Hyaluronansynthase codiert, die die unter SEQ ID No 2 dargestellte Aminosäuresequenz aufweist.

In einer weiteren Ausführungsform weist das Nucleinsäuremolekül, codierend eine Hyaluronansynthase mit der unter SEQ ID NO 1 oder SEQ ID NO 3 dargestellten Nucleinsäuresequenz eine Identität von mindestens 70%, vorzugsweise von mindestens 80%, bevorzugt von mindestens 90% und insbesondere bevorzugt von mindestens 95% auf. In einer besonders bevorzugten Ausführungsform ist das Hyaluronansynthase codierende Nucleinsäuremolekül dadurch gekennzeichnet, dass es die unter SEQ ID No 3 dargestellte Nucleinsäuresequenz aufweist.

Das Plasmid IC 341-222, enthaltend ein synthetisches Nucleinsäuremolekül, das eine *Paramecium bursaria Chlorella* Virus Hyaluronansynthase codiert, wurde am 25.08.2004 unter der Nummer DSM16664 nach dem Budapester Vertrag hinterlegt bei der Deutschen Sammlung von Mikroorganismen und Zellkulturen GmbH, Mascheroder Weg 1b, 38124 Braunschweig, Deutschland. Die in SEQ ID NO 2 dargestellte Aminosäuresequenz kann von der codierenden Region der in Plasmid IC 341-222 integrierten Nucleinsäuresequenz abgeleitet werden und codiert für eine *Paramecium bursaria Chlorella* Virus Hyaluronansynthase.

Die vorliegende Erfindung betrifft daher auch erfindungsgemäße genetisch modifizierte Pflanzenzellen oder erfindungsgemäße genetisch modifizierte Pflanzen, wobei das Hyaluronansynthase codierende Nucleinsäuremolekül dadurch gekennzeichnet ist, dass es ein Protein codiert, dessen Aminosäuresequenz von der codierenden Region der im Plasmid DSM16664 inserierten Nucleinsäuresequenz abgeleitet werden kann oder dass es ein Protein codiert, dessen Aminosäuresequenz mit der Aminosäuresequenz, die von der codierenden Region der im Plasmid DSM16664 inserierten Nucleinsäuresequenz abgeleitet werden kann, eine Identität von mindestens 70%, vorzugsweise von mindestens 80%, bevorzugt von mindestens 90% und insbesondere bevorzugt von mindestens 95% aufweist.

Die vorliegende Erfindung betrifft auch erfindungsgemäße genetisch modifizierte Pflanzenzellen oder erfindungsgemäße genetisch modifizierte Pflanzen, wobei das Hyaluronansynthase codierende Nucleinsäuremolekül dadurch gekennzeichnet ist, dass es die in Plasmid DSM16664 integrierte, Hyaluronansynthase codierende Nucleinsäuresequenz darstellt oder dass sie mit der in Plasmid DSM16664 integrierten Nucleinsäuresequenz eine Identität von mindestens 70%, vorzugsweise von mindestens 80%, bevorzugt von mindestens 90% und insbesondere bevorzugt von mindestens 95% aufweist.

Ein weiterer Gegenstand der vorliegenden Erfindung betrifft erfindungsgemäße genetisch modifizierte Pflanzenzellen oder erfindungsgemäße genetisch modifizierte Pflanzen, dadurch gekennzeichnet, dass sie ein in ihr Genom stabil integriertes fremdes Nucleinsäuremolekül oder mehrere in ihr Genom stabil integrierte fremde Nucleinsäuremoleküle aufweisen, wobei besagtes fremdes Nucleinsäuremolekül oder besagte fremde Nucleinsäuremoleküle zur Erhöhung der Aktivität eines Proteins mit der Aktivität einer GFAT und zur Erhöhung der Aktivität eines Proteins mit der Aktivität einer UDP-Glc-DH führt/führen, im Vergleich zu entsprechenden nicht genetisch modifizierten Wildtyp Pflanzenzellen bzw. zu entsprechenden nicht genetisch modifizierten Wildtyp Pflanzen.

Es kann sich dabei um ein einziges fremdes Nucleinsäuremolekül handeln, welches durch Integration in das Genom erfindungsgemäßer genetisch modifizierter Pflanzenzellen oder erfindungsgemäßer genetisch modifizierter Pflanzen zur Erhöhung der Aktivität eines Proteins mit der Aktivität einer GFAT und gleichzeitig zur Erhöhung der Aktivität eines Proteins mit der Aktivität einer UDP-Glc-DH führt, im Vergleich zu entsprechenden Wildtyp Pflanzenzellen bzw. zu entsprechenden Wildtyp Pflanzen. Es kann sich aber auch um mehrere fremde Nucleinsäuremoleküle handeln, von denen ein fremdes Nucleinsäuremolekül zur Erhöhung der Aktivität eines Proteins mit der Aktivität einer UDP-Glc-DH und ein anderes fremdes Nucleinsäuremolekül zur Erhöhung der Aktivität eines Proteins mit der Aktivität einer UDP-Glc-DH führt, im Vergleich zu entsprechenden Wildtyp Pflanzenzellen bzw. zu entsprechenden Wildtyp Pflanzen. Handelt es sich um mehrere fremde Nucleinsäuremoleküle, die in das Genom einer erfindungsgemäßen genetisch modifizierten Pflanzezelle oder einer erfindungsgemäßen genetisch modifizierten Pflanze integriert sind, so können sich beide fremde Nucleinsäuremoleküle gemeinsam an einem Ort im Genom der Pflanzezelle bzw. der Pflanze befinden oder sie können an verschiedenen Orten im Genom der Pflanzenzelle bzw. der Pflanze (z.B. auf verschiedenen Chromosomen oder verschieden Chromosomenabschnitten) lokalisiert sein. Die fremden Nucleinsäuremoleküle können daher entweder als gemeinsamer Locus oder als gekoppelte Loci nach den Mendel'schen Regeln vererbt werden oder sie können als getrennte Loci unabhängig voneinander nach den Mendel'schen Regeln vererbt werden.

Unter dem Begriff "fremdes Nukleinsäuremolekül" versteht man im Zusammenhang mit der vorliegenden Erfindung ein solches Molekül, das entweder natürlicherweise in entsprechenden Wildtyp-Pflanzenzellen nicht vorkommt, oder das in der konkreten räumlichen Anordnung nicht natürlicherweise in Wildtyp-Pflanzenzellen vorkommt oder das an einem Ort im Genom der Wildtyp-Pflanzenzelle lokalisiert ist, an dem es natürlicherweise nicht vorkommt. Bevorzugt ist das fremde Nukleinsäuremolekül ein rekombinantes Molekül, das aus verschiedenen Elementen besteht, deren Kombination oder spezifische räumliche Anordnung natürlicherweise in pflanzlichen Zellen nicht auftritt.

Unter dem Begriff "rekombinantes Nukleinsäuremolekül" soll im Zusammenhang mit der vorliegenden Erfindung ein Nukleinsäuremolekül verstanden werden, welches unterschiedliche Nucleinsäuremoleküle aufweist, die natürlicherweise nicht in einer Kombination vorliegen, wie sie in einem rekombinanten Nucleinsäuremolekül vorliegen. So können rekombinante Nucleinsäuremoleküle z.B., neben Nucleinsäuremolekülen, die eine Hyaluronansynthase und/oder ein Protein mit der Aktivität einer GFAT und/oder ein Protein mit der Aktivität einer UDP-Glc-DH codieren, zusätzliche Nucleinsäuresequenzen aufweisen, welche natürlicherweise nicht in Kombination mit den genannten Nucleinsäuremolekülen vorliegen. Die genannten zusätzlichen Nucleinsäuresequenzen, die auf einem rekombinanten Nucleinsäuremolekül in Kombination mit einem eine Hyaluronansynthase oder einem ein Protein mit der Aktivität einer GFAT und/oder einem ein Protein mit der Aktivität einer UDP-Glc-DH codierenden Nucleinsäuremolekül vorliegen, können dabei beliebige Sequenzen sein. Sie können z.B. genomische pflanzliche Nudeinsäuresequenzen darstellen. Bevorzugt handelt es sich bei genannten zusätzlichen Nucleinsäuresequenzen um regulatorische Sequenzen (Promotoren, Terminationssignale, Enhancer), besonders bevorzugt um regulatorische Sequenzen, die in pflanzlichem Gewebe aktiv sind, insbesondere bevorzugt um gewebespezifische regulatorische Sequenzen die in pflanzlichem Gewebe aktiv sind. Methoden zur Erzeugung rekombinanter Nucleinsäuremoleküle sind dem Fachmann bekannt und umfassen gentechnische Methoden wie z.B. die Verbindung von Nucleinsäuremolekülen durch Ligation, genetische Rekombination oder die Neusynthese von Nucleinsäuremolekülen (siehe z.B. Sambrok et al., Molecular Cloning, A Laboratory Manual, 3rd edition (2001) Cold Spring Harbour Laboratory Press, Cold Spring Harbour, NY. ISBN: 0879695773, Ausubel et al., Short Protocols in Molecular Biology, John Wiley & Sons; 5th edition (2002),ISBN: 0471250929).

Genetisch modifizierte Pflanzenzellen und genetisch modifizierte Pflanzen, die ein in ihr Genom stabil integriertes fremdes Nucleinsäuremolekül oder mehrere in ihr Genom stabil integrierte fremde Nucleinsäuremoleküle aufweisen, die eine Hyaluronansynthase codieren und die zur Erhöhung der Aktivität eines Proteins mit der Aktivität einer GFAT und zur Erhöhung der Aktivität eines Proteins mit der Aktivität einer UDP-Glc-DH führt/führen, im Vergleich zu entsprechenden nicht genetisch modifizierten Wildtyp Pflanzenzellen bzw. nicht genetisch modifizierten Wildtyp Pflanzen, lassen sich von besagten Wildtyp-Pflanzenzellen bzw. besagten Wildtyp-Pflanzen unter anderem dadurch unterscheiden, dass sie ein fremdes Nucleinsäuremolekül enthalten, das natürlicherweise in Wildtyp-Pflanzenzellen bzw. Wildtyp-Pflanzen nicht vorkommt oder dadurch, dass ein solches Molekül an einem Ort im Genom der erfindungsgemäßen genetisch modifizierten Pflanzenzelle oder im Genom der erfindungsgemäßen genetisch modifizierten Pflanze integriert vorliegt, an dem es bei Wildtyp-Pflanzenzellen bzw. Wildtyp-Pflanzen nicht vorkommt, d.h. in einer anderen genomischen Umgebung. Ferner lassen sich derartige erfindungsgemäße genetisch modifizierte Pflanzenzellen und erfindungsgemäße genetisch modifizierte Pflanzen von nicht genetisch modifizierten Wildtyp-Pflanzenzellen bzw. nicht genetisch modifizierten Wildtyp-Pflanzen dadurch unterscheiden, dass sie mindestens eine Kopie des fremden Nucleinsäuremoleküls stabil integriert in ihr Genom enthalten, gegebenenfalls zusätzlich zu natürlicherweise in den Wildtyp-Pflanzenzellen bzw. Wildtyp-Pflanzen vorkommenden Kopien eines solchen Moleküls. Handelt es sich bei dem (den) in die erfindungsgemäßen genetisch modifizierten Pflanzenzellen oder erfindungsgemäßen genetisch modifizierten Pflanzen eingeführten fremden Nucleinsäuremolekül(en) um zusätzliche Kopien zu bereits natürlicherweise in den Wildtyp-Pflanzenzellen bzw. Wildtyp-Pflanzen vorkommenden Molekülen, so lassen sich die erfindungsgemäßen genetisch modifizierten Pflanzenzellen und die erfindungsgemäßen genetisch modifizierten Pflanzen von Wildtyp-Pflanzenzellen bzw. Wildtyp-Pflanzen insbesondere dadurch unterscheiden, dass diese zusätzliche(n) Kopie(n) an Orten im Genom lokalisiert ist (sind), an denen sie bei Wildtyp-Pflanzenzellen bzw. Wildtyp-Pflanzen nicht vorkommt (vorkommen).
Nachgewiesen werden kann die stabile Integration eines Nucleinsäuremoleküls in das Genom einer Pflanzenzelle oder einer Pflanze durch genetische und/oder molekularbiologische Methoden. Eine stabile Integration eines Nucleinsäuremoleküls in das Genom einer Pflanzenzelle oder in das Genom einer Pflanze zeichnet sich dadurch aus, dass das stabil integrierte Nucleinsäuremolekül bei der Nachkommenschaft, der besagtes Nucleinsäuremolekül vererbt wurde, in derselben genomischen Umgebung vorliegt wie in der Elterngeneration. Das Vorliegen einer stabilen Integration einer Nucleinsäuresequenz im Genom einer Pflanzenzelle oder im Genom einer Pflanze kann mit dem Fachmann bekannten Methoden, u.a. mit Hilfe der Southern-Blot Analyse der RFLP-Analyse (Restriction Fragment Length Polymorphism) (Nam et al., 1989, The Plant Cell 1, 699-705; Leister and Dean, 1993, The Plant Journal 4 (4), 745-750), auf PCR basierenden Methoden, wie z.B. die Analyse von amplifizierten Fragment Längenunterschieden (Amplified Fragment Length Polymorphism, AFLP) (Castiglioni et al., 1998, Genetics 149, 2039-2056; Meksem et al., 2001, Molecular Genetics and Genomics 265, 207-214; Meyer et al., 1998, Molecular and General Genetics 259, 150-160) oder der Verwendung von mit Restriktionsendonucleasen geschnittenen amplifizierten Fragmenten (Cleaved Amplified Polymorphic Sequences, CAPS) (Konieczny und Ausubel, 1993, The Plant Journal 4, 403-410; Jarvis et al., 1994, Plant Molecular Biology 24, 685-687; Bachem et al., 1996, The Plant Journal 9 (5), 745-753) nachgewiesen werden.

Prinzipiell kann das fremde Nucleinsäuremolekül jedes beliebige Nucleinsäuremolekül sein, das in der Pflanzenzelle oder Pflanze eine Erhöhung der Aktivität eines Proteins mit der Aktivität einer GFAT und/oder eine Erhöhung der Aktivität eines Proteins mit der Aktivität einer UDP-Glc-DH bewirkt.

Im Zusammenhang mit der vorliegenden Erfindung können erfindungsgemäße genetisch modifizierte Pflanzenzellen und erfindungsgemäße genetisch modifizierte Pflanzen auch durch die Verwendung der so genannten Insertionsmutagenese (Übersichtsartikel: Thorneycroft et al., 2001, Journal of experimental Botany 52 (361), 1593-1601) hergestellt werden. Unter Insertionsmutagenese ist im Zusammenhang mit der vorliegenden Erfindung insbesondere das Inserieren von Transposons oder so genannter Transfer DNA (T-DNA) in ein Gen oder in die Nähe eines Gens, codierend ein Protein mit der Aktivität einer GFAT und/oder codierend ein Protein mit der Aktivität einer UDP-Glc-DH zu verstehen, wobei dadurch die Aktivität eines Proteins mit der Aktivität einer GFAT und/oder eines Proteins mit der Aktivität einer UDP-Glc-DH in der betreffenden Zelle erhöht wird.
Bei den Transposons kann es sich dabei sowohl um solche handeln, die in der Zelle natürlicherweise vorkommen (endogene Transposons), als auch um solche, die natürlicherweise nicht in besagter Zelle vorkommen, sondern mittels gentechnischer Methoden, wie z.B. Transformation der Zelle, in die Zelle eingeführt wurden (heterologe Transposons). Die Veränderung der Expression von Genen mittels Transposons ist dem Fachmann bekannt. Eine Übersicht über die Nutzung von endogenen und heterologen Transposons als Werkzeuge in der Pflanzenbiotechnologoie ist in Ramachandran und Sundaresan (2001, Plant Physiology and Biochemistry 39, 234-252) dargestellt.

Die T-DNA Insertionsmutagenese beruht darauf, dass bestimmte Abschnitte (T-DNA) von Ti-Plasmiden aus Agrobacterium in das Genom von pflanzlichen Zellen integrieren können. Der Ort der Integration in das pflanzliche Chromosom ist dabei nicht festgelegt, sondern kann an jeder beliebigen Stelle erfolgen. Integriert die T-DNA in einen Abschnitt oder in die Nähe eines Abschnittes des Chromosoms, der eine Genfunktion darstellt, so kann dieses zur Erhöhung der Genexpression und damit auch zur Änderung der Aktivität eines durch das betreffende Gen codierten Proteins führen. Die in das Genom inserierten Sequenzen (insbesondere Transposons oder T-DNA) zeichnen sich dabei dadurch aus, dass sie Sequenzen enthalten, die zu einer Aktivierung von regulatorischen Sequenzen eines Gens, codierend ein Protein mit der Aktivität einer GFAT und/oder codierend ein Protein mit der Aktivität einer UDP-Glc-DH führen ("activation tagging"). Bevorzugt zeichnen sich in das Genom inserierten Sequenzen (insbesondere Transposons oder T-DNA) dadurch aus, dass sie in der Nähe von endogenen Nucleinsäuremolekülen im Genom der Pflanzenzelle oder der Pflanze integriert sind, die ein Protein mit der Aktivität einer GFAT und/oder ein Protein mit der Aktivität einer UDP-Glc-DH codieren.

Erfindungsgemäße genetisch modifizierte Pflanzenzellen und genetisch modifizierte Pflanzen können z.B. mit Hilfe der Methode des so genannten "activation taggings" (siehe z. B. Walden et al., Plant J. (1991), 281-288; Walden et al., Plant Mol. Biol. 26 (1994), 1521-1528) erzeugt werden. Diese Methode beruht auf der Aktivierung endogener Promotoren durch "enhancer"-Sequenzen, wie z.B. dem Enhancer des 35S RNA-Promoters des Blumenkohlmosaikvirus oder dem Octopinsynthase-Enhancers.

Unter dem Begriff "T-DNA activation tagging" soll im Zusammenhang mit der vorliegenden Erfindung ein T-DNA Fragment verstanden werden, das "enhancer"-Sequenzen enthält und durch Integration in das Genom einer Pflanzenzelle zu der Erhöhung der Aktivität eines Proteins mit der Aktivität einer GFAT und/oder eines Proteins mit der Aktivität einer UDP-Glc-DH führt.

Unter dem Begriff "Transposon activation tagging" soll im Zusammenhang mit der vorliegenden Erfindung ein Transposon verstanden werden, das "enhancer"-Sequenzen enthält und durch Integration in das Genom einer Pflanzenzelle zu der Erhöhung der Aktivität eines Proteins mit der Aktivität einer GFAT und/oder eines Proteins mit der Aktivität einer UDP-Glc-DH führt.

Eine bevorzugte Ausführungsform der vorliegenden Erfindung betrifft erfindungsgemäße genetisch modifizierte Pflanzenzellen oder erfindungsgemäße genetisch modifizierte Pflanzen, dadurch charakterisiert, dass mindestens ein fremdes Nucleinsäuremolekül ein Protein mit der enzymatischen Aktivität einer GFAT oder dass mindestens ein fremdes Nucleinsäuremolekül ein Protein mit der enzymatischen Aktivität einer UDP-Glc-DH codiert.

Eine besonders bevorzugte Ausführungsform der vorliegenden Erfindung betrifft erfindungsgemäße genetisch modifizierte Pflanzenzellen oder erfindungsgemäße genetisch modifizierte Pflanzen, dadurch charakterisiert, dass ein erstes fremdes Nucleinsäuremolekül ein Protein mit der enzymatischen Aktivität einer GFAT codiert und ein zweites fremdes Nucleinsäuremolekül ein Protein mit der enzymatischen Aktivität einer UDP-Glc-DH codiert.
Erfindungsgemäß kann das fremde Nucleinsäuremolekül codierend ein Protein mit der enzymatischen Aktivität einer GFAT aus einem beliebigen Organismus stammen, bevorzugt stammt besagtes Nucleinsäuremolekül aus Bakterien, Pilzen, Tieren, Pflanzen oder Viren, besonders bevorzugt aus Säugetieren, Pflanzen oder Bakterien und insbesondere bevorzugt aus Maus oder *Escherichia coli.*
Bezüglich Viren stammt das das fremde Nucleinsäuremolekül codierend ein Protein mit der enzymatischen Aktivität einer GFAT vorzugsweise aus einem Virus, der Algen infiziert, bevorzugt aus einem Virus, der Algen der Gattung *Chlorella* infiziert, besonders bevorzugt aus einem *Paramecium bursaria Chlorella* Virus und insbesondere bevorzugt aus einem *Paramecium bursaria Chlorella* Virus eines H1 Stammes.
Anstelle eines natürlich vorkommenden Nucleinsäuremoleküls, codierend ein Protein mit der enzymatischen Aktivität einer GFAT kann auch ein mittels Mutagenese erzeugtes Nucleinsäuremolekül in erfindungsgemäße genetisch modifizierte Pflanzenzellen oder erfindungsgemäße genetisch modifizierte Pflanzen eingeführt sein, wobei sich besagtes mutagenisierte fremde Nucleinsäuremolekül dadurch auszeichnet, dass es ein Protein mit der enzymatischen Aktivität einer GFAT codiert, das eine verringerte Inhibition durch Stoffwechselmetabolite (z.B. des Glucosaminstoffwechsels) aufweist. Die Herstellung solcher mutagenisierten Nucleinsäuremoleküle ist für ein Protein mit der enzymatischen Aktivität einer GFAT aus *Escherichia coli* exemplarisch beschrieben in Deng et al. (2005, Metabolic Engeneering 7, 201-214; WO 04 003175). Mutanten für ein Protein mit der Aktivität einer GFAT aus Maus sind z.B. bechreiben bei Hu et al. (2004, J. Biol. Chem. 279 (29), 29988-29993).

Erfindungsgemäß kann das fremde Nucleinsäuremolekül codierend ein Protein mit der enzymatischen Aktivität einer UDP-Glc-DH aus einem beliebigen Organismus stammen, bevorzugt stammt besagtes Nucleinsäuremolekül aus Bakterien, Pilzen, Tieren, Pflanzen oder Viren, besonders bevorzugt aus Bakterien, Pflanzen oder Viren, insbesondere bevorzugt aus Viren.

Bezüglich Viren stammt das das fremde Nucleinsäuremolekül codierend ein Protein mit der enzymatischen Aktivität einer UDP-Glc-DH vorzugsweise aus einem Virus, der Algen infiziert, bevorzugt aus einem Virus, der Algen der Gattung *Chlorella* infiziert, besonders bevorzugt aus einem *Paramecium bursaria Chlorella* Virus und insbesondere bevorzugt aus einem *Paramecium bursaria Chlorella* Virus eines H1 Stammes. Anstelle eines natürlich vorkommenden Nucleinsäuremoleküls, codierend ein Protein mit der enzymatischen Aktivität einer UDP-Glc-DH kann auch ein mittels Mutagenese erzeugtes Nucleinsäuremolekül in erfindungsgemäße genetisch modifizierte Pflanzenzellen oder erfindungsgemäße genetisch modifizierte Pflanzen eingeführt sein, wobei sich besagtes mutagenisierte fremde Nucleinsäuremolekül dadurch auszeichnet, dass es ein Protein mit der enzymatischen Aktivität einer UDP-Glc-DH codiert, das eine verringerte Inhibition durch Stoffwechselmetabolite (z.B. des Glucoronsäurestoffwechsels) aufweist.

Nucleinsäuremoleküle, die ein Protein mit der Aktivität einer GFAT codieren sind dem Fachmann bekannt und in der Literatur beschrieben. So sind Nucleinsäuremoleküle, die ein Protein mit der Aktivität einer GFAT codieren aus Viren z.B. für den *Chlorella* Virus k2 (EMBL acc No AB107976.1), aus Bakterien z.B. für *Escherichia coli* (Dutka-Malen, 1988, Biochemie 70 (2), 287-290; EMBL acc No: L10328.1), aus Pilzen für z.B. *Saccharomyces cerevisiae* (EMBL acc No AF334737.1, Watzele et al., 1989, J. Biol. Chem. 264, 8753-8758), *Aspergillus niger* (EMBL acc No AY594332.1), *Candida albicans* (EMBL acc No X94753.1), aus Insekten für z.B. *Aedes aegyti* (Kato et al., *2002,* Insect. Biol. 11 (3), 207,216; EMBL acc No AF399922.1), *Drosophila melanogaster* (GFAT-1: EMBL acc No Y18627.1, GFAT-2: NCBI acc No NM_143360.2), aus Algen für *Volvariella volvacea* (EMBL acc No AY661466.1), aus Vertebraten für z.B. *Homo sapiens* (GFAT-1: EMBL acc No AF334737.1; GFAT-2: NCBI acc No BC000012.2, Oki et al., 1999, Genomics 57 (2), 227-34), *Mus musculus* (GFAT-1: EMBL acc No AF334736.1; GFAT-2: EMBL acc No AB016780.1), oder aus Pflanzen für z.B. *Arabidopsis thaliana* (EMBLI acc No AP001297.1; cds NCBI acc No BAB03027.1) beschrieben.

In einer bevorzugten Ausführungsform betrifft die vorliegende Erfindung erfindungsgemäße genetisch modifizierte Pflanzenzellen und erfindungsgemäße genetisch modifizierte Pflanzen, wobei das fremde Nucleinsäuremolekül codierend ein Protein mit der Aktivität einer GFAT ausgewählt ist, aus der Gruppe bestehend aus

a) Nucleinsäuremolekülen, die ein Protein mit der unter SEQ ID NO 8 oder Protein mit der unter SEQ ID NO 10 oder ein Protein mit der unter SEQ ID NO 12) angegebenen Aminosäuresequenz codieren;
b) Nucleinsäuremolekülen, die ein Protein codieren, dessen Sequenz eine Identität von mindestens 60% zu der unter SEQ ID NO 8 unter SEQ ID NO 10 oder zu der unter SEQ ID NO 12 angegebenen Aminosäuresequenz aufweisen;
c) Nucleinsäuremolekülen, die die unter SEQ ID NO 7 oder die unter SEQ ID NO 9 oder die unter SEQ ID NO 11 die unter SEQ ID NO 13 dargestellte Nucleotidsequenz oder eine komplementäre Sequenz umfassen;
d) Nucleinsäuremolekülen, welche zu den unter a) oder c) beschriebenen Nucleinsäuresequenzen eine Identität von mindestens 70% aufweisen;
e) Nucleinsäuremolekülen, welche mit mindestens einem Strang der unter a) oder c) beschriebenen Nucleinsäuresequenzen unter stringenten Bedingungen hybridisieren;
f) Nucleinsäuremolekülen, deren Nucleotidsequenz aufgrund der Degeneration des genetisches Codes von der Sequenz der unter a) oder c) genannten Nucleinsäuremoleküle abweicht; und
g) Nucleinsäuremolekülen, die Fragmente, allelische Varianten und/oder Derivate der unter a), b), c), d), e) oder f) genannten Nucleinsäuremoleküle darstellen.

Nucleinsäuremoleküle, die ein Protein mit der Aktivität einer UDP-Glc-DH codieren sind in der Literatur beschrieben und dem Fachmann bekannt. So sind Nucleinsäuremoleküle, die ein Protein mit der Aktivität einer UDP-Glc-DH codieren aus Viren z.B. für den *Chlorella* Virus 1 (NCBI acc No NC_000852.3), aus Bakterien z.B. für *Escherichia coli* (EMBL acc No: AF176356.1), aus Pilzen für z.B. *Aspergillus niger* (EMBL acc No AY594332.1), *Cryptococcus neoformans* (EMBL acc AF405548.1), aus Insekten für z.B. *Drosophila melanogaster* (EMBL acc No AF001310.1), aus Vertebraten für z.B. *Homo sapiens* (EMBL acc No AF061016.1), *Mus musculus* (EMBL acc No AF061017.1), *Bos taurus* (EMBL acc No AF095792.1), *Xenopus laevis* (EMBL acc No AY762616.1) oder aus Pflanzen für z.B. Pappel (EMBL acc No AF053973.1), *Colocasia esculenta* (EMBL acc No AY222335.1), *Dunaliella salina* (EMBL acc No AY795899.1), *Glycine max* (EMBL acc No U53418.1) beschrieben.

In einer weiteren bevorzugten Ausführungsform betrifft die vorliegende Erfindung erfindungsgemäße genetisch modifizierte Pflanzenzellen und erfindungsgemäße genetisch modifizierte Pflanzen, wobei das fremde Nucleinsäuremolekül codierend ein Protein mit der Aktivität einer UDP-Glc-DH ausgewählt ist, aus der Gruppe bestehend aus

a) Nucleinsäuremolekülen, die ein Protein mit der unter SEQ ID NO 5 angegebenen Aminosäuresequenz codieren;
b) Nucleinsäuremolekülen, die ein Protein codieren, dessen Sequenz eine Identität von mindestens 60% zu der unter SEQ ID NO 5 angegebenen Aminosäuresequenz aufweisen;

c) Nucleinsäuremolekülen, die die unter SEQ ID NO 4 oder die unter SEQ ID NO 6 dargestellte Nucleotidsequenz oder eine komplementäre Sequenz umfassen;

d) Nucleinsäuremolekülen, welche zu den unter a) oder c) beschriebenen Nucleinsäuresequenzen eine Identität von mindestens 70% aufweisen;

e) Nucleinsäuremolekülen, welche mit mindestens einem Strang der unter a) oder c) beschriebenen Nucleinsäuresequenzen beschriebenen Nucleinsäuremolekülen unter stringenten Bedingungen hybridisieren;

f) Nucleinsäuremolekülen, deren Nucleotidsequenz aufgrund der Degeneration des genetisches Codes von der Sequenz der unter a) oder c) genannten Nucleinsäuremoleküle abweicht; und

g) Nucleinsäuremolekülen, die Fragmente, allelische Varianten und/oder Derivate der unter a), b), c), d), e) oder f) genannten Nucleinsäuremoleküle darstellen.

Der Begriff "Hybridisierung" bedeutet im Rahmen der vorliegenden Erfindung eine Hybridisierung unter konventionellen Hybridisierungsbedingungen, vorzugsweise unter stringenten Bedingungen, wie sie beispielsweise in Sambrock et al., Molecular Cloning, A Laboratory Manual, 2. Aufl. (1989) Cold Spring Harbor Laboratory Press, Cold Spring Harbor, NY) beschrieben sind. Besonders bevorzugt bedeutet "Hybridisierung" eine Hybridisierung unter den folgenden Bedingungen:
Hybridisierungspuffer:
2xSSC; 10xDenhardt-Lösung (Fikoll 400+PEG+BSA; Verhältnis 1:1:1); 0,1% SDS; 5 mM EDTA; 50 mM Na2HPO4; 250 µg/ml Heringssperma DNA; 50 µg/ml tRNA; oder 25 M Natriumphoshphatpuffer pH 7,2; 1 mM EDTA; 7% SDS
Hybridisierungstemperatur:

| | |
|---|---|
| T=65 bis 68°C | |
| Waschpuffer: | 0,1xSSC; 0,1% SDS |
| Waschtemperatur: | T=65 bis 68°C. |

Nucleinsäuremoleküle, die mit Nucleinsäuremolekülen, codierend ein Protein mit der
Aktivität einer UDP-Glc-DH oder mit der Aktivität einer GFAT hybridisieren, können aus einem beliebigen Organismus stammen, sie können daher aus Bakterien, Pilzen, Tieren, Pflanzen oder sie können aus Viren stammen.
Nucleinsäuremoleküle, die mit Nucleinsäuremolekülen, codierend ein Protein mit der Aktivität einer UDP-Glc-DH hybridisieren, stammen vorzugsweise aus einem Virus, der Algen infiziert, bevorzugt aus einem Virus, der Algen der Gattung *Chlorella* infiziert, besonders bevorzugt aus einem *Paramecium bursaria Chlorella* Virus und insbesondere bevorzugt aus einem *Paramecium bursaria Chlorella* Virus eines H1 Stammes.
Nucleinsäuremoleküle, die mit Nucleinsäuremolekülen, codierend ein Protein mit der Aktivität einer GFAT hybridisieren, stammen. besonders bevorzugt aus Säugetieren, Pflanzen oder Bakterien und insbesondere bevorzugt aus Maus oder *Escherichia coli.*
Nucleinsäuremoleküle, die mit Nucleinsäuremolekülen, codierend ein Protein mit der Aktivität einer GFAT-1 oder GFAT-2 hybridisieren, stammen bevorzugt aus einem eukaryontischen Organismus, besonders, bevorzugt stammen sie aus einem tierischen Organismus, insbesondere bevorzugt aus Maus.
Nucleinsäuremoleküle, die mit den genannten Molekülen hybridisieren können z.B. aus genomischen oder aus cDNA-Bibliotheken isoliert werden. Die Identifizierung und Isolierung derartiger Nucleinsäuremoleküle kann dabei unter Verwendung der genannten Nucleinsäuremoleküle oder Teile dieser Moleküle bzw. der reversen Komplemente dieser Moleküle erfolgen, z.B. mittels Hybridisierung nach Standardverfahren (siehe z.B. Sambrook et al., 1989, Molecular Cloning, A Laboratory Manual, 2. Aufl. Cold Spring Harbor Laboratory Press, Cold Spring Harbor, NY) oder durch Amplifikation mittels PCR.
Als Hybridisierungsprobe zur Isolierung einer Nucleinsäuresequenz, codierend ein Protein mit der Aktivität einer UDP-Glc-DH können z.B. Nucleinsäuremoleküle verwendet werden, die exakt die oder im Wesentlichen die unter SEQ ID NO 4 oder SEQ ID NO 6 angegebene Nucleotidsequenz oder Teile dieser Sequenzen aufweisen.
Als Hybridisierungsprobe zur Isolierung einer Nucleinsäuresequenz, codierend ein Protein mit der Aktivität einer GFAT können z.B. Nucleinsäuremoleküle verwendet werden, die exakt die oder im Wesentlichen die unter SEQ ID NO 7) oder zu der unter SEQ ID NO 9 oder zu der unter SEQ ID NO 11 oder zu der unter SEQ ID NO 13 angegebene Nucleotidsequenz oder Teile dieser Sequenzen aufweisen.
Bei den als Hybridisierungsprobe verwendeten Fragmenten kann es sich auch um synthetische Fragmente oder Oligonucleotide handeln, die mit Hilfe der gängigen Synthesetechniken hergestellt wurden und deren Sequenz im wesentlichen mit der eines in Rahmen der vorliegenden Erfindung beschriebenen Nucleinsäuremoleküls übereinstimmt. Hat man Gene identifiziert und isoliert, die mit den im Rahmen der vorliegenden Erfindung beschriebenen Nucleinsäuresequenzen hybridisieren, sollte eine Bestimmung der Sequenz und eine Analyse der Eigenschaften der von dieser Sequenz codierten Proteine erfolgen, um festzustellen, ob es sich um Proteine handelt, die die Aktivität einer GFAT, GFAT-1 oder GFAT-2 bzw. die Aktivität einer UDP-Glc-DH aufweisen. Methoden, wie bestimmt werden kann, ob ein Protein die Aktivität eines Proteins mit der Aktivität einer GFAT (z.B. Mayer et al., 1968, Plant Physiol. 43, 1097-1107; Deng et al., 2005, Metabolic Engeneering 7, 201-214), GFAT-1 oder GFAT-2 (z.B. Hu et al., 2004, J. Biol. Chem. 279 (29), 29988-29993) bzw. einer UDP-Glc-DH (z.B. De Luca et al., 1976, Connective Tissue Research 4, 247-254; Bar-Peled et6 al., 2004, Biochem. J. 381, 131-136; Turner und Botha, 2002, Archives Biochem. Biophys. 407, 209-216) aufweist, sind dem Fachmann bekannt und u.a. in der angegebenen Literatur beschrieben.

Die mit den im Rahmen der vorliegenden Erfindung beschriebenen Nucleinsäuremolekülen hybridisierenden Moleküle umfassen insbesondere Fragmente, Derivate und allelische Varianten der genannten Nucleinsäuremoleküle. Der Begriff "Derivat" bedeutet im Zusammenhang mit der vorliegenden Erfindung, dass die Sequenzen dieser Moleküle sich von den Sequenzen der oben beschriebenen Nucleinsäuremoleküle an einer oder mehreren Positionen unterscheiden und einen hohen Grad an Identität zu diesen Sequenzen aufweisen. Die Abweichungen zu den oben beschriebenen Nucleinsäuremolekülen können dabei z.B. durch Deletion, Addition, Substitution, Insertion oder Rekombination entstanden sein.

Der Begriff "Identität" bedeutet im Zusammenhang mit der vorliegenden Erfindung eine Sequenzidentität über die gesamte Länge der codierenden Region eines Nucleinsäuremoleküls oder die gesamte Länge einer Aminosäuresequenz, die ein Protein codiert, von mindestens 60%, insbesondere eine Identität von mindestens 70%, vorzugsweise von mindestens 80%, besonders bevorzugt von mindestens 90% und insbesondere bevorzugt von mindestens 95%. Unter dem Begriff "Identität" soll im Zusammenhang mit der vorliegenden Erfindung die Anzahl der übereinstimmenden Aminosäuren/Nucleotide (Identität) mit anderen Proteinen/Nucleinsäuren, ausgedrückt in Prozent verstanden werden. Bevorzugt wird die Identität betreffend ein Protein mit der Aktivität einer UDP-Glc-DH durch Vergleiche der unter SEQ ID NO 5 angegebenen Aminosäuresequenz bzw. die Identität betreffend ein Nucleinsäuremolekül codierend ein Protein mit der Aktivität einer UDP_Glc_DH durch Vergleiche der unter SEQ ID NO 4 oder SEQ ID NO 6 angegebenen Nucleinsäuresequenz, die Identität betreffend ein Protein mit der Aktivität einer GFAT durch Vergleiche der unter SEQ ID NO 8 oder SEQ ID NO 10 oder SEQ ID NO 12 angegebenen Aminosäuresequenz bzw. die Identität betreffend ein Nucleinsäuremolekül codierend ein Protein mit der Aktivität einer GFAT durch Vergleiche der unter SEQ ID NO 7 oder SEQ ID NO 9 oder SEQ ID NO 11 oder SEQ ID NO 13 angegebenen Nucleinsäuresequenz zu anderen Proteinen/Nucleinsäuren mit Hilfe von Computerprogrammen ermittelt. Weisen Sequenzen, die miteinander verglichen werden, unterschiedliche Längen auf, ist die Identität so zu ermitteln, dass die Anzahl an Aminosäuren, welche die kürzere Sequenz mit der längeren Sequenz gemeinsam hat, den prozentualen Anteil der Identität bestimmt. Vorzugsweise wird die Identität mittels des bekannten und der Öffentlichkeit zur Verfügung stehenden Computerprogramms ClustalW (Thompson et al., Nucleic Acids Research 22 (1994), 4673-4680) ermittelt. ClustalW wird öffentich zur Verfügung gestellt von Julie Thompson (Thompson@EMBL-Heidelberg.DE) und Toby Gibson (Gibson@EMBL-Heidelberg.DE), European Molecular Biology Laboratory, Meyerhofstrasse 1, D 69117 Heidelberg, Germany. ClustalW kann ebenfalls von verschiedenen Internetseiten, u.a. beim IGBMC (Institut de Génétique et de Biologie Moléculaire et Cellulaire, B.P.163, 67404 Illkirch Cedex, France; ftp://ftp-igbmc.u-strasbg.fr/pub/) und beim EBI (ftp://ftp.ebi.ac.uk/pub/software/) sowie bei allen gespiegelten Internetseiten des EBI (European Bioinformatics Institute, Wellcome Trust Genome Campus, Hinxton, Cambridge CB10 1SD, UK), heruntergeladen werden.

Vorzugsweise wird das ClustalW Computerprogramm der Version 1.8 benutzt, um die Identität zwischen im Rahmen der vorliegenden Erfindung beschriebenen Proteinen und anderen Proteinen zu bestimmen. Dabei sind folgende Parameter einzustellen: KTUPLE=1, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Vorzugsweise wird das ClustalW Computerprogramm der Version 1.8 benutzt, um die Identität zwischen z.B. der Nucleotidsequenz der im Rahmen der vorliegenden Erfindung beschriebenen Nucleinsäuremoleküle und der Nucleotidsequenz von anderen Nucleinsäuremolekülen zu bestimmen. Dabei sind folgende Parameter einzustellen: KTUPLE=2, TOPDIAGS=4, PAIRGAP=5, DNAMATRIX:IUB, GAPOPEN=10, GAPEXT=5, MAXDIV=40, TRANSITIONS: unweighted.

Identität bedeutet ferner, dass funktionelle und/oder strukturelle Äquivalenz zwischen den betreffenden Nucleinsäuremolekülen oder den durch sie codierten Proteinen, besteht. Bei den Nucleinsäuremolekülen, die homolog zu den oben beschriebenen Molekülen sind und Derivate dieser Moleküle darstellen, handelt es sich in der Regel um Variationen dieser Moleküle, die Modifikationen darstellen, die dieselbe biologische Funktion ausüben. Es kann sich dabei sowohl um natürlicherweise auftretende Variationen handeln, beispielsweise um Sequenzen aus anderen Spezies oder um Mutationen, wobei diese Mutationen auf natürliche Weise aufgetreten sein können oder durch gezielte Mutagenese eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Eine spezielle Form von Derivaten stellen z.B. Nucleinsäuremoleküle dar, die auf Grund der Degeneration des genetischen Codes von im Rahmen der vorliegenden Erfindung beschriebenen Nucleinsäuremolekülen abweichen.

Die von den verschiedenen Derivaten der Nucleinsäuremoleküle codierend ein Protein mit der Aktivität einer GFAT oder einer UDP-Glc-DH weisen bestimmte gemeinsame Charakteristika auf.
Dazu können z.B. biologische Aktivität, Substratspezifität, Molekulargewicht, immunologische Reaktivität, Konformation etc. gehören, sowie physikalische Eigenschaften wie z.B. das Laufverhalten in Gelelektrophoresen, chromatographisches Verhalten, Sedimentationskoeffizienten, Löslichkeit, spektroskopische Eigenschaften, Stabilität; pH-Optimum, Temperatur-Optimum etc..

Bevorzugte Eigenschaften von Proteinen mit der Aktivität einer oder einer UDP-Glc-DH sind dem Fachmann bekannt, wurden oben bereits erörtert und sind hier entsprechend anzuwenden.

In einer weiteren bevorzugten Ausführungsform betrifft die vorliegende Erfindung erfindungsgemäße genetisch modifizierte Pflanzenzellen oder erfindungsgemäße genetisch modifizierte Pflanzen, wobei Nucleinsäuremoleküle codierend ein Protein mit der enzymatischen Aktivität einer GFAT und/oder codierend ein Protein mit der enzymatischen Aktivität einer UDP-Glc-DH dadurch gekennzeichnet sind, dass die Codons besagter Nucleinsäuremoleküle verändert sind, im Vergleich zu den Codons der Nucleinsäuremoleküle, welche besagtes Protein mit der enzymatischen Aktivität einer GFAT bzw. codierend ein besagtes Protein mit der enzymatischen Aktivität einer UDP-Glc-DH des Ursprungsorganismus codieren. Besonders bevorzugt sind die Codons der Nucleinsäuremoleküle codierend ein Protein mit der enzymatischen Aktivität einer GFAT bzw. codierend ein Protein mit der enzymatischen Aktivität einer UDP-Glc-DH dahingehend verändert, dass sie an die Häufigkeit der Verwendung der Codons der Pflanzenzelle oder der Pflanze, in dessen Genom sie integriert sind oder werden, angepasst sind.

Ein weiterer Gegenstand der vorliegenden Erfindung betrifft erfindungsgemäße genetisch modifizierte Pflanzenzellen oder erfindungsgemäße genetisch modifizierte Pflanzen, dadurch gekennzeichnet, dass die stabil in das Genom der Pflanzenzelle oder der Pflanze integrierten fremden Nucleinsäuremoleküle, codierend eine Hyaluronansynthase und/oder codierend ein Protein mit der enzymatischen Aktivität einer GFAT und/oder codierend ein Protein mit der enzymatischen Aktivität einer UDP-Glc-DH mit regulatorischen Elementen verknüpft sind, die die Transkription in Pflanzenzellen initiieren (Promotoren). Es kann sich um homologe oder heterologe Promotoren handeln. Bei den Promotoren kann es sich um konstitutive, gewebespezifische, entwicklungsspezifische oder um durch äußere Einflüsse (z.B. nach Applikation chemischer Substanzen, durch Einwirkung abiotischer Faktoren wie Hitze und/oder Kälte, Trokkenheit, Krankheitsbefall etc.) regulierte Promotoren handeln. Dabei können Nucleinsäuremoleküle, codierend eine Hyaluronansynthase oder ein Protein mit der enzymatischen Aktivität einer GFAT oder ein Protein mit der enzymatischen Aktivität einer UDP-Glc-DH, die in das Genom einer erfindungsgemäßen genetisch modifizierten Pflanzenzelle oder einer erfindungsgemäßen genetisch modifizierten Pflanze integriert sind, mit jeweils demselben Promotor verknüpft sein, oder es können unterschiedliche Promotoren mit den einzelnen Sequenzen verknüpft sein. Es können dabei zwei oder drei verschiedene Promotoren in jeder beliebigen Kombination jeweils verknüpft mit einem betreffenden fremden Nucleinsäuremoleküle codierend eine Hyaluronansynthase oder ein Protein mit der enzymatischen Aktivität einer GFAT oder ein Protein mit der enzymatischen Aktivität einer UDP-Glc-DH in einer erfindungsgemäßen genetisch modifizierten Pflanzezelle oder einer erfindungsgemäßen genetisch modifizierten Pflanze vorliegen.

Eine bevorzugte Ausführungsform der vorliegenden Erfindung betrifft erfindungsgemäße genetisch modifizierte Pflanzenzellen oder erfindungsgemäße genetisch modifizierte Pflanzen, wobei mindestens ein fremdes Nucleinsäuremolekül, besonders bevorzugt mindestens zwei fremde Nucleinsäuremoleküle, insbesondere bevorzugt drei fremde Nucleinsäuremoleküle ausgewählt aus der Gruppe von Nucleinsäuremolekülen codierend eine Hyaluronansynthase oder ein Protein mit der enzymatischen Aktivität einer GFAT oder ein Protein mit der enzymatischen Aktivität einer UDP-Glc-DH mit einem gewebespezifische Promotoren verknüpft ist (sind). Bevorzugte gewebespezifische Promotoren stellen Promotoren dar, die die Initiation der Transkription spezifisch in pflanzlichen Knollen-, Frucht- oder Samenzellen oder Blättern initiieren.

Zur Expression von Nucleinsäuremolekülen, die eine Hyaluronansynthase oder ein Protein mit der enzymatischen Aktivität einer GFAT oder ein Protein mit der enzymatischen Aktivität einer UDP-Glc-DH codieren, werden diese vorzugsweise mit regulatorischen DNA-Sequenzen verknüpft, die die Transkription in pflanzlichen Zellen gewährleisten. Hierzu zählen insbesondere Promotoren. Generell kommt für die Expression jeder in pflanzlichen Zellen aktive Promotor in Frage.
Der Promotor kann dabei so gewählt sein, dass die Expression konstitutiv erfolgt oder nur in einem bestimmten Gewebe, zu einem bestimmten Zeitpunkt der Pflanzenentwicklung oder zu einem durch äußere Einflüsse determinierten Zeitpunkt.

Sowohl in Bezug auf die Pflanze als auch in Bezug auf das zu exprimierende Nucleinsäuremolekül kann der Promotor homolog oder heterolog sein.
Geeignete Promotoren sind z.B. der Promotor der 35S RNA des Cauliflower Mosaic Virus oder der Ubiquitin-Promotor aus Mais oder der *Cestrum* YLCV (Yellow Leaf Curling Virus; WO 01 73087; Stavolone et al., 2003, Plant Mol. Biol. 53, 703-713) für eine konstitutive Expression, der Patatingen-Promotor B33 (Rocha-Sosa et al., EMBO J. 8 (1989), 23-29) für eine knollenspezifische Expression in Kartoffeln oder ein fruchtspezifischer Promotor für Tomate wie z.B. der Polygalacturonase Promotor aus Tomate (Montgomery et al., 1993, Plant Cell 5, 1049-1062) oder der E8 Promotor aus Tomate (Metha et al., 2002, Nature Biotechnol. 20(6), 613-618) oder der ACC Oxidase Promotor aus Pfirsich (Moon und Callahan, 2004, J. Experimental Botany 55 (402), 1519-1528) oder ein Promotor, der eine Expression lediglich in photosynthetisch aktiven Geweben sicherstellt, z.B. der ST-LS1-Promotor (Stockhaus et al., Proc. Natl. Acad. Sci. USA 84 (1987), 7943-7947; Stockhaus et al., EMBO J. 8 (1989), 2445-2451) oder für eine endosperm-spezifische Expression der HMWG-Promotor aus Weizen, der USP-Promotor, der Phaseolinpromotor, Promotoren von Zein-Genen aus Mais (Pedersen et al., Cell 29 (1982), 1015-1026; Quatroccio et al., Plant Mol. Biol. 15 (1990), 81-93), Glutelin-Promotor (Leisy et al., Plant Mol. Biol. 14 (1990), 41-50; Zheng et al., Plant J. 4 (1993), 357-366; Yoshihara et al., FEBS Lett. 383 (1996), 213-218) oder Shrunken-1 Promotor (Werr et al., EMBO J. 4 (1985), 1373-1380). Es können jedoch auch Promotoren verwendet werden, die nur zu einem durch äußere Einflüsse determinierten Zeitpunkt aktiviert werden (siehe beispielsweise WO 9307279). Von besonderem Interesse können hierbei Promotoren von heat-shock Proteinen sein, die eine einfache Induktion erlauben. Ferner können samenspezifische Promotoren verwendet werden, wie z.B. der USP-Promoter aus *Vicia faba,* der eine samenspezifische Expression in Vicia faba und anderen Pflanzen gewährleistet (Fiedler et al., Plant Mol. Biol. 22 (1993), 669-679; Bäumlein et al., Mol. Gen. Genet. 225 (1991), 459-467).
Auch die Verwendung von Promotoren, die im Genom von Algen infizierenden Viren vorkommen, sind für die Expression von Nucleinsäuresequenzen in Pflanzen geeignet (Mitra et al., 1994, Biochem. Biophys Res Commun 204(1), 187-194; Mitra und Higgins, 1994, Plant Mol Biol 26(1), 85-93, Van Etten et al., 2002, Arch Virol 147, 1479-1516).

Unter dem Begriff "gewebespezifisch" soll im Zusammenhang mit der vorliegenden Erfindung die überwiegende Beschränkung einer Ausprägung (z.B. die Initiation der Transkription) auf ein bestimmtes Gewebe verstanden werden.

Unter den Begriffen "Knollen-, Frucht-, oder Samenzelle" sollen im Zusammenhang mit der vorliegenden Erfindung alle Zellen verstanden werden, die in einer Knolle, Frucht bzw. in einem Samen enthalten sind.

Unter dem Begriff "homologer Promotor" soll im Zusammenhang mit der vorliegenden Erfindung ein Promotor verstanden werden, der natürlicherweise in Pflanzenzellen oder Pflanzen vorkommt, die für die Herstellung erfindungsgemäßer genetisch modifizierter Pflanzenzellen bzw. erfindungsgemäßer genetisch modifizierter Pflanzen verwendet wurden (Homolog bezüglich der Pflanzenzelle oder Pflanze) oder ein Promotor verstanden werden, der die Regulation der Expression eines Gens in dem Organismus reguliert, aus dem die Sequenz isoliert wurde (homolog bezüglich des zu exprimierenden Nucleinsäuremoleküls).

Unter dem Begriff "heterologer Promotor" soll im Zusammenhang mit der vorliegenden Erfindung ein Promotor verstanden werden, der natürlicherweise nicht in Pflanzenzellen oder Pflanzen vorkommt, die für die Herstellung erfindungsgemäßer genetisch modifizierter Pflanzenzellen bzw. erfindungsgemäßer genetisch modifizierter Pflanzen verwendet wurden (heterolog bezüglich der Pflanzenzelle oder Pflanze) oder ein Promotor verstanden werden, der natürlicherweise in dem Organismus, aus dem eine zu exprimierende Nucleinsäuresequenz isoliert wurde, nicht zur Regulation der Expression besagter Nucleinsäuresequenz vorkommt.

Ferner kann eine Terminationssequenz (Polyandenylierungssignal) vorhanden sein, die der Addition eines Poly-A-Schwanzes an das Transkript dient. Dem Poly-A-Schwanz wird eine Funktion bei der Stabilisierung der Transkripte beigemessen. Derartige Elemente sind in der Literatur beschrieben (vgl. Gielen et al., EMBO J. 8 (1989), 23-29) und sind beliebig austauschbar. -

Es können auch Intronsequenzen zwischen dem Promotor und der codierenden Region vorhanden sein. Solche Intronsequenzen können zur Stabilität der Expression und zu einer erhöhten Expression in Pflanzen führen (Callis et al., 1987, Genes Devel. 1, 1183-1200; Luehrsen, and Walbot, 1991, Mol. Gen. Genet. 225, 81-93; Rethmeier et al., 1997; Plant Journal 12(4), 895-899; Rose and Beliakoff, 2000, Plant Physiol. 122 (2), 535-542; Vasil et al., 1989, Plant Physiol. 91, 1575-1579; XU et al., 2003, Science in China Series C Vol.46 No.6, 561-569). Geeignete Intronsequenzen sind beispielsweise das erste Intron des sh1-Gens aus Mais, das erste Intron des Poly-Ubiquitin Gens 1 aus Mais, das erste Intron des EPSPS Gens aus Reis oder eines der beiden ersten Introns des PAT1 Gens aus *Arabidopsis.*

Ein weiterer Gegenstand der vorliegenden Erfindung betrifft Pflanzen, enthaltend erfindungsgemäße genetisch modifizierte Pflanzenzellen. Derartige Pflanzen können durch Regeneration aus erfindungsgemäßen genetisch modifizierten Pflanzenzellen erzeugt werden.

Die vorliegende Erfindung betrifft auch verarbeitbare oder konsumierbare Teile von erfindungsgemäßen genetisch modifizierten Pflanzen, enthaltend erfindungsgemäße genetisch modifizierte Pflanzenzellen.

Unter dem Begriff "verarbeitbare Teile" sollen im Zusammenhang mit der vorliegenden Erfindung Pflanzenteile verstanden werden, die Verwendung in der Herstellung von Nahrungs- oder Futtermitteln finden, die als Rohstoffquelle für industrielle Prozesse, als Rohstoffquelle für die Herstellung pharmazeutischer oder als Rohstoffquelle für die Herstellung kosmetischer Produkte eingesetzt werden.

Unter dem Begriff "konsumierbare Teile" sollen im Zusammenhang mit der vorliegenden Erfindung Pflanzenteile verstanden werden, die dem Menschen als Nahrungsmittel dienen oder als Tierfutter verwendet werden.

Die vorliegende Erfindung betrifft auch Vermehrungsmaterial erfindungsgemäßer genetisch modifizierter Pflanzen, enthaltend eine erfindungsgemäße genetisch modifizierte Pflanzenzelle.

Der Begriff "Vermehrungsmaterial" umfasst dabei jene Bestandteile der Pflanze, die geeignet sind zur Erzeugung von Nachkommen auf vegetativem oder sexuellem Weg. Für die vegetative Vermehrung eignen sich beispielsweise Stecklinge, Calluskulturen, Rhizome oder Knollen. Anderes Vermehrungsmaterial umfasst beispielsweise Früchte, Samen, Sämlinge, Protoplasten, Zellkulturen, etc. Vorzugsweise handelt es sich bei dem Vermehrungsmaterial um Knollen, Früchte oder Samen.

In einer weiteren Ausführungsform betrifft die vorliegende Erfindung erntebare Pflanzenteile erfindungsgemäßer genetisch modifizierter Pflanzen, wie Früchte, Speicherwurzeln, Wurzeln, Blüten, Knospen, Sprosse, Blätter oder Stämme, vorzugsweise Samen, Früchte oder Knollen, wobei diese erntebaren Teile erfindungsgemäße genetisch modifizierte Pflanzenzellen enthalten.

Bevorzugt betrifft die vorliegende Erfindung erfindungsgemäßes Vermehrungsmaterial oder erfindungsgemäße erntebare Teile von Pflanzen, enthaltend Hyaluronan. Besonders bevorzugt handelt es sich dabei um erfindungsgemäßes Vermehrungsmaterial oder erfindungsgemäße erntebare Teile von Pflanzen, die Hyaluronan synthetisieren.

Der Begriff "Kartoffelpflanze" oder "Kartoffel" meint im Zusammenhang mit der vorliegenden Erfindung Pflanzenspezies der Gattung *Solanum,* besonders Knollen produzierende Spezies der Gattung *Solanum* und insbesondere *Solanum tuberosum.*

Der Begriff "Tomatenpflanze" oder "Tomate" meint im Zusammenhang mit der vorliegenden Erfindung Pflanzenspezies der Gattung *Lycopersicon,* besonders *Lycopersicon esculentum.*

Ein weiterer Vorteil der vorliegenden Erfindung liegt darin, dass erntebare Teile, Vermehrungsmaterial, verarbeitbare Teile oder konsumierbare Teile von erfindungsgemäßen genetisch modifizierten Pflanzen mehr Hyaluronan enthalten als in der Literatur beschriebene transgene Pflanzen, die Hyaluronan synthetisieren. Erfindungsgemäße genetisch modifizierte Pflanzen sind daher nicht nur besonders geeignet zur Verwendung als Rohmaterial, aus dem Hyaluronan isoliert werden kann, sondern auch als direkte Verwendung als Nahrungs-/Futtermittel oder zur Herstellung von Nahrungs-/Futtermitteln, welche einen vorbeugenden oder therapeutischen Charakter aufweisen (z.B. zur Vorbeugung gegen Osteoarthritis, US 6,607,745). Da erfindungsgemäße genetisch modifizierte Pflanzen einen höheren Hyaluronangehalt aufweisen als in der Literatur beschriebene Pflanzen, müssen geringere Mengen an erntebaren Teilen, Vermehrungsmaterial, verarbeitbaren Teilen oder konsumierbaren Teilen von erfindungsgemäßen genetisch modifizierten Pflanzen eingesetzt werden, um solche Nahrungs-/Futtermittel herzustellen. Werden konsumierbare Teile von erfindungsgemäßen genetisch modifizierten Pflanzen z.B. direkt als so genanntes "Nutraceutical" konsumiert, so ist es möglich einen positiven Effekt bereits durch den Verzehr geringerer Mengen an Substanz zu erreichen. Diese kann u.a. bei der Herstellung von Tierfutter eine besondere Bedeutung erlangen, da Tierfutter mit einem zu hohen Gehalt an pflanzlichen Bestandteilen, für verschiedenste Tierarten nicht als Futtermittel geeignet ist.

Durch die hohe Wasserbindungskapazität von Hyaluronan weisen erntebare Teile, Vermehrungsmaterial, verarbeitbare Teile oder konsumierbare Teile von erfindungsgemäßen genetisch modifizierten Pflanzen weiterhin den Vorteil auf, dass bei der Herstellung von verfestigten Nahrungs-/Futtermitteln weniger Dickungsmittel eingesetzt werden müssen. So kann z.B. bei der Herstellung von Gelee weniger Zucker verwendet werden, was einen zusätzlichen positiven Gesundheitseffekt mit sich bringt. Bei der Herstellung von Nahrungs-/Futtermitteln, bei welchen es notwendig ist, dem pflanzlichen Rohstoff Wasser zu entziehen, besteht der Vorteil bei der Verwendung von erntebaren Teilen, Vermehrungsmaterial, verarbeitbaren Teilen oder konsumierbaren Teilen von erfindungsgemäßen genetisch modifizierten Pflanzen darin, dass dem betreffenden Pflanzenmaterial weniger Wasser entzogen werden muss, was zu geringern Produktionskosten führt und durch schonendere Herstellungsverfahren (z.B. geringere und/oder kürzere Wärmezufuhr) einen erhöhten Nährwert der betreffenden Nahrungs-/Futtermittel gewährleistet. So muss z.B. bei der Herstellung von Tomaten Ketchup weniger Energie zugeführt werden, um die gewünschte Konsistenz zu erreichen.

Ein weiterer Gegenstand der vorliegenden Erfindung betrifft ein Verfahren zur Herstellung einer Pflanze, die Hyaluronan synthetisiert, worin

a) eine Pflanzenzelle, genetisch modifiziert wird, wobei die genetische Modifikation die folgenden Schritte i bis iii umfasst

i) Einführung eines fremden Nucleinsäuremoleküls, codierend eine Hyaluronansynthase in die Pflanzenzelle

ii) Einführung einer genetischen Modifikation in die Pflanzenzelle, wobei die genetische Modifikation zur Erhöhung der Aktivität eines Proteins mit der enzymatischen Aktivität einer GFAT im Vergleich zu entsprechenden nicht genetisch modifizierten Wildtyp-Pflanzenzellen führt

iii) Einführung einer genetischen Modifikation in die Pflanzenzelle, wobei die genetische Modifikation zur Erhöhung der Aktivität eines Proteins mit der enzymatischen Aktivität einer UDP-Glc-DH im Vergleich zu entsprechenden nicht genetisch modifizierten Wildtyp-Pflanzenzellen führt

wobei die Schritte i bis iii in beliebiger Reihenfolge, einzeln oder beliebige Kombinationen der Schritte i bis iii gleichzeitig durchgeführt werden können

b) aus Pflanzenzellen von Schritt a) eine Pflanze regeneriert wird;

c) gegebenenfalls weitere Pflanzen mit Hilfe der Pflanzen nach Schritt b) erzeugt werden.,

wobei gegebenenfalls aus Pflanzen gemäß den Schritten b) oder c) Pflanzenzellen isoliert werden und die Verfahrensschritte a) bis c) solange wiederholt werden, bis eine Pflanze erzeugt wurde, die ein fremdes Nucleinsäuremolekül, codierend eine Hyaluronansynthase aufweist und eine erhöhte Aktivität eines Proteins mit der enzymatischen Aktivität einer GFAT im Vergleich zu entsprechenden nicht genetisch modifizierten Wildtyp-Pflanzenzellen aufweist und eine erhöhte Aktivität eines Proteins mit der enzymatischen Aktivität einer UDP-Glc-DH im Vergleich zu entsprechenden nicht genetisch modifizierten Wildtyp-Pflanzenzellen aufweist.

Ein bevorzugter Gegenstand der vorliegenden Erfindung betrifft Verfahren zur Herstellung einer Pflanze die Hyaluronan synthetisiert, worin

a) eine Pflanzenzelle, genetisch modifiziert wird, wobei die genetische Modifikation die folgenden Schritte i bis iii in beliebiger Reihenfolge umfasst oder beliebige Kombinationen der folgenden Schritte i bis iii einzeln oder gleichzeitig durchgeführt werden

i) Einführung eines fremden Nucleinsäuremoleküls, codierend eine Hyaluronansynthase in die Pflanzenzelle
ii) Einführung einer genetischen Modifikation in die Pflanzenzelle, wobei die genetische Modifikation zur Erhöhung der Aktivität eines Proteins mit der enzymatischen Aktivität einer GFAT, im Vergleich zu entsprechenden nicht genetisch modifizierten Wildtyp-Pflanzenzellen führt
iii) Einführung einer genetischen Modifikation in die Pflanzenzelle, wobei die genetische Modifikation zur Erhöhung der Aktivität eines Proteins mit der enzymatischen Aktivität einer Glutamin:Fructose-6-Phosphat UDP-Glc-DH im Vergleich zu entsprechenden nicht genetisch modifizierten Wildtyp-Pflanzenzellen führt

b) aus Pflanzenzellen enthaltend die genetische Modifikation gemäß den Schritten

i) a) i
ii) a) ii
iii) a) iii
iv) a) i und a) ii,
v) a) i und a) iii,
vi) a) ii und a) iii, oder
vii) a) i und a) ii und a) iii

eine Pflanze regeneriert wird
c) in Pflanzenzellen von Pflanzen gemäß Schritt

i) b) i eine genetische Modifikation gemäß Schritt a) ii,
ii) b) i eine genetische Modifikation gemäß Schritt a) iii,
iii) b) i eine genetische Modifikation gemäß Schritt a) ii und gleichzeitig eine genetische Modifikation gemäß Schritt a) iii,
iv) b) ii eine genetische Modifikation gemäß Schritt a) i,
v) b) ii eine genetische Modifikation gemäß Schritt a) iii,
vi) b) ii eine genetische Modifikation gemäß Schritt a) i und gleichzeitig eine genetische Modifikation gemäß Schritt a) iii,
vii) b) iii eine genetische Modifikation gemäß Schritt a) i,
viii) b) iii eine genetische Modifikation gemäß Schritt a) ii,
ix) b) iii eine genetische Modifikation gemäß Schritt a) i und gleichzeitig eine genetische Modifikation gemäß Schritt a) ii,
x) b) iv eine genetische Modifikation gemäß Schritt a) iii,
xi) b) v eine genetische Modifikation gemäß Schritt a) ii, oder
xii) b) vi eine genetische Modifikation gemäß Schritt a) i

eingeführt und eine Pflanze regeneriert wird
d) in Pflanzenzellen von Pflanzen gemäß Schritt

i) c) i eine genetische Modifikation gemäß Schritt a) iii,
ii) c) ii eine genetische Modifikation gemäß Schritt a) ii,
iii) c) iv eine genetische Modifikation gemäß Schritt a) iii,
iv) c) v eine genetische Modifikation gemäß Schritt a) ii,
v) c) vii eine genetische Modifikation gemäß Schritt a) ii,

vi) c) vii eine genetische Modifikation gemäß Schritt a) i, oder
vii) c) ix eine genetische Modifikation gemäß Schritt a) ii

eingeführt und eine Pflanze regeneriert wird
e) gegebenenfalls weitere Pflanzen mit Hilfe der Pflanzen nach einem der Schritte b) vii c) iii, c) vi, c) x, c) xi, c) xii oder nach einem der Schritte d) i bis d) vii erzeugt werden.

Für die laut Schritt a) in die Pflanzenzelle eingeführten genetischen Modifikationen gilt, dass es sich grundsätzlich um jede Art von Modifikation handeln kann, die zur Erhöhung der Aktivität eines Proteins mit der enzymatischen Aktivität einer GFAT und die zur Erhöhung der Aktivität eines Proteins mit der enzymatischen Aktivität einer UDP-Gluccose Dehydrogenase führt.

Die Regeneration der Pflanzen gemäß Schritt b) und gegebenenfalls Schritt c) und d) der erfindungsgemäßen Verfahren kann nach dem Fachmann bekannten Methoden erfolgen (z.B. beschrieben in "Plant Cell Culture Protocols", 1999, edt. by R.D. Hall, Humana Press, ISBN 0-89603-549-2).

Die Erzeugung weiterer Pflanzen (je nach Verfahren gemäß Schritt c) oder Schritt e)) der erfindungsgemäßen Verfahren kann z.B. erfolgen durch vegetative Vermehrung (beispielsweise über Stecklinge, Knollen oder über Calluskultur und Regeneration ganzer Pflanzen) oder durch sexuelle Vermehrung. Die sexuelle Vermehrung findet dabei vorzugsweise kontrolliert statt, d.h. es werden ausgewählte Pflanzen mit bestimmten Eigenschaften miteinander gekreuzt und vermehrt. Die Auswahl erfolgt dabei bevorzugt in der Weise, dass die weiteren Pflanzen (die je nach Verfahren gemäß Schritt c) oder Schritt e) erzeugt werden) die in den vorangegangenen Schritten eingeführten Modifikationen aufweisen.

In erfindungsgemäßen Verfahren zur Herstellung von Pflanzen, die Hyaluronan synthetisieren, können die genetischen Modifikationen zur Erzeugung der erfindungsgemäßen genetisch modifizierten Pflanzenzellen gleichzeitig oder in aufeinander folgenden Schritten und in jeder beliebigen Kombination erfolgen. Es kann sowohl von Wildtyp-Pflanzen bzw. Wildtyp-Pflanzenzellen ausgegangen werden, in die noch keine Einführung eines fremden Nucleinsäuremoleküls, codierend eine Hyaluronansynthase erfolgt ist und in denen noch keine vorherige genetische Modifikation, die zur Erhöhung der Aktivität eines Proteins mit der enzymatischen Aktivität einer GFAT im Vergleich zu entsprechenden nicht genetisch modifizierten Wildtyp-Pflanzenzellen führt, eingeführt wurde und in denen noch keine vorherige genetische Modifikation, die zur Erhöhung der Aktivität eines Proteins mit der enzymatischen Aktivität einer UDP-Glc-DH im Vergleich zu entsprechenden nicht genetisch modifizierten Wildtyp-Pflanzenzellen führt, eingeführt wurde oder es kann von bereits genetisch modifizierten Pflanzenzellen bzw. Pflanzen, in die bereits ein Nucleinsäuremolekül, codierend eine Hyaluronansynthase und/oder in die bereits eine genetische Modifikation zur Erhöhung der Aktivität eines Proteins mit der enzymatischen Aktivität einer GFAT im Vergleich zu entsprechenden nicht genetisch modifizierten Wildtyp-Pflanzenzellen eingeführt wurde und/oder in die bereits eine genetische Modifikation zur Erhöhung der Aktivität eines Proteins mit der enzymatischen Aktivität einer GFAT im Vergleich zu entsprechenden nicht genetisch modifizierten Wildtyp-Pflanzenzellen eingeführt wurde. Es ist dabei unerheblich, ob für auf einander folgende genetische Modifikationen, die zu einer erhöhten Aktivität eines Proteins mit der enzymatischen Aktivität einer GFAT führt, die gleiche Methode verwendet wird wie für die genetische Modifikation, die zu einer erhöhten Aktivität eines Proteins mit der enzymatischen Aktivität einer UDP-Glc-DH führt, solange beide genetische Modifikationen zusammen zu einer erhöhten Aktivität eines Proteins mit der enzymatischen Aktivität einer GFAT und eines Proteins mit der enzymatischen Aktivität einer UDP-Glc-DH in derselben Pflanzenzelle führen. Es ist dabei ebenfalls unerheblich, welche Methode für die Einführung eines fremden Nucleinsäuremoleküls, codierend eine Hyaluronansynthase in die Pflanzenzelle verwendet wird.

In einer weiteren Ausführungsform erfindungsgemäßer Verfahren zur Herstellung Pflanze, die Hyaluronan synthetisiert, besteht die genetische Modifikation in der Einführung mindestens eines fremden Nucleinsäuremoleküls in das Genom Pflanzenzelle, wobei das Vorhandensein oder die Expression des/der fremden Nucleinsäuremoleküls/Nukleinsäuremoleküle zu einer erhöhten Aktivität eines Proteins mit der enzymatischen Aktivität einer GFAT und eines Proteins mit der enzymatischen Aktivität einer UDP-Glc-DH in derselben Pflanzenzelle führt/führen.

In einer weiteren Ausführungsform erfindungsgemäßer Verfahren zur Herstellung Pflanze, die Hyaluronan synthetisiert, besteht die genetische Modifikation in der Einführung von mindestens einem fremden Nucleinsäuremolekül oder von mehreren fremden Nucleinsäuremolekülen in das Genom der Pflanzenzelle, wobei das/die fremde/fremden Nucleinsäuremolekül/Nukleinsäuremoleküle eine codierende Sequenz für eine Hyaluronansynthase und eine codierende Sequenz für ein Protein mit der enzymatischen Aktivität einer GFAT und eine codierende Sequenz für eine Protein mit der enzymatischen Aktivität einer UDP-Glc-DH umfassen.

Wie oben bereits für zur genetischen Modifikation in die Pflanzezelle oder Pflanze eingebrachten fremden Nukleinsäuremolekülen beschrieben, kann es sich in Schritt a) der erfindungsgemäßen Verfahren zur Herstellung einer Pflanze, die Hyaluronan synthetisiert, um ein einzelnes Nucleinsäuremolekül oder um mehrere Nucleinsäuremoleküle handeln. So können die fremden Nucleinsäuremoleküle, codierend eine Hyaluronansynthase bzw. codierend ein Protein mit der enzymatischen Aktivität einer GFAT bzw. codierend ein Protein mit der enzymatischen Aktivität einer UDP-Glc-DH zusammen auf einem einzigen Nucleinsäuremolekül vorliegen oder es können zwei der genannten fremden Nucleinsäuremoleküle zusammen auf einem einzigen Nucleinsäuremolekül und das dritte fremde Nucleinsäuremolekül auf einem andern Nucleinsäuremolekül in jeder möglichen Kombination vorliegen, oder es können alle drei genannten fremden Nucleinsäuremoleküle jeweils auf einzelnen, getrennten Nucleinsäuremolekülen vorliegen.

Weiterhin können zur Einführung eines fremden Nukleinsäuremoleküls bei der Durchführung erfindungsgemäßer Verfahren zur Herstellung einer Pflanze, die Hyaluronan synthetisiert, anstelle einer Wildtyp-Pflanzenzelle bzw. Wildtyp-Pflanze, Mutantenzellen bzw. Mutanten, die sich dadurch auszeichnen, dass sie bereits eine erhöhte Aktivität eines Proteins mit der enzymatischen Aktivität einer GFAT und/oder eine erhöhte Aktivität eines Proteins mit der enzymatischen Aktivität einer UDP-Glc-DH aufweisen, verwendet werden. Weist die Mutantenzelle bzw. die Mutante bereits eine erhöhte Aktivität eines Proteins mit der enzymatischen Aktivität einer GFAT oder eine erhöhte Aktivität eines Proteins mit der enzymatischen Aktivität einer UDP-Glc-DH im Vergleich zu entsprechenden Wildtyp Pflanzenzellen bzw. Wildtyp Pflanzen auf, so ist es zur Durchführung eines erfindungsgemäßen Verfahrens zur Herstellung einer Pflanze, die Hyaluronan synthetisiert, ausreichend, dass in besagte Mutantenzelle bzw. Mutante ein fremdes Nucleinsäuremolekül, codierend eine Hyaluronansynthase und eine genetische Modifikation, die zur Erhöhung der Aktivität eines Proteins mit der enzymatischen Aktivität einer UDP-Glc-DH bzw. zur Erhöhung der Aktivität eines Proteins mit der enzymatischen Aktivität einer GFAT im Vergleich zu entsprechenden nicht genetisch modifizierten Wildtyp-Pflanzenzellen führt, einzuführen. Weist die Mutantenzelle bzw. die Mutante bereits eine erhöhte Aktivität eines Proteins mit der enzymatischen Aktivität einer GFAT und eine erhöhte Aktivität eines Proteins mit der enzymatischen Aktivität einer UDP-Glc-DH im Vergleich zu entsprechenden Wildtyp Pflanzenzellen bzw. Wildtyp Pflanze auf, so kann zur Durchführung eines erfindungsgemäßen Verfahrens zur Herstellung einer Pflanze, die Hyaluronan synthetisiert, in besagte Mutantenzelle bzw. Mutante ein fremdes Nucleinsäuremolekül, codierend eine Hyaluronansynthase, eingeführt werden.

Die weiter oben gemachten Angaben zur Verwendung von Mutanten für die Herstellung erfindungsgemäßer genetisch modifizierter Pflanzenzellen oder erfindungsgemäßer genetisch modifizierter Pflanzen, sind hier entsprechend anzuwenden.

In bevorzugten Ausführungsformen betrifft die vorliegende Erfindung erfindungsgemäße Verfahren zur Herstellung einer Pflanze, die Hyaluronan synthetisiert, worin das Nucleinsäuremolekül, codierend eine Hyaluronansynthase in Schritt a) ausgesucht ist aus der Gruppe bestehend aus:

a) Nucleinsäuremoleküle, dadurch gekennzeichnet, dass sie eine virale Hyaluronansynthase codieren,
b) Nucleinsäuremoleküle, dadurch gekennzeichnet, dass sie eine Hyaluronansynthase eines Chlorella infizierenden Virus codieren,
c) Nucleinsäuremoleküle, dadurch gekennzeichnet, dass sie eine Hyaluronansynthase eines *Paramecium bursaria Chlorella* Virus 1 codieren,
d) Nucleinsäuremoleküle, dadurch gekennzeichnet, dass sie eine Hyaluronansynthase eines *Paramecium bursaria Chlorella* Virus 1 des Stammes H1 codieren,
e) Nucleinsäuremoleküle, dadurch gekennzeichnet, dass die Codons des Nucleinsäuremoleküls codierend eine Hyaluronansynthase verändert sind, im Vergleich zu den Codons des Nucleinsäuremoleküls, welches die Hyaluronansynthase des Ursprungsorganismus der Hyaluronansynthase codieren,
f) Nucleinsäuremoleküle, dadurch gekennzeichnet, dass die Codons der Hyaluronansynthase dahingehend verändert sind, dass sie an die Häufigkeit der Verwendung der Codons der Pflanzenzelle oder der Pflanze, in dessen Genom sie integriert werden oder sind, angepasst sind,
g) Nucleinsäuremoleküle, dadurch gekennzeichnet, dass sie eine Hyaluronansynthase mit der unter SEQ ID NO 2 dargestellten Aminosäuresequenz codieren oder dass sie eine Hyaluronansynthase codieren, dessen Aminosäuresequenz mit der unter SEQ ID No 2 dargestellten Aminosäuresequenz eine Identität von mindestens 70%, vorzugsweise von mindestens 80%, besonders bevorzugt von mindestens 90% und insbesondere bevorzugt von mindestens 95% aufweist,
h) Nucleinsäuremoleküle, dadurch gekennzeichnet , dass sie ein Protein codieren, dessen Aminosäuresequenz von der codierenden Region der im Plasmid DSM16664 inserierten Nucleinsäuresequenz abgeleitet werden kann oder dass sie ein Protein codieren, dessen Aminosäuresequenz mit der Aminosäuresequenz, die von der codierenden Region der im Plasmid DSM16664 inserierten Nucleinsäuresequenz abgeleitet werden kann, eine Identität von mindestens 70%, vorzugsweise von mindestens 80%, bevorzugt von mindestens 90% und insbesondere bevorzugt von mindestens 95% aufweist.,
i) Nucleinsäuremoleküle, die eine unter SEQ ID NO 1 oder SEQ I)D NO 3 dargestellte Nucleinsäuresequenz umfassen oder die zu der unter SEQ ID NO 1 oder SEQ I)D NO 3 dargestellten Nucleinsäuresequenz eine Identität von mindestens 70%, vorzugsweise von mindestens 80%, bevorzugt von mindestens 90% und insbesondere bevorzugt von mindestens 95% aufweisen
j) Nucleinsäuremoleküle, die die in Plasmid DSM16664 inserierte Nucleinsäuresequenz umfassen oder die mit der in Plasmid DSM16664 inserierten Nucleinsäuresequenz eine Identität von mindestens 70%, vorzugsweise von mindestens 80%, bevorzugt von mindestens 90% und insbesondere bevorzugt von mindestens 95% aufweisen

k) Nucleinsäuremoleküle, codierend eine Hyaluronansynthase, wobei die Hyaluronansynthase codierenden Nucleinsäuresequenzen mit regulatorischen Elementen (Promotor) verknüpft sind, die die Transkription in Pflanzenzellen initiieren oder

l) Nucleinsäuremoleküle, nach k), wobei die Promotoren gewebespezifische Promotoren, besonders bevorzugt Promotoren, die die Initiation der Transkription spezifisch in pflanzlichen Knollen-, Frucht- oder Samenzellen initiieren, darstellen.

In bevorzugten Ausführungsformen betrifft die vorliegende Erfindung erfindungsgemäße Verfahren zur Herstellung einer Pflanze, die Hyaluronan synthetisiert, worin das Nucleinsäuremolekül, codierend ein Protein mit der Aktivität einer GFAT ausgewählt ist, aus der Gruppe bestehend aus

a) Nucleinsäuremoleküle, dadurch gekennzeichnet, dass sie ein Protein mit der Aktivität einer GFAT codieren, welches aus Bakterien, Tieren oder Pflanzen, bevorzugt aus *Escherichia coli* oder Maus stammt,

b) Nucleinsäuremoleküle, dadurch gekennzeichnet, dass sie ein Protein mit der Aktivität einer GFAT eines *Chlorella* infizierenden Virus codieren,

c) Nucleinsäuremoleküle, dadurch gekennzeichnet, dass sie eine ein Protein mit der Aktivität einer GFAT eines *Paramecium bursaria Chlorella* Virus codieren,

d) Nucleinsäuremoleküle, dadurch gekennzeichnet, dass die Codons des Nucleinsäuremoleküls codierend ein Protein mit der Aktivität einer GFAT verändert sind, im Vergleich zu den Codons des Nucleinsäuremoleküls, welches das entsprechende Protein mit der Aktivität einer GFAT des Ursprungsorganismus der codiert,

e) Nucleinsäuremoleküle, dadurch gekennzeichnet, dass die Codons des Proteins mit der Aktivität einer GFAT dahingehend verändert sind, dass sie an die Häufigkeit der Verwendung der Codons der Pflanzenzelle oder der Pflanze, in dessen Genom sie integriert werden oder sind, angepasst sind,

f) Nucleinsäuremolekülen, die ein Protein mit der unter SEQ ID NO 8 oder ein Protein mit der unter SEQ ID NO 10 oder ein Protein mit der unter SEQ ID NO 12 angegebenen Aminosäuresequenz codieren;

g) Nucleinsäuremolekülen, die ein Protein codieren, dessen Sequenz eine Identität von mindestens 70%, vorzugsweise von mindestens 80%, bevorzugt von mindestens 90% und insbesondere bevorzugt von mindestens 95% zu der unter SEQ ID NO 8 oder zu der unter SEQ ID NO 10 oder zu der unter SEQ ID NO 12 angegebenen Aminosäuresequenz aufweisen;

h) Nucleinsäuremolekülen, die die unter SEQ ID NO 7 oder die unter SEQ ID NO 9 oder die unter SEQ ID NO 11 oder die unter SEQ ID NO 13 dargestellte Nucleinsäuresequenz oder eine komplementäre Sequenz umfassen;

i) Nucleinsäuremolekülen, welche zu den unter h) beschriebenen Nucleinsäuresequenzen eine Identität von mindestens 70%, vorzugsweise von mindestens 80%, bevorzugt von mindestens 90% und insbesondere bevorzugt von mindestens 95%;aufweisen,

j) Nucleinsäuremolekülen, welche mit mindestens einem Strang der unter f) oder h) beschriebenen Nucleinsäuresequenzen unter stringenten Bedingungen hybridisieren;

k) Nucleinsäuremolekülen, deren Nucleotidsequenz aufgrund der Degeneration des genetisches Codes von der Sequenz der unter f), oder h) genannten Nucleinsäuremoleküle abweicht; und

l) Nucleinsäuremolekülen, die Fragmente, allelische Varianten und/oder Derivate der unter a), b), c), d), e), f) oder h) genannten Nucleinsäuremoleküle darstellen,

m) Nucleinsäuremoleküle, codierend eine Protein mit der Aktivität einer GFAT, wobei die ein Protein mit der Aktivität einer GFAT codierenden Nucleinsäuresequenzen mit regulatorischen Elementen (Promotor) verknüpft sind, die die Transkription in Pflanzenzellen initiieren oder

n) Nucleinsäuremoleküle, nach m), wobei die Promotoren gewebespezifische Promotoren, besonders bevorzugt Promotoren, die die Initiation der Transkription spezifisch in pflanzlichen Knollen-, Blatt-, Frucht- oder Samenzellen initiieren, darstellen.

In bevorzugten Ausführungsformen betrifft die vorliegende Erfindung erfindungsgemäße Verfahren zur Herstellung einer Pflanze, die Hyaluronan synthetisiert, worin das fremde Nucleinsäuremolekül codierend ein Protein mit der Aktivität einer UDP-Glc-DH ausgewählt ist, aus der Gruppe bestehend aus

a) Nucleinsäuremoleküle, dadurch gekennzeichnet, dass sie ein Protein mit der Aktivität einer UDP-Glc-DH codieren, welches aus Viren, Bakterien, Tieren oder Pflanzen, stammt,

b) Nucleinsäuremoleküle, dadurch gekennzeichnet, dass sie ein Protein mit der Aktivität einer UDP-Glc-DH eines *Chlorella* infizierenden Virus codieren,

c) Nucleinsäuremoleküle, dadurch gekennzeichnet, dass sie ein Protein mit der Aktivität einer UDP-Glc-DH eines *Paramecium bursaria Chlorella* Virus codieren,

d) Nucleinsäuremoleküle, dadurch gekennzeichnet, dass die Codons des Nucleinsäuremoleküls codierend ein Protein mit der Aktivität einer UDP-Glc-DH verändert sind, im Vergleich zu den Codons des Nucleinsäuremoleküls, welches das entsprechende Protein mit der Aktivität einer UDP-Glc-DH des Ursprungsorganismus codiert,
e) Nucleinsäuremoleküle, dadurch gekennzeichnet, dass die Codons des Proteins mit der Aktivität einer UDP-Glc-DH dahingehend verändert sind, dass sie an die Häufigkeit der Verwendung der Codons der Pflanzenzelle oder der Pflanze, in dessen Genom sie integriert werden oder sind, angepasst sind,
f) Nucleinsäuremolekülen, die ein Protein mit der unter SEQ ID NO 5 angegebenen Aminosäuresequenz codieren;
g) Nucleinsäuremolekülen, die ein Protein codieren, dessen Sequenz eine Identität von mindestens 70%, vorzugsweise von mindestens 80%, bevorzugt von mindestens 90% und insbesondere bevorzugt von mindestens 95% zu der unter SEQ ID NO 5 angegebenen Aminosäuresequenz aufweisen;
h) Nucleinsäuremolekülen, die die unter SEQ ID NO 4 oder die unter SEQ ID NO 6 dargestellte Nucleotidsequenz oder eine komplementäre Sequenz umfassen;
i) Nucleinsäuremolekülen, welche zu den unter h) beschriebenen Nucleinsäuresequenzen eine Identität von mindestens 70%, vorzugsweise von mindestens 80%, bevorzugt von mindestens 90% und insbesondere bevorzugt von mindestens 95% aufweisen;
j) Nucleinsäuremolekülen, welche mit mindestens einem Strang der unter f) oder h) beschriebenen Nucleinsäuremolekülen unter stringenten Bedingungen hybridisieren;
k) Nucleinsäuremolekülen, deren Nucleotidsequenz aufgrund der Degeneration des genetisches Codes von der Sequenz der unter f) oder h) genannten Nucleinsäuremoleküle abweicht; und
l) Nucleinsäuremolekülen, die Fragmente, allelische Varianten und/oder Derivate der unter a), b), c), d), e), f) oder h) genannten Nucleinsäuremoleküle darstellen,
m) Nucleinsäuremoleküle, codierend eine Protein mit der Aktivität einer UDP-Glc-DH, wobei die ein Protein mit der Aktivität einer UDP-Glc-DH codierenden Nucleinsäuresequenzen mit regulatorischen Elementen (Promotor) verknüpft sind, die die Transkription in Pflanzenzellen initiieren oder
n) Nucleinsäuremoleküle, nach m), wobei die Promotoren gewebespezifische Promotoren, besonders bevorzugt Promotoren, die die Initiation der Transkription spezifisch in pflanzlichen Knollen-, Blatt-, Frucht- oder Samenzellen initiieren, darstellen.

In einer vorzugsweisen Ausführungsform der vorliegenden Erfindung betreffen die Verfahren zur Herstellung einer Pflanze, die Hyaluronan synthetisiert, Verfahren zur Herstellung einer Pflanze, die mindestens 100 bevorzugt mindestens 600 besonders bevorzugt mindestens 1000 insbesondere bevorzugt mindestens 1500 $\mu$g Hyaluronan pro g Frischgewicht (FG) Pflanzenmaterial synthetisieren.

In einer weiteren bevorzugten Ausführungsform werden erfindungsgemäße Verfahren zur Herstellung einer Pflanze, die Hyaluronan synthetisiert zur Herstellung erfindungemäßer genetisch modifizierter Pflanzen verwendet.

Pflanzen erhältlich nach erfindungsgemäßen Verfahren zur Herstellung einer Pflanze, die Hyaluronan synthetisiert, sind ebenfalls Gegenstand der vorliegenden Erfindung.

Ein weiterer Gegenstand der vorliegenden Erfindung betrifft ein Verfahren zur Herstellung von Hyaluronan, umfassend den Schritt der Extraktion von Hyaluronan aus erfindungsgemäßen genetisch modifizierten Pflanzenzellen, aus erfindungsgemäßen genetisch modifizierten Pflanzen, aus erfindungsgemäßem Vermehrungsmaterial, aus erfindungsgemäßen erntebaren Pflanzenteilen oder aus Pflanzen oder Teilen dieser Pflanzen, erhältlich nach einem erfindungsgemäßen Verfahren zur Herstellung von Pflanzen, die Hyaluronan synthetisieren. Vorzugsweise umfasst ein solches Verfahren auch den Schritt des Erntens der kultivierten erfindungsgemäßen genetisch modifizierten Pflanzenzellen, der erfindungsgemäßen genetisch modifizierten Pflanzen, des erfindungsgemäßen Vermehrungsmaterials, der erfindungsgemäßen erntebaren Pflanzeteile, der erfindungsgemäßen verarbeitbaren Pflanzenteile vor der Extraktion des Hyaluronans und besonders bevorzugt ferner den Schritt der Kultivierung erfindungsgemäßer genetisch modifizierten Pflanzenzellen oder erfindungsgemäßer genetisch modifizierten Pflanzen vor dem Ernten.

Im Gegensatz zu bakteriellen oder tierischen Geweben weisen pflanzliche Gewebe keine Hyaluronidasen auf und enthalten keine Hyaladherine. Daher ist wie oben bereits beschrieben die Extraktion von Hyaluronan aus pflanzlichen Geweben mit Hilfe relativ einfacher Verfahren möglich. Die oben beschriebenen wässrigen Extrakte aus pflanzlichen Zellen oder Geweben, enthaltend Hyaluronan, können bei Bedarf mit dem Fachmann bekannten Methoden, wie z.B. von mehrfach hintereinander folgenden Fällungen mit Ethanol, weiter aufgereinigt werden. Eine bevorzugte Methode zur Aufreinigung von Hyaluronan ist unter Allgemeine Methoden Punkt 3 beschrieben.

Die bereits beschriebenen Verfahren zur Extraktion von Hyaluronan aus erfindungsgemäßen genetisch modifizierten Pflanzenzellen oder erfindungsgemäßen genetisch modifizierten Pflanzen sind auch für die Isolierung von Hyaluronan aus erfindungsgemäßem Vermehrungsmaterial, aus erfindungsgemäßen erntebaren Pflanzenteilen oder aus Pflanzen oder Teilen dieser Pflanzen, erhältlich nach einem erfindungsgemäßen Verfahren zur Herstellung von Pflanzen, die Hyaluronan synthetisieren, anwendbar.

Gegenstand der vorliegenden Erfindung ist auch die Verwendung erfindungsgemäßer genetisch modifizierter Pflanzenzellen, erfindungsgemäßer genetisch modifizierter Pflanzen, erfindungsgemäßen Vermehrungsmaterials, erfindungsgemäßen erntebaren Pflanzeteilen, erfindungsgemäßen verarbeitbaren Pflanzenteilen oder von Pflanzen, erhältlich nach einem erfindungsgemäßen Verfahren, zur Herstellung von Hyaluronan.

Ein weiterer Gegenstand der vorliegenden Erfindung betrifft Zusammensetzungen, enthaltend erfindungsgemäße genetisch modifizierte Pflanzenzellen. Es ist dabei unerheblich, ob die Pflanzenzellen intakt oder nicht mehr intakt sind, weil sie z.B. durch Prozessierungsverfahren zerstört wurden. Bevorzugt handelt es sich bei den Zusammensetzungen um Nahrungs- oder Futtermittel, um pharmazeutische oder um kosmetische Produkte.

Ein bevorzugter Gegenstand der vorliegenden Erfindung betrifft Zusammensetzungen, enthaltend Bestandteile von erfindungsgemäßen genetisch modifizierten Pflanzenzellen, von erfindungsgemäßen genetisch modifizierten Pflanzen, von erfindungsgemäßem Vermehrungsmaterial, von erfindungsgemäßen erntebaren Pflanzenteilen oder von Pflanzen, erhältlich nach einem erfindungsgemäßen Verfahren und enthaltend rekombinante Nucleinsäuremoleküle, wobei die rekombinanten Nucleinsäuremoleküle dadurch gekennzeichnet sind, dass sie Nucleinsäuremoleküle umfassen, die eine Hyaluronansynthase und Proteine mit der enzymatischen Aktivität einer GFAT und Proteine mit der enzymatischen Aktivität einer UDP-Glc-DH codieren.

Eine stabile Integration von fremden Nucleinsäuremolekülen in das Genom einer Pflanzenzelle oder Pflanze führt dazu, dass die fremden Nucleinsäuremoleküle nach Integration in das Genom der Pflanzenzelle oder Pflanze von genomischen pflanzlichen Nucleinsäuresequenzen flankiert sind.

In einer bevorzugten Ausführungsform sind erfindungsgemäße Zusammensetzungen daher dadurch gekennzeichnet, dass die rekombinanten Nucleinsäuremoleküle, enthaltend in der erfindungsgemäßen Zusammensetzung von genomischen pflanzlichen Nucleinsäuresequenzen flankiert sind.

Die genomischen pflanzlichen Nucleinsäuresequenzen können dabei beliebige Sequenzen sein, die im Genom der Pflanzenzelle oder Pflanze, die zur Herstellung der Zusammensetzung verwendet wurde, natürlicherweise vorliegen.

Bei den in erfindungsgemäßen Zusammensetzungen enthaltenden rekombinanten Nucleinsäuremolekülen kann es sich um einzelne oder verschiedene rekombinante Nucleinsäuremoleküle handeln, bei welchen Nucleinsäuremoleküle, die eine Hyaluronansynthase und Proteine mit der enzymatischen Aktivität einer GFAT und Proteine mit der enzymatischen Aktivität einer UDP-Glc-DH codieren, auf einem Nucleionsäuremolekül vorliegen, oder um solche, bei welchen die genannten Nucleinsäuremoleküle auf getrennten rekombinanten Nucleinsäuremolekülen vorliegen handeln. Es können Nucleinsäuremoleküle, codierend eine Hyaluronansynthase bzw. codierend ein Protein mit der enzymatischen Aktivität einer GFAT bzw. codierend ein Protein mit der enzymatischen Aktivität einer UDP-Glc-DH zusammen auf einem einzigen rekombinanten Nucleinsäuremolekül vorliegen oder es können zwei der genannten Nucleinsäuremoleküle zusammen auf einem einzigen rekombinanten Nucleinsäuremolekül und das dritte Nucleinsäuremolekül auf einem anderen rekombinanten Nucleinsäuremolekül in jeder möglichen Kombination vorliegen, oder es können alle drei genannten Nucleinsäuremoleküle jeweils auf einzelnen, getrennten rekombinanten Nucleinsäuremolekülen vorliegen. Je nachdem, in welcher Weise die Nucleinsäuremoleküle, codierend eine Hyaluronansynthase bzw. codierend ein Protein mit der enzymatischen Aktivität einer GFAT bzw. codierend ein Protein mit der enzymatischen Aktivität einer UDP-Glc-DH in einer erfindungsgemäßen Zusammensetzung vorliegen, können sie von identischen oder von unterschiedlichen genomischen pflanzlichen Nucleinsäuresequenzen flankiert sein.

Dass erfindungsgemäße Zusammensetzungen rekombinante Nucleinsäuremoleküle enthalten, kann mit dem Fachmann bekannten Methoden, wie z.B. auf Hybridisierung basierenden Methoden oder bevorzugt mit auf PCR (Polymerase Chain Reaction) basierenden Methoden nachgewiesen werden.

Vorzugsweise enthalten erfindungsgemäße Zusammensetzungen mindestens 0,005%, bevorzugt mindestens 0.01%, besonders bevorzugt mindestens 0,05%, insbesondere bevorzugt mindestens 0,1% Hyaluronan.

Wie oben bereits ausgeführt können erfindungsgemäße genetisch modifizierte Pflanzenzellen, erfindungsgemäße genetisch modifizierte Pflanzen, erfindungsgemäßes Vermehrungsmaterial, erfindungsgemäße erntebare Pflanzeteile, erfindungsgemäße verarbeitbare Pflanzenteile, erfindungsgemäßen konsumierbare Pflanzenteile oder Pflanzen, erhältlich nach einem erfindungsgemäßen Verfahren verwendet werden, um Nahrungs- oder Futtermittel herzustellen. Aber auch die Verwendung als Rohstoff für technische Anwendungen ist möglich, ohne dass Hyaluronan isoliert werden muss. So können z.B. erfindungsgemäße genetisch modifizierte Pflanzen bzw. Teile von erfindungsgemäßen genetisch modifizierten Pflanzen auf Ackerflächen aufgebracht werden, um eine erhöhte Wasserbindung des Bodens zu erreichen. Weiterhin ist eine Verwendung von erfindungsgemäßen genetisch modifizierten Pflanzen oder erfindungsgemäßen genetisch modifizierten Pflanzenzellen zur Herstellung von Trocknungsmitteln (z.B. für die Verwendung beim Versand von Gegenständen, die empfindlich auf Feuchtigkeit reagieren) oder als Absorber von Flüssigkeiten (z.B. in Babywindeln, oder zum Aufsaugen ausgelaufener wässriger Flüssigkeiten) möglich. Für solche Anwendungen können je nach Bedarf ganze erfindungsgemäße genetisch modifizierte Pflanzen, Teile von erfindungsgemäßen genetisch modifizierten Pflanzen oder zerkleinerte (z.B. zermahlene) erfindungsgemäße genetisch modifizierten Pflanzen oder erfindungsgemäße Pflanzenteile Verwendung finden. Insbesondere für Gebiete, in denen gemahlene Pflanzen oder Pflanzenteile zur Anwendung kommen, sind Pflanzenteile, die Hyaluronan enthalten, jedoch selbst einen geringen Wasseranteil aufweisen. Es handelt es sich hierbei bevorzugt um Körner von Getreidepflanzen (Mais, Reis, Weizen, Roggen, Hafer, Gerste, Sago, oder Sorghum). Da erfindungsgemäße genetisch modifizierte Pflanzenzellen und erfindungsgemäße genetisch modifizierte Pflanzen einen höheren Gehalt an Hyaluronan aufweisen, als in der Literatur beschriebene transgene Pflanzen, muss im Vergleich zu diesen für technische Anwendungen weniger Material eingesetzt werden, wenn erfindungsgemäße genetisch modifizierte Pflanzenzellen oder erfindungsgemäße genetisch modifizierte Pflanzen verwendet werden.

Weiterhin sind Gegenstand der vorliegenden Erfindung Verfahren zur Herstellung einer erfindungsgemäßen Zusammensetzung, worin erfindungsgemäße genetisch modifizierte Pflanzenzellen, erfindungsgemäße genetisch modifizierte Pflanzen, erfindungsgemäßes Vermehrungsmaterial, erfindungsgemäße erntebare Pflanzeteile, erfindungsgemäße verarbeitbare Pflanzenteile, erfindungsgemäße konsumierbare Pflanzenteile oder Pflanzen, erhältlich nach einem erfindungsgemäßen Verfahren zur Herstellung einer Pflanze, die Hyaluronan synthetisiert, verwendet werden. Bevorzugt handelt es sich bei den Verfahren zur Herstellung einer erfindungsgemäßen Zusammensetzung um Verfahren zur Herstellung von Nahrungs- oder Futtermitteln, Verfahren zur Herstellung eines pharmazeutischen oder Verfahren zur Herstellung zur Herstellung eines kosmetischen Produktes.

Verfahren zur Herstellung von Lebens- oder Futtermitteln sind dem Fachmann bekannt. Verfahren für die Verwendung von erfindungsgemäßen genetisch modifizierten Pflanzen oder erfindungsgemäßen Pflanzenteilen auf technischen Gebieten sind dem Fachmann ebenfalls bekannt und umfassen u.a., sind aber nicht ausschließlich beschränkt auf das Zerkleinern oder das Mahlen von erfindungsgemäßen genetisch modifizierten Pflanzen oder erfindungsgemäßen Pflanzenteilen. Einige Vorteile, die sich aus der Verwendung erfindungsgemäßer Gegenstände für die Herstellung von Nahrungs-/Futtermitteln oder für den Einsatz in technischen Gebieten ergeben, sind bereits oben beschrieben worden.

Besonders bevorzugt handelt es sich bei einem erfindungsgemäßen Verfahren zur Herstellung einer Zusammensetzung um ein Verfahren zur Herstellung einer Zusammensetzung, die Hyaluronan enthält.

Zusammensetzungen erhältlich nach einem Verfahren zur Herstellung einer erfindungsgemäßen Zusammensetzung sind ebenfalls Gegenstand der vorliegenden Erfindung.

Die vorliegende Erfindung betrifft auch die Verwendung von erfindungsgemäßen genetisch modifizierten Pflanzenzellen, erfindungsgemäßen genetisch modifizierten Pflanzen, erfindungsgemäßem Vermehrungsmaterial, erfindungsgemäßen erntebaren Pflanzeteilen, erfindungsgemäßen verarbeitbaren Pflanzenteilen, erfindungsgemäßen konsumierbaren Pflanzenteilen oder Pflanzen, erhältlich nach einem erfindungsgemäßen Verfahren zur Herstellung einer Pflanze, die Hyaluronan synthetisiert, zur Herstellung einer erfindungsgemäßen Zusammensetzung. Bevorzugt handelt es sich um die Verwendung von erfindungsgemäßen genetisch modifizierten Pflanzenzellen, erfindungsgemäßen genetisch modifizierten Pflanzen, erfindungsgemäßem Vermehrungsmaterial, erfindungsgemäßen erntebaren Pflanzeteilen, erfindungsgemäßen verarbeitbaren Pflanzenteilen, erfindungsgemäßen konsumierbaren Pflanzenteilen oder von Pflanzen, erhältlich nach einem erfindungsgemäßen Verfahren zur Herstellung einer Pflanze, die Hyaluronan synthetisiert, zur Herstellung von Lebens- oder Futtermitteln, zur Herstellung eines Pharmazeutikums oder zur Herstellung eines kosmetischen Produktes.

**Beschreibung der Sequenzen**

SEQ ID NO 1: Nucleinsäuresequenz, codierend eine Hyaluronansynthase des *Paramecium bursaria Chlorella* Virus 1.

SEQ ID NO 2: Aminosäuresequenz einer Hyaluronansynthase des *Paramecium bursaria Chlorella* Virus 1. Die dargestellte Aminosäuresequenz kann von SEQ ID NO 1 abgeleitet werden.

SEQ ID NO 3: Synthetische Nucleinsäuresequenz, codierend eine Hyaluronansynthase des *Paramecium bursaria Chlorella* Virus 1. Die Synthese der Codons der dargestellten Sequenz erfolgte in der Weise, dass sie an die Verwendung von Codons in Pflanzenzellen angepasst ist. Die dargestellte Nucleinsäuresequenz codiert ein Protein mit der unter SEQ ID No 2 dargestellten Aminosäuresequenz.

SEQ ID NO 4: Nucleinsäuresequenz, codierend ein Protein mit der Aktivität einer UDP-Glc-DH des *Paramecium bursaria Chlorella* Virus 1.

SEQ ID NO 5: Aminosäuresequenz eines Proteins mit der Aktivität einer UDP-Glc-DH des *Paramecium bursaria Chlorella* Virus 1. Die dargestellte Aminosäuresequenz kann von SEQ ID NO 4 abgeleitet werden.

SEQ ID NO 6: Synthetische Nucleinsäuresequenz, codierend ein Protein mit der Aktivität einer UDP-Glc-DH des

*Paramecium bursaria Chlorella* Virus 1. Die Synthese der Codons der dargestellten Sequenz erfolgte in der Weise, dass sie an die Verwendung von Codons in Pflanzenzellen angepasst ist. Die dargestellte Nucleinsäuresequenz codiert ein Protein mit der unter SEQ ID No 5 dargestellten Aminosäuresequenz.

SEQ ID NO 7: Nucleinsäuresequenz, codierend ein Protein mit der Aktivität einer GFAT-1 aus Maus.

SEQ ID NO 8: Aminosäuresequenz eines Proteins mit der Aktivität GFAT-1 aus Maus. Die dargestellte Aminosäuresequenz kann von SEQ ID NO 7 abgeleitet werden.

SEQ ID NO 9: Nucleinsäuresequenz, codierend ein Protein mit der Aktivität einer GFAT-2 aus Maus.

SEQ ID NO 10: Aminosäuresequenz eines Proteins mit der Aktivität GFAT-2 aus Maus. Die dargestellte Aminosäuresequenz kann von SEQ ID NO 9 abgeleitet werden.

SEQ ID NO 11: Nucleinsäuresequenz, codierend ein Protein mit der Aktivität einer GFAT aus *Escherichia coli.*

SEQ ID NO 12: Aminosäuresequenz eines Proteins mit der Aktivität einer GFAT aus *Escherichia coli.* Die dargestellte Aminosäuresequenz kann von SEQ ID NO 11 abgeleitet werden.

SEQ ID NO 13: Synthetische Nucleinsäuresequenz, codierend ein Protein mit der Aktivität einer GFAT aus *Escherichia coli.* Die Synthese der Codons der dargestellten Sequenz erfolgte in der Weise, dass sie an die Verwendung von Codons in Pflanzenzellen angepasst ist. Die dargestellte Nucleinsäuresequenz codiert ein Protein mit der unter SEQ ID No 11 dargestellten Aminosäuresequenz.

**Beschreibung der Abbildungen**

Fig.1: Stellt eine Eichgerade und die dazugehörige Gleichung der Regressionsgeraden dar, welche zur Berechnung des Hyaluronangehaltes in pflanzlichem Gewebe verwendet wurde. Die Eichgerade wurde mit Hilfe des käuflich erwerbbaren Testkits (Hyaluronic Acid (HA) Test Kit der Firma Corgenix, Inc., Colorado, USA, Prod. Nr. 029-001) und den darin mitgelieferten Standardlösungen erstellt.

**Allgemeine Methoden**

Im Folgenden werden Methoden beschrieben, die im Zusammenhang mit der vorliegenden Erfindung verwendet werden können. Diese Methoden stellen konkrete Ausführungsformen dar, beschränken die vorliegende Erfindung jedoch nicht auf diese Methoden. Dem Fachmann ist bekannt, dass er durch Modifikation der beschriebenen Methoden und/oder durch Ersetzen einzelner Methoden bzw. Methodenteile durch alternative Methoden bzw. alternative Methodenteile die Erfindung in gleicher Weise ausführen kann.

1. Transformation von Kartoffelpflanzen

Kartoffelpflanzen wurden mit Hilfe von *Agrobacterium,* wie bei Rocha-Sosa et al. (EMBO J. 8, (1989), 23-29) beschrieben, transferiert.

2. Isolierung von Hyaluronan aus pflanzlichem Gewebe

Pflanzenmaterial wurde zum Nachweis des Vorliegens von Hyaluronan und zur Bestimmung des Hyaluronangehaltes in pflanzlichem Gewebe folgender maßen aufgearbeitet: Zu ca. 0,3 g Knollenmaterial wurden 200 μl Wasser (demineralisiert, Leitfähigkeit ≥18 MΩ) zugegeben und mit einer Labor-Schwingkugelmühle (MM200, Firma Retsch, Germany) zerkleinert (30 sec. bei 30 HZ). Anschließend erfolgte eine weitere Zugabe von 800 μl Wasser (demineralisiert, Leitfähigkeit ≥18 MΩ) und eine gute Durchmischung (z.B. mit Hilfe eines Vortex). Die Zelltrümmer und unlösliche Bestandteile wurden vom Überstand durch 5 minütiges Zentrifugieren bei 16000xg abgetrennt.

3. Aufreinigung von Hyaluronan

Ca. 100 Gramm Knollen wurden geschält, in etwa 1 $cm^3$ kleine Stücke geschnitten und nach Zugabe von 100 ml Wasser (demineralisiert, Leitfähigkeit ≥18 MΩ) in einem Warring Blendor für ca. 30 Sekunden bei maximaler Geschwindigkeit zerkleinert. Anschließend wurden die Zelltrümmer über ein Teesieb abgetrennt. Die abgetrennten Zelltrümmer wurden nochmals mit 300 ml Wasser (demineralisiert, Leitfähigkeit ≥18 MΩ) aufgeschlemmt und erneut über ein Teesieb abgetrennt. Die beiden erhaltenen Suspensionen (100 ml + 300 ml) wurden vereinigt und für 15 Minuten bei 13000xg zentrifugiert. Dem erhaltenen Zentrifugationsüberstand wurde NaCl bis zu einer Endkonzentration von 1% zugegeben. Nachdem das NaCl gelöst war, erfolgte eine Fällung, durch Zugabe des doppelten Volumens Ethanol, anschließendem gutem Durchmischen und Inkubation über Nacht bei -20°C. Anschließend erfolgte eine Zentrifugation für 15 Minuten bei 13000xg. Der nach dieser Zentrifugation erhaltene, sedimentierte Niederschlag wurde in 100 ml Puffer (50mM TrisHCl, pH 8, 1 mM $CaCl_2$) gelöst, bevor Proteinase K bis zu einer Endkonzentration von 100 μg/ml zugegeben und die Lösung für 2 Stunden bei 42°C inkubiert wurde. Es folgte eine Inkubation für 10 Minuten bei 95°C. Es wurde zu dieser Lösung erneut NaCl bis zu einer Endkonzentration von 1 % zugegeben. Nachdem das NaCl gelöst war, erfolgte eine erneute Fällung, durch Zugabe des doppelten Volumens Ethanol, gutem Durchmischen und Inkubation für ca. 96 Stunden bei -20°C. Anschließend erfolgte eine Zentrifugation für 15 Minuten bei 13000xg. Der nach dieser Zentrifugation erhaltene, sedimentierte Niederschlag wurde in 30 ml Wasser (demineralisiert, Leitfähigkeit ≥18 MΩ) gelöst und erneut mit NaCl bis zu einer Endkonzentration von 1 % versetzt. Durch Zugabe des doppelten Volumens Ethanol, gutem Durchmischen und Inkubation über Nacht bei -20°C erfolgte eine erneute Fällung. Der nach anschließender 15 minütiger Zentrifugation bei 13000xg erhaltene Niederschlag wurde in 20 ml Wasser (demineralisiert, Leitfähigkeit ≥18 MΩ) gelöst.
Eine weitere Aufreinigung erfolgte mittels Zentrifugalfiltration. Dazu wurden jeweils 5 ml des gelösten Niederschlages über einen Membranfilter (CentriconAmicon, Porenweite 10000 NMWL, Prod. Nr. UCF8 010 96) gegeben und bei 2200xg solange zentrifugiert, bis noch ca. 3 ml der Lösung oberhalb des Filters übrig waren. Anschließend wurden noch zweimal jeweils 3 ml Wasser (demineralisiert, Leitfähigkeit ≥18 MΩ) zu der über der Membran befindlichen Lösung hinzu gegeben und jeweils erneut unter gleichen Bedingungen zentrifugiert, bis am Ende noch ca. 3 ml der Lösung oberhalb des Filters übrig waren. Die nach Zentrifugalfiltration noch über der Membran vorliegenden Lösungen werden abgenommen und die Membran noch mehrmals (drei- bis fünfmal) mit ca. 1,5 ml Wasser (demineralisiert, Leitfähigkeit ≥18 MΩ) gespült. Alle noch über der Membran vorliegenden Lösungen und die Spüllösungen werden vereinigt, mit NaCl bis zu einer Endkonzentration von 1 % versetzt, nach Lösen des NaCl mit dem doppelten Volumen an Ethanol versehen, gemischt und durch Lagerung bei -20°C über Nacht gefällt. Der nach anschließender 15 minütiger Zentrifugation bei 13000xg erhaltene Niederschlag wurde in 4 ml Wasser (demineralisiert, Leitfähigkeit ≥18 MΩ) gelöst und anschließend gefriergetrocknet (24 Stunden bei einem Druck von 0,37 mbar, Gefriertrocknungsanlage Christ Alpha 1-4, Firma Christ, Osterode).

4. Nachweis von Hyaluronan und Bestimmung des Hyaluronangehaltes

Der Nachweis von Hyaluronan erfolgte mit Hilfe eines käuflich erwerbbaren Tests (Hyaluronic Acid (HA) Test Kit der Firma Corgenix, Inc., Colorado, USA, Prod. Nr. 029-001) nach den Angaben des Herstellers, die durch Bezugnahme hiermit als Gegenstand in die Beschreibung aufgenommen sind. Das Testprinzip beruht auf der Verfügbarkeit eines spezifisch an Hyaluronan bindenden Proteins (HABP) und erfolgt ähnlich einem ELISA, wobei eine Farbreaktion den Hyaluronangehalt in der untersuchten Probe anzeigt. Die zu vermessenden Proben sollten daher für die quantitative Bestimmung von Hyaluronan in solch einer Konzentration eingesetzt werden (z.B: verdünnen der jeweiligen Probe oder Verwendung von weniger Wasser für die Extraktion von Hyaluronan aus dem pflanzlichen Gewebe, je nachdem ob ein Grenzwert über- oder unterschritten wurde), dass sie innerhalb der angegebenen Grenzen liegen.
In parallel-Ansätzen wurden Aliquots der zu bestimmenden Proben zunächst einem Hyaluronidaseverdau unterzogen, bevor sie mit dem käuflich erwerbbaren Test (Hyaluronic Acid (HA) Test Kit der Firma Corgenix, Inc., Colorado, USA, Prod. Nr. 029-001) vermessen wurden. Der Hyaluronidaseverdau erfolgte mit 400 μl Kartoffelknollen Extrakt in Hyaluronidase Puffer (0,1 M Kalium Phosphatpuffer, pH 5,3; 150 mm NaCl) durch Zugabe von 5 μg (~3 units) Hyaluronidase (Hyaluronidase Typ III from Sigma, Prd. Nr. H 2251) bei einer Inkubation von 30 min bei 37 °C.
Anschließend wurden alle Proben jeweils in einer Verdünnung von 1:10 für die Bestimmung des Hyaluronangehaltes eingesetzt.

5. Berechnung von Standardabweichungen

Angegebene Standardabweichungen wurden nach folgender Formel berechnet:

$$\text{Quadrat Wurzel } [n\sum x^2-(\sum x)^2/n(n-1)]$$

Wobei x den Wert von einzelnen Messwerten darstellt und n die Summe aller Messwerte darstellet, die zur Ermittlung der betreffenden Standardabweichung herangezogen werden.

6. Bestimmung der Aktivität einer GFAT

Die Bestimmung der Aktivität eines Proteins mit der Aktivität einer GFAT wird wie bei Rachel et al. (1996, J. Bacteriol. 178 (8), 2320-2327) beschrieben durchgeführt. Zur Unterscheidung ob ein Protein die Aktivität einer GFAT-1 oder GFAT-2 aufweist, wird die bei Hu et al. (2004, J. Biol. Chem. 279 (29), 29988-29993) beschriebene Methode verwendet.

7. Bestimmung der Aktivität einer UDP-Glc-DH

Die Bestimmung der Aktivität eines Proteins mit der Aktivität einer UDP-Glc-DH wird wie bei Spicerl et al. (1998, J. Bacteriol. 273 (39), 25117-25124) beschrieben durchgeführt.

## Beispiele

1. Herstellung des pflanzlichen Expressionsvektors IR 47-71

Das Plasmid pBinAR ist ein Derivat des binären Vektorplasmids pBin19 (Bevan, 1984, Nucl Acids Res 12: 8711-8721), das folgendermaßen konstruiert wurde: Ein 529 bp langes Fragment, das die Nukleotide 6909-7437 des 35S-Promotor des Blumenkohl-Mosaik-Virus umfasst, wurde als *EcoR* I/*Kpn* I Fragment aus dem Plasmid pDH51 (Pietrzak et al, 1986 Nucleic Acids Res. 14, 5858) isoliert und zwischen die EcoR I und *Kpn* I Restriktionsschnittstellen des Polylinkers von pUC18 ligiert. Dabei entstand das Plasmid pUC18-35S. Aus dem Plasmid pAGV40 (Herrera-Estrella et al, 1983 Nature, 303, 209-213) wurde mit Hilfe der Restriktionsendonucleasen *Hind* III und *Pvu* II ein 192 bp langes Fragment isoliert, das das Polyadenylierungssignal (3'-Ende) des *Octopin Synthase*-Gens (Gen 3) der T-DNA des Ti-Plasmids pTiACH5 (Gielen et al, 1984, EMBO Journal 3, .835-846) umfasst (Nucleotide 11749-11939). Nach Addition von *Sph* I Linkern an die *Pvu* II-Schnittstelle wurde das Fragment zwischen die *Sph* I- und *Hind* III-Schnittstellen von pUC18-35S ligiert. Daraus resultierte das Plasmid pA7. Hier wurde der gesamte Polylinker enthaltend den 35S-Promotor und Ocs-Terminator mit *E*coR I und *Hind* III herausgeschnitten und in den entsprechend geschnittenen Vektor pBin19 ligiert. Dabei entstand der pflanzliche Expressionsvektor pBinAR (Höfgen und Willmitzer, 1990, Plant Science 66, 221-230).

Der Promotor des Patatin Gens B33 aus *Solanum tuberosum* (Rocha-Sosa et al., 1989, EMBO J. 8, 23-29) wurde als *Dra* I Fragment (Nukleotide -1512 - +14) in den mit *Sst* I geschnittenen Vektor pUC19, dessen Enden mit Hilfe der T4-DNA Polymerase geglättet worden waren, ligiert. Daraus entstand das Plasmid pUC19-B33. Aus diesem Plasmid wurde der B33-Promotor mit *EcoR* I und *Sma* I herausgeschnitten und in den entsprechend geschnittenen Vektor pBinAR ligiert. Hieraus entstand der pflanzliche Expressionsvektor pBinB33.

Um weitere Klonierungsschritte zu erleichtern, wurde die MCS (Multiple Cloning Site) erweitert. Hierfür wurden zwei komplementäre Oligonucleotide synthetisiert, für 5 Minuten auf 95°C erhitzt, auf Raumtemperatur langsam abgekühlt, um ein gutes fixieren (annealen), zu ermöglichen und in die *Sal* I- und Kpn I Schnittstellen von pBinB33 kloniert. Die dazu verwendeten Oligonucleotide wiesen folgende Sequenz auf: 5'-TCg ACA ggC CTg gAT CCT TAA TTA AAC TAg TCT CgA ggA gCT Cgg TAC-3' 5'-CgA gCT CCT CgA gAC TAg TTT AAT TAA ggA TCC Agg CCT g-3' Das erhaltene Plasmid wurde mit IR 47-71 bezeichnet.

2. Herstellung des pflanzlichen Expressionsvektors pBinARHyg

Das Fragment, enthaltend den 35S-Promotor, den Ocs-Terminator und die gesamte Multiple Cloning Site wurde mittels der Restriktionsendonukleasen *E*coR I und *Hind* III aus pA7 herausgeschnitten und in den mit den gleichen Restiktionsendonukleasen geschnittenen Vektor pBIBHyg Becker, 1990, Nucleic Acids Res. 18, 203) kloniert. Das erhaltene Plasmid wurde mit pBinARHyg bezeichnet.

3. Herstellung des Klonierungsvektors IC 317-204

Nuckleinsäurefragmente, umfassend den OCS Terminator wurden mittels der Restriktionsendonucleasen *Xho* I und *Hind* III aus dem Plasmid IR 47-71 isoliert und in den mit den gleichen Restriktionsendonukleasen geschnittenen Vektor pBlueScript KS (Firma Stratagene, Prod. Nr. 212207) kloniert. Das erhaltene Plasmid wurde mit IC 306-204 bezeichnet.
Nuckleinsäurefragmente, umfassend den B33 Promoter wurden mittels der Restriktionsendonucleasen *Bam* HI und *Eco* RI aus dem Plasmid IR 47-71 isoliert und in den mit den gleichen Restriktionsendonukleasen geschnittenen Vektor pBlueScript KS (Firma Stratagene, Prod. Nr. 212207) kloniert. Das erhaltene Plasmid wurde mit IC 314-204 bezeichnet. Aus IC 306-204 wurde der OCS-Terminator mittels der Restriktionsendonuklease *Bam* HI isoliert und in das mit der gleichen Restriktionsendonuklease geschnittene Plasmid IC 314-204 kloniert. Das erhaltene Plasmid wurde mit IC 317-204 bezeichnet.

4. Synthese von Nucleinsäuremolekülen

a) Synthese von Nucleinsäuremolekülen codierend eine Hyaluronansynthase des *Paramecium bursaria Chlorella* Virus 1

Die Nucleinsäuresequenz, codierend eine Hyaluronansynthase aus *Paramecium bursaria Chlorella* Virus 1, wurde von der Firma Medigenomix GmbH (München, Deutschland) synthetisiert und in den Vektor pCR2.1 der Firma Invitrogen (Prod. Nr. K2000-01) kloniert. Das erhaltene Plasmid wurde mit IC 323-215 bezeichnet. Die synthetische Nucleinsäuresequenz codierend das HAS Protein aus *Paramecium bursaria Chlorella* Virus 1, ist unter SEQ ID NO 3 dargestellt. Die korrespondierende, ursprünglich aus dem *Paramecium bursaria Chlorella* Virus 1 isolierte Nucleinsäuresequenz, ist unter SEQ ID NO 1 dargestellt.

b) Synthese von Nucleinsäuremolekülen codierend ein Protein mit der Aktivität einer UDP-Glc-DH des *Paramecium bursaria Chlorella* Virus 1

Die Nucleinsäuresequenz, codierend ein Protein mit der Aktivität einer UDP-Glc-DH aus *Paramecium bursaria Chlorella* Virus 1, wurde von der Firma Entelechon GmbH synthetisiert und in den Vektor pCR4Topo der Firma Invitrogen (Prod. Nr. K4510-20) kloniert. Das erhaltene Plasmid wurde mit IC 339-222 bezeichnet. Die synthetische Nucleinsäuresequenz codierend das UDP-Glc-DH Protein aus *Paramecium bursaria Chlorella* Virus 1, ist unter SEQ ID NO 6 dargestellt. Die korrespondierende, ursprünglich aus *Paramecium bursaria Chlorella* Virus 1 isolierte Nucleinsäuresequenz, ist unter SEQ ID NO 4 dargestellt.

c) Synthese von Nucleinsäuremolekülen codierend ein Protein mit der Aktivität einer GFAT aus *Escherichia coli*

Die Nucleinsäuresequenz, codierend ein Protein mit der Aktivität einer GFAT aus *Escherichia coli* wurde von der Firma Entelechon GmbH synthetisiert und in den Vektor pCR4Topo der Firma Invitrogen (Prod. Nr. K4510-20) kloniert. Das erhaltene Plasmid wurde mit IC 373-256 bezeichnet. Die synthetische Nudeinsäuresequenz codierend ein Protein mit der Aktivität einer GFAT aus *Escherichia coli,* ist unter SEQ ID NO 13 dargestellt. Die korrespondierende, ursprünglich aus *Escherichia coli* isolierte Nucleinsäuresequenz, ist unter SEQ ID NO 11 dargestellt.

5. Herkunft weiterer Nucleinsäuremoleküle

a) Nucleinsäuremoleküle codierend ein Protein mit der Aktivität einer GFAT-1 aus Maus

Die Nucleinsäuresequenz, codierend ein Protein mit der Aktivität einer GFAT-1 wurde von der Firma BioCat GmbH, Heidelberg (Art.Nr. MMM1013-65346, cDNA clone MGC:58262, IMAGE:6742987) erworben. Hierbei handelt es sich um einen Klon der vom I.M.A.G.E. Konsortium (http://image.llnl.gov) hergestellt und von BioCat GmbH vertrieben wird. Hierbei wurde die cDNA, codierend ein Protein mit der Aktivität einer GFAT-1 in den Vektor pCMV Sport 6 der Firma Invitrogen kloniert. Das erhaltene Plasmid wurde mit IC 365-256 bezeichnet. Die in IC 365-256 inserierte Nucleinsäuresequenz codierend ein Protein mit der Aktivität einer GFAT-1 aus *Mus musculus* weist zu der ist unter SEQ ID NO 7 dargestellten Nucleinsäuresequenz an Position 1090 einen Basenaustausch von T zu C und an der Position 2027 einen Basenaustausch von G zu A auf. Diese Basenaustausche führen nicht zu Aminosäureaustauschen der von den beiden unterschiedlichen Nucleinsäuremolekülen codierten Aminosäuresequenzen.
Die codierende Nucleinsäuresequenz für das Protein mit der Aktivität einer GFAT-1 aus Maus ist in SEQ ID NO 8 dargestellt.
Um nachfolgende Klonierungsschritte zu erleichtern, wurde die für ein Protein mit der Aktivität einer GFAT-1 codierende Sequenz mittels Verwendung der Restriktionsendonucleasen *Xho* I und Eco RV aus IC 365-256 isoliert und in das mit den gleichen Restriktionsendonucleasen geschnittene Plasmid pME9 (pBlueSkript Vektor der Firma Stratagene, Prod. Nr. 212207) mit veränderter Multiple Cloning Site, die zusätzlich an beiden Enden eine *Pac* I Schnittstelle aufweist) kloniert. Das erhaltene Plasmid wurde mit IC 367-256 bezeichnet.

b) Nucleinsäuremoleküle codierend ein Protein mit der Aktivität einer GFAT-2 aus Maus

Nucleinsäuremoleküle, codierend ein Protein mit der Aktivität einer GFAT-2 aos, wurde von der Firma Invitrogen (Clone ID 4167189, cDNA clone MGC:18324, IMAGE:4167189) erworben. Hierbei handelt es sich um einen Klon der vom I.M.A.G.E. Konsortium (http://image.llnl.gov) hergestellt und von Invitrogen vertrieben wird. Hierbei wurde die cDNA, codierend ein Protein mit der Aktivität einer GFAT-2 in den Vektor pCMV Sport 6 der Firma Invitrogen kloniert. Das Plasmid wurde mit IC 369-256 bezeichnet. Die Nucleinsäuresequenz codierend das Protein mit der Aktivität einer GFAT-2 aus *Mus musculus,* ist unter SEQ ID NO 9 dargestellt.

6. Herstellung des pflanzlichen Expressionsvektors IC 341-222, umfassend eine codierende Nucleinsäuresequenz für eine Hyaluronansynthase des *Paramecium bursaria* Chlorella Virus 1

Aus dem Plasmid IC 323-215 wurden mittels Restriktionsverdau mit *BamH* I und *Xho* I Nucleinsäuremoleküle, umfassend die codierende Sequenz der Hyaluronansynthase, isoliert und in die *BamH* I und *Xho* I Schnittstellen des Plasmides IR 47-71 kloniert. Der erhaltene pflanzliche Expressionsvektor wurde mit IC 341-222 bezeichnet.

7. Herstellung der pflanzlichen Expressionsvektoren IC 370-256 und IC 376-256, umfassend codierende Nucleinsäuresequenzen für ein Protein mit der Aktivität einer GFAT-1 aus Maus und für ein Protein mit der Aktivität einer UDP-Glc-DH des *Paramecium bursaria Chlorella* Virus 1

Aus dem Plasmid IC 339-222 wurden mittels Restriktionsverdau mit *BamH* 1 und *Kpn* 1 Nucleinsäuremoleküle, umfassend die codierende Sequenz für ein Protein mit der Aktivität einer UDP-Gic-DH des *Paramecium bursaria Chlorella* Virus 1 isoliert und in das mittels gleicher Restriktionsendonukleasen geschnittene Plasmide pA7 kloniert. Das erhaltene Plasmid wurde mit IC 342-222 bezeichnet.
Aus dem Plasmid IC 342-222 wurden durch Restriktionsverdau mit *Xba* I und *Kpn* I Nucleinsäuremoleküle, umfassend die codierende Sequenz für ein Protein mit der Aktivität einer UDP-Glc-DH des *Paramecium bursaria Chlorella* Virus 1 isoliert und in den mit *Xba* I und *Kpn* I geschnittenen Expressionsvektor pBinAR Hyg kloniert. Das erhaltene Plasmid wurde mit IC 349-222 bezeichnet.
Im nächsten Schritt wurde in die *Eco* RI Schnittstelle von IC 349-222 ein Nucleinsäurefragment, umfassend den B33-Promotor und den OCS-Terminator, das aus IC 317-204 durch Restriktionsverdau mittels *Eco* RI isoliert wurde, kloniert. Hierbei wurde auf die Entgegengesetzte Orientierung der Promotoren (35S und B33) geachtet (head-to-head). Der erhaltene Vektor wurde mit IC 354-222 bezeichnet.
In einem weiteren Klonierungsschritt wurde ein Nucleinsäurefragment, umfassend die kodierende Sequenz des Proteins mit der Aktivität einer GFAT-1 aus Maus mittels Restriktionsverdau mit *Xho* I und Eco RV aus IC 367-256 isoliert und in das mit *Xho* I und *Ecl*136 II geschnittene Plasmid IC 354-222 kloniert. Der erhaltene pflanzliche Expressionsvektor wurde mit IC 370-256 bezeichnet.
Nach einer Sequenzanalyse des Plasmides IC 370-256, wurde festgestellt, dass die kodierende Nucleinsäuresequenz des Proteins mit der Aktivität einer GFAT-1 aus Maus an zwei Positionen Veränderungen aufwies, im Vergleich zur Nucleinsäuresequenz, die in Plasmid IC 365-256 inseriert ist. Die Nucleinsäuresequenz codierend für das Protein mit der Aktivität einer GFAT-1 aus Maus, welche in dem Plasmid IC 370-256 enthalten ist, weist gegenüber der unter SEQ ID NO 7 dargestellten Nucleinsäuresequenz an Position 1160 einen Basenbaustausch von G zu A, an Position 1190 einen Basenbaustausch von T zu C, an Position 1245 einen Basenbaustausch von T zu C und an Position 2027 von G zu A auf. Diese veränderte Nucleinsäuresequenz codiert ein Protein, das bezüglich der unter SEQ ID NO 8 dargestellten Aminosäuresequenz eine Änderung der Aminosäuren in Position 304 von R zu Q und in Position 366 von C zu R aufweist.
Um einen pflanzlichen Expressionsvektor zu erhalten, der die korrekte Nucleinsäuresequenz, codierend ein Protein mit der Aktivität einer GFAT-1 aus Maus umfasst, wurde die kodierende Sequenz des Proteins mit der Aktivität einer GFAT-1 aus Maus erneut mittels Restriktionsverdau mit *Xho* I und *Eco* RV aus IC 365-256 isoliert und in das mit *Xho* I und *Ecl*136 II geschnittene Plasmid IC 354-222 kloniert. Der erhaltene pflanzliche Expressionsvektor wurde mit IC 376-256 bezeichnet.
Die Nucleinsäuresequenz codierend für das Protein mit der Aktivität einer GFAT-1 aus Maus, welche in dem Plasmid IC 376-256 enthalten ist, stimmt mit der codierenden Sequenz für ein Protein mit der Aktivität einer GFAT-1 aus Maus, die in Plasmid 365-256 inseriert ist, überein. Die von diesem Nucleinsäuremolekül codierte Aminosäuresequenz ist unter SEQ ID NO 8 dargestellt.

8. Herstellung des pflanzlichen Expressionsvektors IC 372-256, umfassend codierende Nucleinsäuresequenzen für ein Protein mit der Aktivität einer GFAT-2 aus Maus und für ein Protein mit der Aktivität einer UDP-Glc-DH des *Paramecium bursaria Chlorella* Virus 1

Ein Nucleinsäurefragment, umfassend die kodierende Sequenz des Proteins mit der Aktivität einer GFAT-2 aus Maus wurde mittels Restriktionsverdau mit *Xho* I und Eco RV aus IC 369-256 isoliert und in das mit *Xho* I und *Ecl* 136 II geschnittene Plasmid IC 354-222 kloniert. Der erhaltene pflanzliche Expressionsvektor wurde mit IC 372-256 bezeichnet.

9. Herstellung des pflanzlichen Expressionsvektors IC 374-256, umfassend codierende Nucleinsäuresequenzen für ein Protein mit der Aktivität einer GFAT aus *Escherichia coli* und für ein Protein mit der Aktivität einer UDP-Glc-DH des *Paramecium bursaria Chlorella* Virus 1

Ein Nucleinsäurefragment, umfassend die kodierende Sequenz des Proteins mit der Aktivität einer GFAT aus *Escherichia coli* wurde mittels Restriktionsverdau mit *Xho* I und *Eco* RV aus IC 373-256 isoliert und in das mit *Xho* I und *Ecl* 136 II geschnittene Plasmid IC 354-222 kloniert. Der erhaltene pflanzliche Expressionsvektor wurde mit IC 374-256 bezeichnet.

10. Transformation von Pflanzen, mit pflanzlichen Expressionsvektoren, die Nucleinsäuremoleküle enthalten, die eine Hyaluronansynthase codieren Kartoffelpflanzen wurden mit dem pflanzlichen Expressionsvektor IC 341-222, enthaltend eine codierende Nucleinsäuresequenz für eine Hyaluronansynthase aus *Paramecium bursaria Chlorella* Virus 1 unter der Kontrolle des Promotors des Patatin Gens B33 aus *Solanum tuberosum* (Rocha-Sosa et al., 1989, EMBO J. 8, 23-29) nach der unter Allgemeine Methoden Punkt 1 angegebnen Methode transformiert. Die erhaltenen transgenen Kartoffelpflanzen, die mit dem Plasmid IC 341-222 transformiert waren, wurden mit 365 ES bezeichnet.

11. Analyse der transgenen Pflanzen, die mit pflanzlichen Expressionsvektoren, umfassend Nucleinsäuremoleküle, die eine Hyaluronansynthase codieren, transformiert wurden

a) Erstellung einer Eichreihe

Eine Eichreihe wurde unter Verwendung der im käuflichen Testkit (Hyaluronic Acid (HA) Test Kit der Firma Corgenix, Inc., Colorado, USA, Prod. Nr. 029-001) beiliegenden Standardlösungen nach den vom Hersteller angegeben Verfahren erstellt. Für die Bestimmung der Extinktion von 1600 ng/ml Hyaluronan wurde die zweifache Menge, bezogen auf die vom Hersteller vorgegebene Menge des mitgelieferten Standards, enthaltend 800 ng/ ml Hyaluronan eingesetzt. Es wurden je drei unabhängige Messreihen durchgeführt und der betreffende Mittelwert bestimmt. Es wurde folgende Eichreihe erhalten:

| Hyaluronan Konzentration | Unabhängige Einzelmessungen | | | Mittelwert | Stabw |
|---|---|---|---|---|---|
| | $E_{450nm}$ | $E_{450nm}$ | $E_{450nm}$ | | |
| 0 ng/ml | 0,100 | 0,096 | 0,096 | 0,097 | 0,002 |
| 50 ng/ml | 0,224 | 0,183 | 0,222 | 0,210 | 0,023 |
| 100 ng/ml | 0,396 | 0,263 | 0,377 | 0,345 | 0,072 |
| 200 ng/ml | 0,554 | 0,443 | 0,653 | 0,550 | 0,105 |
| 500 ng/ml | 1,231 | 0,850 | 1,221 | 1,101 | 0,217 |
| 800 ng/ml | 1,465 | 1,265 | 1,795 | 1,508 | 0,268 |
| 1600 ng/ml | 2,089 | 2,487 | 3,170 | 2,582 | 0,547 |

**Tabelle 1:** Messwerte zur Ermittlung einer Eichkurve für die quantitative Bestimmung des Hyaluronangehaltes in pflanzlichem Gewebe. Mit Hilfe von Software (Microsoft Office Excel 2002, SP2) wurden die erhaltenen Messwerte in ein Diagramm eingetragen und die Funktionsgleichung der Trendlinie bestimmt (siehe Fig 1) $E_{450nm}$ bezeichnet die Extinktion bei einer Wellenlänge von 450 nm, Stabw bezeichnet die Standardabweichung des errechneten Mittelwertes von den Einzelwerten.

b) Analyse von Kartoffelknollen, der Linien 365 ES

Einzelne Pflanzen der Linie 365 ES wurden im Gewächshaus in 6 cm Töpfen in Erde kultiviert. Ca. jeweils 0,3 g Material von Kartoffelknollen der einzelnen Pflanzen wurden nach der unter Allgemeine Methoden Punkt 2 beschriebenen Methode aufgearbeitet. Die Menge des darin in den jeweiligen Pflanzenextrakten enthaltenen Hyaluronans wurde mit der unter Allgemeine Methoden Punkt 4. beschriebenen Methode und unter zur Hilfenahme der in Beispiel 10a) und Fig 1 dargestellten Eichgerade bestimmt. Der nach Zentrifugation erhaltene Überstand wurde dabei in einer Verdünnung von 1:10 für die Bestimmung des Hyaluronangehaltes eingesetzt, Für ausgewählte Pflanzen wurden folgende Ergebnisse erhalten:

| Bezeichnung der Pflanze | Gewicht des eingesetzten Pflanzenmaterials [g] | Extinktion E450 | Menge an Hyaluronan [ng/ml] | Hyaluronan bezogen auf Frischgewicht des Pflanzenmaterials [μg/g] |
|---|---|---|---|---|
| 365 ES 13 | 0,297 | 2,746 | 14038 | 47 |
| 365 ES 74 | 0,306 | 4,000 | 20816 | 68 |
| Wildtyp | 0,305 | 0,111 | - | - |

**Tabelle 2:** Menge an Hyaluronan (in μg Hyaluronan pro g Frischgewicht), das von unabhängigen, ausgewählten transgenen Pflanzen der Linie 365 ES produziert wurde. Spalte 1 bezeichnet die Pflanze, von welcher Knollenmaterial geerntet wurde ("Wildtyp" bezeichnet hierbei Pflanzen, die nicht transformiert sind, jedoch den Genotyp aufweisen, der als Ausgangsmaterial für die Transformation verwendet wurde). Spalte 2 gibt die Menge des Knollenmaterials an, welches von der betreffenden Pflanze für die Bestimmung des Hyaluronangehaltes eingesetzt wurde. Spalte 3 enthält die gemessene Extinktion einer 1:10 Verdünnung des jeweiligen Pflanzenextraktes. Spalte 4 wurde mit Hilfe der Regressionsgeradengleichung (siehe Fig 1) unter Berücksichtigung des Verdünnungsfaktors wie folgt berechnet: ((Wert Spalte 3 - 0,149)/0,00185) x 10. Spalte 5 gibt die Menge Hyaluronan bezogen auf das eingesetzte Frischgewicht an und wurde wie folgt berechnet: (Wert Spalte 4 /Wert Spalte 2) / 1000.

12. Transformation von Pflanzen, die Hyaluronan synthetisieren, mit pflanzlichen Expressionsvektoren, umfassend codierende Nucleinsäuresequenzen für ein Protein mit der Aktivität einer GFAT und für ein Protein mit der Aktivität einer UDP-Glc-DH

Kartoffelpflanzen der Linien 365 ES 13 und 365 ES 74 wurden jeweils mit den pflanzlichen Expressionsvektoren IC 370-256, IC 376-256, IC 372-256 bzw. IC 374-256 nach der unter Allgemeine Methoden Punkt 1 angegebnen Methode transformiert.
Die erhaltenen transgenen Kartoffelpflanzen der Linie 365 ES 13, die mit dem Plasmid IC 370-256 transformiert waren, wurden mit 393 ES bezeichnet.
Die erhaltenen transgenen Kartoffelpflanzen der Linie 365 ES 74, die mit dem Plasmid IC 370-256 transformiert waren, wurden mit 394 ES bezeichnet.
Die erhaltenen transgenen Kartoffelpflanzen der Linie 365 ES 13, die mit dem Plasmid IC 372-256 transformiert waren, wurden mit 395 ES bezeichnet.
Die erhaltenen transgenen Kartoffelpflanzen der Linie 365 ES 74, die mit dem Plasmid IC 372-256 transformiert waren, wurden mit 396 ES bezeichnet.
Die erhaltenen transgenen Kartoffelpflanzen der Linie 365 ES 13, die mit dem Plasmid IC 374-256 transformiert waren, wurden mit 403 ES bezeichnet.
Die erhaltenen transgenen Kartoffelpflanzen der Linie 365 ES 74, die mit dem Plasmid IC 374-256 transformiert waren, wurden mit 404 ES bezeichnet.
Die erhaltenen transgenen Kartoffelpflanzen der Linie 365 ES 13, die mit dem Plasmid IC 376-256 transformiert waren, wurden mit 408 ES bezeichnet.
Die erhaltenen transgenen Kartoffelpflanzen der Linie 365 ES 74, die mit dem Plasmid IC 376-256 transformiert waren, wurden mit 409 ES bezeichnet.

13. Analyse transgener Hyaluronan synthetisierender Pflanzen, die zusätzlich mit pflanzlichen Expressionsvektoren tranformiert wurden, die codierende Nucleinsäuresequenzen für ein Protein mit der Aktivität einer GFAT und für ein Protein mit der Aktivität einer UDP-Glc-DH umfassen

Einzelne Pflanzen der Linie 393 ES, 394 ES, 395 ES, 396 ES, 403 ES und 404 ES wurden im Gewächshaus in 6 cm Töpfen in Erde kultiviert. Ca. jeweils 0,3 g Material von Kartoffelknollen oder Blättern der einzelnen Pflanzen wurden nach der unter Allgemeine Methoden Punkt 2 beschriebenen Methode aufgearbeitet. Die Menge des darin in den jeweiligen Pflanzenextrakten enthaltenen Hyaluronans wurde mit der unter Allgemeine Methoden Punkt 4. beschriebenen Methode und unter zur Hilfenahme einer gemäß Beispiel 10a) erstellten Eichgerade, die für jede unabhängig durchgeführte Messreihe neu erstellt wurde, bestimmt. Der nach Zentrifugation erhaltene Überstand wurde dabei für die Bestimmung des Hyaluronangehaltes jeweils mit Wasser (demineralisiert, Leitfähigkeit ≥18 MΩ) so verdünnt, dass die gemessenen Extinktionswerte der einzelnen Proben im linearen Bereich der Eichgerade lagen. Für aus einzelnen Transformationen mit unterschiedlichen Plasmiden hervorgegange Pflanzen sind die Ergebisse im Folgenden dargstellt.

a) Analyse von Knollen der Linie 393 ES

Für jede Knolle einzelner transgener Pflanzen der Linien mit der Bezeichnung 393 ES wurden zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES 13 wurden errechnet, indem die Menge an Hyaluronan in Knollen von jeweils zehn verschiedenen Pflanzen, die vegetative Abkömmlinge des Wildtyps *(Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [μg/g] | Standardabweichung |
|---|---|---|
| 393 ES 6 | 126,42 | 12,7 |
| 393 ES 18 | 113,10 | 26,1 |
| 393 ES 23 | 112,83 | 18,7 |
| 393 ES 38 | 102,81 | 19,2 |
| 393 ES 36 | 99,71 | 16,7 |
| 393 ES 52 | 90,78 | 3,5 |
| 393 ES 50 | 90,31 | 8,7 |
| 393 ES 49 | 88,63 | 14,4 |
| 393 ES 32 | 87,82 | 15,2 |
| 393 ES 16 | 86,09 | 17,8 |
| 393 ES 33 | 80,47 | 16,3 |
| Wildtyp | 0,52 | 0,8 |
| 365 ES 13 | 72,62 | 16,4 |

**Tabelle 3:** Menge an Hyaluronan (in μg Hyaluronan pro g Frischgewicht), die in Knollen von unabhängigen, ausgewählten transgenen Pflanzen der Linie 393 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 13 bezeichnet Pflanzen, die eine Hyaluronansynthase exprimieren und als Ausgangangsmaterial für die Transformation mit dem pflanzlichen Expressionsvektor IC 370-256 zur Erzeugung der Linien 393 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an.

b) Analyse von Knollen der Linie 394 ES

Für jede Knolle einzelner transgener Pflanzen der Linien mit der Bezeichnung 394 ES wurden zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Knollen von 18 verschiedenen Wildtyp Pflanzen und 26 verschiedenen Pflanzen der Linie 365 ES 74, die vegetative Abkömmlinge des Wildtyps *(Solanum tuberosum* cv. Désirée) bzw. der Linie 365 ES 74 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [μg/g] | Standardabweichung |
|---|---|---|
| 394 ES 47 | 242,24 | 12,7 |
| 394 ES 37 | 227,66 | 15,2 |
| 394 ES 45 | 185,90 | 12,8 |
| 394 ES 56 | 176,82 | 25,1 |
| 394 ES 43 | 172,83 | 15,3 |
| 394 ES 14 | 168,80 | 27,1 |
| 394 ES 52 | 157,81 | 16,1 |
| 394 ES 28 | 145,20 | 8,5 |
| 394 ES 5 | 131,11 | 17,9 |
| 394 ES 26 | 127,56 | 13,5 |
| 394 ES 1 | 126,22 | 15,4 |
| 394 ES 15 | 125,46 | 9,9 |
| Wildtyp | 1,29 | 0,8 |
| 365 ES 74 | 104,34 | 26,1 |

**Tabelle 4** Menge an Hyaluronan (in μg Hyaluronan pro g Frischgewicht), die in Knollen von unabhängigen, ausgewählten transgenen Pflanzen der Linie 394 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 74 bezeichnet Pflanzen, die eine Hyaluronansynthase exprimieren und als Ausgangsmaterial für die Transformation mit dem pflanzlichen Expressionsvektor IC 370-256 zur Erzeugung der Linien 394 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an.

c) Analyse von Knollen der Linie 395 ES

Für jede Knolle einzelner transgener Pflanzen der Linien mit der Bezeichnung 395 ES wurden, wenn möglich, zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Knollen von 10 verschiedenen Wildtyp Pflanzen und 18 verschiedenen Pflanzen der Linie 365 ES 13, die vegetative Abkömmlinge des Wildtyps *(Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [µg/g] | Standardabweichung |
|---|---|---|
| 395 ES 17 | 1321,75 | |
| 395 ES 29 | 1145,32 | |
| 395 ES 60 | 999,66 | 484,50 |
| 395 ES 16 | 791,64 | |
| 395 ES 53 | 770,93 | 57,35 |
| 395 ES 10 | 651,30 | |
| 395 ES 26 | 299,37 | 58,78 |
| 395 ES 3 | 288,21 | |
| 395 ES 13 | 228,22 | |
| 395 ES 38 | 96,10 | 12,30 |
| Wildtyp | 0,31 | 0,22 |
| 365 ES 13 | 77,16 | 16,56 |

**Tabelle 5** Menge an Hyaluronan (in µg Hyaluronan pro g Frischgewicht), die in Knollen von unabhängigen, ausgewählten transgenen Pflanzen der Linie 395 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 13 bezeichnet Pflanzen, die eine Hyaluronansynthase exprimieren und als Ausgangsmaterial für die Transformation mit dem pflanzlichen Expressionsvektor IC 372-256 zur Erzeugung der Linien 395 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. Bei Pflanzen, für die keine Standardabweichung angegeben ist, wurde der Hyaluronangehalt nur für eine Knollenprobe bestimmt.

d) Analyse von Blättern der Linie 395 ES

Für einzelne Blätter von Pflanzen der Linien mit der Bezeichnung 395 ES wurden eine Bestimmung des Hyaluronangehaltes durchgeführt. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Blättern von 4 verschiedenen Wildtyp Pflanzen und 9 verschiedenen Pflanzen der Linie 365 ES 13, die vegetative Abkömmlinge des Wildtyps *(Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [µg/g] | Standardabweichung |
|---|---|---|
| 395 ES 34 | 619,17 | |
| 395 ES 45 | 589,52 | |
| 395 ES 51 | 420,81 | |
| 395 ES 46 | 405,81 | |
| 395 ES 24 | 401,68 | |
| 395 ES 12 | 392,90 | |
| 395 ES 43 | 381,78 | |
| 395 ES 21 | 368,04 | |
| 395 ES 33 | 352,25 | |
| 395 ES 25 | 350,90 | |

(fortgesetzt)

| Bezeichnung der Pflanze | Mittelwert der Menge an Hyaluronan bezogen auf Frischgewicht des Pflanzenmaterials [μg/g] | Standardabweichung |
|---|---|---|
| 395 ES 22 | 344,44 | |
| 395 ES 48 | 338,52 | |
| 395 ES 4 | 300,86 | |
| 395 ES 28 | 298,30 | |
| 395 ES 36 | 291,51 | |
| 395 ES 2 | 274,05 | |
| 395 ES 14 | 219,96 | |
| 395 ES 56 | 158,39 | |
| 395 ES 57 | 94,17 | |
| Wildtyp | 0,18 | 0,14 |
| 365 ES 13 | 44,76 | 18,71 |

**Tabelle 6** Menge an Hyaluronan (in μg Hyaluronan pro g Frischgewicht), die in Blättern von unabhängigen, ausgewählten transgenen Pflanzen der Linie 395 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 13 bezeichnet Pflanzen, die eine Hyaluronansynthase exprimieren und als Ausgangsmaterial für die Transformation mit dem pflanzlichen Expressionsvektor IC 372-256 zur Erzeugung der Linien 395 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. Bei Pflanzen, für die keine Standardabweichung angegeben ist, wurde der Hyaluronangehalt nur für eine Blattprobe bestimmt.

e) Analyse von Knollen der Linie 396 ES

Für jede Knolle einzelner transgener Pflanzen der Linien mit der Bezeichnung 396. Es wurden zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Knollen von 12 verschiedenen Wildtyp Pflanzen und 14 verschiedenen Pflanzen der Linie 365 ES 74, die vegetative Abkömmlinge des Wildtyps *(Solanum tuberosum* cv. Désirée) bzw. der Linie 365 ES 74 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [μg/g] | Standardabweichung |
|---|---|---|
| 396 ES 42 | 1283,02 | 229,8 |
| 396 ES44 | 1146,29 | 235,1 |
| 396 ES 33 | 804,90 | 500,1 |
| 396 ES 9 | 670,56 | 91,7 |
| 396 ES 2 | 389,61 | 67,2 |
| 396 ES 15 | 380,60 | 18,4 |
| 396 ES 28 | 371,66 | 159,9 |
| 396 ES 30 | 204,20 | 13,5 |
| 396 ES 8 | 186,69 | 55,5 |

(fortgesetzt)

| Bezeichnung der Pflanze | Mittelwert der Menge an Hyaluronan bezogen auf Frischgewicht des Pflanzenmaterials [µg/g] | Standardabweichung |
|---|---|---|
| 396 ES 4 | 161,61 | 25,8 |
| Wildtyp | 0,95 | 0,6 |
| 365 ES 74 | 142,70 | 57,5 |

**Tabelle 7** Menge an Hyaluronan (in µg Hyaluronan pro g Frischgewicht), die in Knollen von unabhängigen, ausgewählten transgenen Pflanzen der Linie 396 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 74 bezeichnet Pflanzen, die eine Hyaluronansynthase exprimieren und als Ausgangsmaterial für die Transformation mit dem pflanzlichen Expressionsvektor IC 372-256 zur Erzeugung der Linien 396 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an.

f) Analyse von Blättern der Linie 396 ES

Für einzelne Blätter von Pflanzen der Linien mit der Bezeichnung 396 ES wurde eine Bestimmung des Hyaluronangehaltes durchgeführt. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Blättern von 4 verschiedenen Wildtyp Pflanzen und 6 verschiedenen Pflanzen der Linie 365 ES 13, die vegetative Abkömmlinge des Wildtyps *(Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [µg/g] | Standardabweichung |
|---|---|---|
| 396 ES 51 | 1160,57 | |
| 396 ES 32 | 941,89 | |
| 396 ES 11 | 938,33 | |
| 396 ES 36 | 860,54 | |
| 396 ES 57 | 807,97 | |
| 396 ES 25 | 801,58 | |
| 396 ES 34 | 796,79 | |
| 396 ES 50 | 619,23 | |
| 396 ES 49 | 538,75 | |
| 396 ES 48 | 461,05 | |
| 396 ES 24 | 443,57 | |
| 396 ES 17 | 426,79 | |
| 396 ES 16 | 416,43 | |
| 396 ES 23 | 271,85 | |
| 396 ES 43 | 258,47 | |
| 396 ES 14 | 186,78 | |
| Wildtyp | 0,15 | 0,08 |
| 365 ES 74 | 106,35 | 56,77 |

**Tabelle 8** Menge an Hyaluronan (in µg Hyaluronan pro g Frischgewicht), die in Blättern von unabhängigen, ausgewählten transgenen Pflanzen der Linie 396 ES produziert wurde. Spalte 1 bezeichnet die Pflanze, von welcher Blattmaterial geerntet wurde ("Wildtyp" bezeichnet hierbei Pflanzen, die nicht transformiert sind; 365 ES 74 bezeichnet Pflanzen, die eine Hyaluronansynthase exprimieren und als Ausgangsmaterial für die Transformation mit dem pflanzlichen Expressionsvektor IC 372-256 zur Erzeugung der Linien 396 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. Bei Pflanzen, für die keine Standardabweichung angegeben ist, wurde der Hyaluronangehalt nur für eine Blattprobe bestimmt.

g) Analyse von Knollen der Linie 403 ES

Für jede Knolle einzelner transgener Pflanzen der Linien mit der Bezeichnung 403 ES wurden, wenn möglich, zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Knollen von 10 verschiedenen Wildtyp Pflanzen und 10 verschiedenen Pflanzen der Linie 365 ES 13, die vegetative Abkömmlinge des Wildtyps (*Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [μg/g] | Standardabweichung |
|---|---|---|
| 403 ES 2 | 687,90 | |
| 403 ES 5 | 457,56 | |
| 403 ES 4 | 366,34 | |
| 403 ES 15 | 295,00 | |
| 403 ES 30 | 241,03 | |
| 403 ES 8 | 140,51 | |
| 403 ES 41 | 107,65 | |
| Wildtyp | n.d. | - |
| 365 ES 13 | 89,42 | 24,87 |

**Tabelle 9** Menge an Hyaluronan (in μg Hyaluronan pro g Frischgewicht), die in Knollen von unabhängigen, ausgewählten transgenen Pflanzen der Linie 403 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 13 bezeichnet Pflanzen, die eine Hyaluronansynthase exprimieren und als Ausgangsmaterial für die Transformation mit dem pflanzlichen Expressionsvektor IC 374-256 zur Erzeugung der Linien 403 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. "n.d." besagt, dass kein Hyaluronan in den Knollen nachgewiesen werden konnte. Bei Pflanzen, für die keine Standardabweichung angegeben ist, wurde der Hyaluronangehalt nur für eine Knollenprobe bestimmt.

h) Analyse von Blättern der Linie 403 ES

Für einzelne Blätter von Pflanzen der Linien mit der Bezeichnung 403 ES wurde eine Bestimmung des Hyaluronangehaltes durchgeführt. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Blättern von 5 verschiedenen Wildtyp Pflanzen und 5 verschiedenen Pflanzen der Linie 365 ES 13, die vegetative Abkömmlinge des Wildtyps (*Solanum tuberosum* cv. Désirée) bzw. der Linie 365 ES 13 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [µg/g] | Standardabweichung |
|---|---|---|
| 403 ES 10 | 1186,32 | |
| 403 ES 50 | 1141,75 | |
| 403 ES 9 | 1017,62 | |
| 403 ES 42 | 959,01 | |
| 403 ES 40 | 930,39 | |
| 403 ES 33 | 904,65 | |
| 403 ES 6 | 884,45 | |
| 403 ES 47 | 841,92 | |
| 403 ES 37 | 725,39 | |
| 403 ES 2 | 653,23 | |
| 403 ES 48 | 579,14 | |
| 403 ES 27 | 510,98 | |
| Wildtyp | 3,93 | 2,87 |
| 365 ES 13 | 85,46 | 17,3 |

**Tabelle 10** Menge an Hyaluronan (in µg Hyaluronan pro g Frischgewicht), die in Blättern von unabhängigen, ausgewählten transgenen Pflanzen der Linie 403 ES produziert wurde. Spalte 1 bezeichnet die Pflanze, von welcher Blattmaterial geerntet wurde ("Wildtyp" bezeichnet hierbei Pflanzen, die nicht transformiert sind; 365 ES 13 bezeichnet Pflanzen, die eine Hyaluronansynthase exprimieren und als Ausgangsmaterial für die Transformation mit dem pflanzlichen Expressionsvektor IC 374-256 zur Erzeugung der Linien 403 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. Bei Pflanzen, für die keine Standardabweichung angegeben ist, wurde der Hyaluronangehalt nur für eine Blattprobe bestimmt.

i) Analyse von Knollen der Linie 404 ES

Für jede Knolle einzelner transgener Pflanzen der Linien mit der Bezeichnung 404 ES wurden, wenn möglich, zwei voneinander unabhängige Proben entnommen und jeweils eine getrennte Bestimmung des Hyaluronangehaltes durchgeführt. Anschließend wurde der Mittelwert und die Standardabweichung aus den für die einzelnen Messungen jeder Knolle erhaltenen Werte gemäß der unter Allgemeine Methoden Punkt 5 angegebenen Formel berechnet. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Knollen von 10 verschiedenen Wildtyp Pflanzen und 12 verschiedenen Pflanzen der Linie 365 ES 74, die vegetative Abkömmlinge des Wildtyps *(Solanum tuberosum* cv. Désirée) bzw. der Linie 365 ES 74 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzenmaterials [µg/g] | Standardabweichung |
|---|---|---|
| 404 ES 16 | 633,53 | |
| 404 ES 47 | 188,42 | 7,80 |
| 404 ES 45 | 174,21 | 1,03 |
| 404 ES 17 | 155,09 | |
| 404 ES 2 | 138,33 | 4,91 |
| 404 ES 30 | 124,38 | |

(fortgesetzt)

| Bezeichnung der Pflanze | Mittelwert der Menge an Hyaluronan bezogen auf Frischgewicht des Pflanzenmaterials [µg/g] | Standardabweichung |
|---|---|---|
| 404 ES 18 | 116,10 | 14,98 |
| Wildtyp | n.d. | - |
| 365 ES 74 | 110,23 | 15,94 |

**Tabelle 11** Menge an Hyaluronan (in µg Hyaluronan pro g Frischgewicht), die in Knollen von unabhängigen, ausgewählten transgenen Pflanzen der Linie 404 ES produziert wurde. Spalte 1 bezeichnet die Pflanze, von welcher Knollenmaterial geerntet wurde ("Wildtyp" bezeichnet hierbei Pflanzen, die nicht transformiert sind; 365 ES 74 bezeichnet Pflanzen, die eine Hyaluronansynthase exprimieren und als Ausgangsmaterial für die Transformation mit dem pflanzlichen Expressionsvektor IC 374-256 zur Erzeugung der Linien 404 ES verwendet wurden). Spalte 2 gibt den Mittelwert der Menge an Hyaluronan, der für die betreffenden Knollen ermittelt wurde, an. Spalte 3 gibt die Standardabweichung für die ermittelten Mittelwerte an. "n.d." besagt, dass kein Hyaluronan in den Knollen nachgewiesen werden konnte. Bei Pflanzen, für die keine Standardabweichung angegeben ist, wurde der Hyaluronangehalt nur für eine Knollenprobe bestimmt.

j) Analyse von Blättern der Linie 404 ES

Für einzelne Blätter von Pflanzen der Linien mit der Bezeichnung 404 ES wurde eine Bestimmung des Hyaluronangehaltes durchgeführt. Die angegebenen Mittelwerte bzw. Standardabweichungen für Pflanzen mit der Bezeichnung Wildtyp und Pflanzen mit der Bezeichnung 365 ES wurden errechnet, indem jeweils die Menge an Hyaluronan in Blättern von 7 verschiedenen Wildtyp Pflanzen und 9 verschiedenen Pflanzen der Linie 365 ES 74, die vegetative Abkömmlinge des Wildtyps *(Solanum tuberosum* cv. Désirée) bzw. der Linie 365 ES 74 darstellten, berechnet wurden. Für ausgewählte Pflanzen wurden folgende Ergebnisse erhalten:

| Bezeichnung der Pflanze | Mittelwert der Menge an Hyaluronan bezogen auf Frischgewicht des Pflanzen materials [µg/g] | Standardabweichung |
|---|---|---|
| 404 ES 13 | 1547,12 | |
| 404 ES 35 | 1388,51 | |
| 404 ES 29 | 1146,68 | |
| 404 ES 36 | 1095,11 | |
| 404 ES 44 | 921,11 | |
| 404 ES 42 | 849,43 | |
| 404 ES 46 | 846,81 | |
| 404 ES 15 | 832,32 | |
| 404 ES 23 | 817,91 | |
| 404 ES 1 | 801,14 | |
| 404 ES 38 | 651,12 | |
| 404 ES 14 | 616,79 | |
| 404 ES 16 | 615,92 | |
| 404 ES 20 | 581,11 | |
| 404 ES 37 | 533,89 | |
| 404 ES 8 | 521,92 | |
| 404 ES 21 | 489,73 | |
| 404 ES 43 | 479,34 | |

(fortgesetzt)

| Bezeichnung der Pflanze | Mittelwert der Menge an Hyaluronan bezogen auf Frischgewicht des Pflanzen materials [µg/g] | Standardabweichung |
|---|---|---|
| 404 ES 24 | 434,06 | |
| 404 ES 40 | 371,88 | |
| 404 ES 9 | 366,46 | |
| 404 ES 6 | 365,15 | |
| 404 ES 28 | 359,96 | |
| 404 ES 39 | 353,74 | |
| 404 ES 34 | 310,76 | |
| 404 ES 48 | 302,54 | |
| 404 ES 11 | 231,39 | |
| 404 ES 10 | 226,83 | |
| 404 ES 7 | 218,42 | |
| 404 ES 26 | 205,00 | |
| Wildtyp | 0,25 | 0,09 |
| 365 ES 74 | 83,24 | 44,73 |

**Tabelle 12** Menge an Hyaluronan (in µg Hyaluronan pro g Frischgewicht), die in Blättern von unabhängigen, ausgewählten transgenen Pflanzen der Linie 404 ES produziert wurde. Spalte 1 bezeichnet die Pflanze, von welcher Blattmaterial geerntet wurde ("Wildtyp" bezeichnet hierbei Pflanzen, die nicht transformiert sind; 365 ES 74 bezeichnet Pflanzen, die eine Hyaluronansynthase exprimieren und als Ausgangsmaterial für die Transformation mit dem pflanzlichen Expressionsvektor IC 374-256 zur Erzeugung der Linien 404 ES verwendet wurden). Spalte 2 gibt den Mittelwert der Menge an Hyaluronan, der für die betreffenden Blätter ermittelt wurde, an. Spalte 3 gibt die Standardabweichung für die ermittelten Mittelwerte an. Bei Pflanzen, für die keine Standardabweichung angegeben ist, wurde der Hyaluronangehalt nur für eine Blattprobe bestimmt.

k) Bestimmung des Hyaluronangehaltes bezüglich des Frischgewichtes und bezüglich des Trockengewichtes

Einzelne Blätter von Pflanzen der Linien 395 ES bzw. 396 ES wurden vor der Ernte der Knollen betreffender Pflanzen von den Pflanzen entfernt und in der Mitte zerteilt. Eine Hälfte jedes einzelnen Blattes wurde jeweils in flüssigem Stickstoff eingefroren, die dazugehörige andere Hälfte wurde über Nacht gefriergetrocknet.
Ca. 0,3 g Blattmaterial der gefrorenen bzw. ca. 0,02 g der gefriergetrockneten Blattproben wurden mit einer Labor-Schwingkugelmühle (MM200, Firma Retsch, Germany) zerkleinert (30 sec. bei 30 HZ) zerkleinert. Anschließend erfolgte eine Zugabe von 300 µl Wasser (demineralisiert, Leitfähigkeit ≥18 MΩ) zu jeder einzelnen zerkleinerten Probe, die dann intensiv mit Hilfe eines Vortex durchmischt wurden, bevor die Zelltrümmer und unlösliche Bestandteile mittels Zentrifugation (5 Minuten bei 16000xg) vom Überstand abgetrennt wurden. Der Überstand wurde abgenommen und jede Probe wurde mit Wasser (demineralisiert, Leitfähigkeit ≥18 MΩ) auf 500 µl aufgefüllt. Aliquots der so hergestellten Proben wurden für die Bestimmung des Hyaluronangehaltes nach der unter Allgemeine Methoden Punkt 4 beschriebenen Methode eingesetzt. Der Mittelwerte und die Standardabweichungen wurden nach der unter Allgemeine Methoden Punkt 5 angegebenen Formel berechnet. Für ausgewählte Pflanzen wurden folgende Ergebnisse erhalten:

| Bezeichnung der Pflanze | Menge an Hyaluronan bezogen auf Frischgewicht des Pflanzen-materials [µg/g] | Mittelwert der Menge an Hyaluronan bezogen auf Frischgewicht des Pflanzen-materials [µg/g] | Standardabweichung |
|---|---|---|---|
| 395ES 16 I | 570,87 | 491,04 | 146,80 |
| 395ES 16 II | 321,62 | | |
| 395ES 16 III | 580,64 | | |
| 395ES 17 I | 414,39 | 532,55 | 120,37 |
| 395ES 17 II | 655,02 | | |
| 395ES 17 III | 528,23 | | |
| 396ES 9 I | 316,64 | 241,21 | 88,31 |
| 396ES 9 II | 144,08 | | |
| 396ES 9 III | 262,92 | | |
| 396ES 16 I | 462,80 | 622,99 | 139,45 |
| 396ES 16 II | 688,92 | | |
| 396ES 16 III | 717,24 | | |
| 365ES 13 I | 43,23 | 52,77 | 16,04 |
| 365ES 13 II | 71,28 | | |
| 365ES 13 III | 43,80 | | |
| 365ES 74 I | 169,75 | 158,00 | 12,52 |
| 365ES 74 II | 144,83 | | |
| 365ES 74 III | 159,42 | | |

**Tabelle 13** Menge an Hyaluronan (in µg Hyaluronan pro g Frischgewicht), die in Blättern von unabhängigen, ausgewählten transgenen Pflanzen der Linien 395 ES bzw. 396 ES produziert wurde. Spalte 1 bezeichnet die Pflanze, von welcher Blattmaterial geerntet wurde. 365 ES 13 bezeichnet Pflanzen, die eine Hyaluronansynthase exprimieren und als Ausgangsmaterial für die Transformation mit dem pflanzlichen Expressionsvektor IC 372-256 zur Erzeugung der Linien 395 ES verwendet wurden. 365 ES 74 bezeichnet Pflanzen, die eine Hyaluronansynthase exprimieren und als Ausgangsmaterial für die Transformation mit dem pflanzlichen Expressionsvektor IC 372-256 zur Erzeugung der Linien 396 ES verwendet wurden. Spalte 2 gibt die Menge an Hyaluronan, die für unterschiedliche Blätter der betreffenden Pflanzen ermittelt wurde, an. Spalte 3 gibt den Mittelwert für die Mengen an Hyaluronan, gemessen in unterschiedlichen Blättern einer Pflanze an. Spalte 4 gibt die Standardabweichung für die ermittelten Mittelwerte an.

| Bezeichnung der Pflanze | Menge an Hyaluronan bezogen auf Trockengewicht des Pflanzen-materials [µg/g] | Mittelwert der Menge an Hyaluronan bezogen auf Trockengewicht des Pflanzenmaterials [µg/g] | Standardabweichung |
|---|---|---|---|
| 395ES 16 I | 5212,63 | 4633,54 | 636,00 |
| 395ES 16 II | 3952,86 | | |
| 395ES 16 III | 4735,12 | | |
| 395ES 17 I | 4402,04 | 4313,57 | 77,25 |
| 395ES 17 II | 4259,45 | | |
| 395ES 17 III | 4279,23 | | |

(fortgesetzt)

| Bezeichnung der Pflanze | Menge an Hyaluronan bezogen auf Trockengewicht des Pflanzen-materials [µg/g] | Mittelwert der Menge an Hyaluronan bezogen auf Trockengewicht des Pflanzenmaterials [µg/g] | Standardabweichung |
|---|---|---|---|
| 396ES 9 I | 3918,45 | 2543,02 | 1383,15 |
| 396ES 9 II | 1152,27 | | |
| 396ES 9 III | 2558,35 | | |
| 396ES 16 I | 4428,93 | 5077,92 | 932,12 |
| 396ES 16 II | 6146,03 | | |
| 396ES 16 III | 4658,81 | | |
| 365ES 13 I | 373,90 | 398,74 | 104,57 |
| 365ES 13 II | 513,49 | | |
| 365ES 13 III | 308,82 | | |
| 365ES 74 I | 1403,83 | 1207,71 | 170,52 |
| 365ES 74 II | 1094,43 | | |
| 365ES 74 III | 1124,87 | | |

**Tabelle 14** Menge an Hyaluronan (in µg Hyaluronan pro g Trockengewicht), die in Blättern von unabhängigen, ausgewählten transgenen Pflanzen der Linien 395 ES bzw. 396 ES produziert wurde. Spalte 1 bezeichnet die Pflanze, von welcher Blattmaterial geerntet wurde. 365 ES 13 bezeichnet Pflanzen, die eine Hyaluronansynthase exprimieren und als Ausgangsmaterial für die Transformation mit dem pflanzlichen Expressionsvektor IC 372-256 zur Erzeugung der Linien 395 ES verwendet wurden. 365 ES 74 bezeichnet Pflanzen, die eine Hyaluronansynthase exprimieren und als Ausgangsmaterial für die Transformation mit dem pflanzlichen Expressionsvektor IC 372-256 zur Erzeugung der Linien 396 ES verwendet wurden. Spalte 2 gibt die Menge an Hyaluronan, die für unterschiedliche Blätter der betreffenden Pflanzen ermittelt wurde, an. Spalte 3 gibt den Mittelwert für die Mengen an Hyaluronan, gemessen in unterschiedlichen Blättern einer Pflanze an. Spalte 4 gibt die Standardabweichung für die ermittelten Mittelwerte an.

I) Abschließende Anmerkungen

Bei der Bestimmung des Hyaluronangehaltes unterschiedlicher Blätter von einer Pflanze wurde festgestellt, dass ältere Blätter derselben Pflanze in der Regel mehr Hyaluronan enthielten, als jüngere Blätter der gleichen Pflanze. Der Gehalt an Hyaluronan in Blättern scheint daher mit zunehmendem Alter des Blattes anzusteigen, so dass davon ausgegangen werden kann, dass Hyaluronan über die Zeit akkumuliert. Unterschiedliche Hyaluronanmengen, die in unabhängigen Messungen für Abkömmlinge der gleichen Linie ermittelt wurden, können durch dieses Phänomen erklärt werden.

**Patentansprüche**

1. Genetisch modifizierte Pflanzenzelle, die ein in ihr Genom stabil integriertes Nucleinsäuremolekül, codierend eine Hyaluronansynthase aufweist, **dadurch gekennzeichnet, dass** besagte Pflanzenzelle zusätzlich eine erhöhte Aktivität eines Proteins mit der Aktivität einer Glutamin:Fructose-6-Phosphat Amidotransferase (GFAT) und eine erhöhte Aktivität eines Proteins mit der Aktivität einer UDP-Glucose Dehydrogenase (UDP-Glc-DH) aufweist, im Vergleich zu entsprechenden nicht genetisch modifizierten Wildtyp Pflanzenzellen.

2. Genetisch modifizierte Pflanzenzelle nach Anspruch 1 **dadurch gekennzeichnet, dass** sie ein in ihr Genom stabil integriertes fremdes Nucleinsäuremolekül oder mehrere in ihr Genom stabil integrierte fremde Nucleinsäuremoleküle aufweist, wobei besagtes fremdes Nucleinsäuremolekül oder besagte fremde Nucleinsäuremoleküle zur Erhöhung der Aktivität eines Proteins mit der Aktivität einer Glutamin:Fructose-6-Phosphat Amidotransferase (GFAT) und zur Erhöhung der Aktivität eines Proteins mit der Aktivität einer UDP-Glucose Dehydrogenase (UDP-Glc-DH) führt/führen, im Vergleich zu entsprechenden nicht genetisch modifizierten Wildtyp Pflanzenzellen.

3. Genetisch modifizierte Pflanzenzelle nach Anspruch 2 wobei ein erstes fremdes Nucleinsäuremolekül ein Protein mit der enzymatischen Aktivität einer Glutamin:Fructose-6-Phosphat Amidotransferase (GFAT) codiert und ein zweites fremdes Nucleinsäuremolekül ein Protein mit der enzymatischen Aktivität einer UDP-Glucose Dehydrogenase (UDP-Gluc DH) codiert.

4. Genetisch modifizierte Pflanzenzelle nach einem der Ansprüche 1, 2 oder 3 die eine erhöhte Menge an Hyaluronan synthetisiert im Vergleich zu Pflanzenzellen, die die Aktivität einer Hyaluronansynthase aufweisen und keine erhöhte Aktivität einer Glutamin:Fructose-6-Phosphat Amidotransferase (GFAT) und keine erhöhte Aktivität einer UDP-Glucose Dehydrogenase aufweisen.

5. Pflanze enthaltend genetisch modifizierte Pflanzenzellen nach einem der Ansprüche 1 bis 4.

6. Vermehrungsmaterial von Pflanzen nach Anspruch 5, enthaltend genetisch modifizierte Pflanzenzellen nach einem der Ansprüche 1 bis 4.

7. Erntebare Pflanzenteile von Pflanzen nach Anspruch 5, enthaltend genetisch modifizierte Pflanzenzellen nach einem der Ansprüche 1 bis 4.

8. Verfahren zur Herstellung einer Pflanze, die Hyaluronan synthetisiert, worin

   a) eine Pflanzenzelle, genetisch modifiziert wird, wobei die genetische Modifikation die folgenden Schritte i bis iii umfasst

      i) Einführung eines fremden Nucleinsäuremoleküls, codierend eine Hyaluronansynthase in die Pflanzenzelle
      ii) Einführung einer genetischen Modifikation in die Pflanzenzelle, wobei die genetische Modifikation zur Erhöhung der Aktivität eines Proteins mit der enzymatischen Aktivität einer Glutamin:Fructose-6-Phosphat Amidotransferase (GFAT) im Vergleich zu entsprechenden nicht genetisch modifizierten Wildtyp-Pflanzenzellen führt
      iii) Einführung einer genetischen Modifikation in die Pflanzenzelle, wobei die genetische Modifikation zur Erhöhung der Aktivität eines Proteins mit der enzymatischen Aktivität einer Glutamin:Fructose-6-Phosphat UDP-Glucose Dehydrogenase im Vergleich zu entsprechenden nicht genetisch modifizierten Wildtyp-Pflanzenzellen führt

   wobei die Schritte i bis iii in beliebiger Reihenfolge, einzeln oder beliebige Kombinationen der Schritte i bis iii gleichzeitig durchgeführt werden
   b) aus Pflanzenzellen von Schritt a) eine Pflanze regeneriert wird;
   c) gegebenenfalls weitere Pflanzen mit Hilfe der Pflanzen nach Schritt b) erzeugt werden.
   wobei gegebenenfalls aus Pflanzen gemäß den Schritten b) oder c) Pflanzenzellen isoliert werden und die Verfahrensschritte a) bis c) solange wiederholt werden, bis eine Pflanze erzeugt wurde, die ein fremdes Nucleinsäuremolekül, codierend eine Hyaluronansynthase aufweist und eine erhöhte Aktivität eines Proteins mit der enzymatischen Aktivität einer Glutamin:Fructose-6-Phosphat Amidotransferase (GFAT) im Vergleich zu entsprechenden nicht genetisch modifizierten Wildtyp-Pflanzenzellen aufweist und eine erhöhte Aktivität eines Proteins mit der enzymatischen Aktivität einer Glutamin:Fructose-6-Phosphat UDP-Glucose Dehydrogenase im Vergleich zu entsprechenden nicht genetisch modifizierten Wildtyp-Pflanzenzellen aufweist.

**9.** Verfahren zur Herstellung von Hyaluronan, umfassend den Schritt der Extraktion von Hyaluronan aus genetisch modifizierte Pflanzenzellen nach einem der Ansprüche 1 bis 4, aus Pflanzen nach Anspruch 5 aus Vermehrungsmaterial nach Anspruch 6 aus erntebaren Pflanzenteilen nach Anspruch 7 oder aus Pflanzen erhältlich nach einem Verfahren nach Anspruch 8.

**10.** Verwendung einer genetisch modifizierte Pflanzenzelle nach einem der Ansprüche 1 bis 4, einer Pflanze nach Anspruch 5, Vermehrungsmaterial nach Anspruch 6, erntebaren Pflanzenteilen nach Anspruch 7 oder von Pflanzen, erhältlich nach einem Verfahren nach Anspruch 8 zur Herstellung von Hyaluronan.

**11.** Zusammensetzung enthaltend genetisch modifizierte Pflanzenzellen nach einem der Ansprüche 1 bis 4.

**12.** Verfahren zur Herstellung einer Zusammensetzung enthaltend Hyaluronan, worin genetisch modifizierte Pflanzenzellen nach einem der Ansprüche 1 bis 4, Pflanzen nach Anspruch 5, Vermehrungsmaterial nach Anspruch 6, erntebare Pflanzenteile nach Anspruch 7 oder Pflanzen erhältlich nach einem Verfahren nach Anspruch 8 verwendet werden.

**13.** Verwendung von genetisch modifizierte Pflanzenzellen nach einem der Ansprüche 1 bis 4, von Pflanzen nach Anspruch 5, von Vermehrungsmaterial nach Anspruch 6, von erntebaren Pflanzenteilen nach Anspruch 7 oder von Pflanzen, erhältlich nach einem Verfahren nach Anspruch 8, zur Herstellung einer Zusammensetzung nach Anspruch 11.

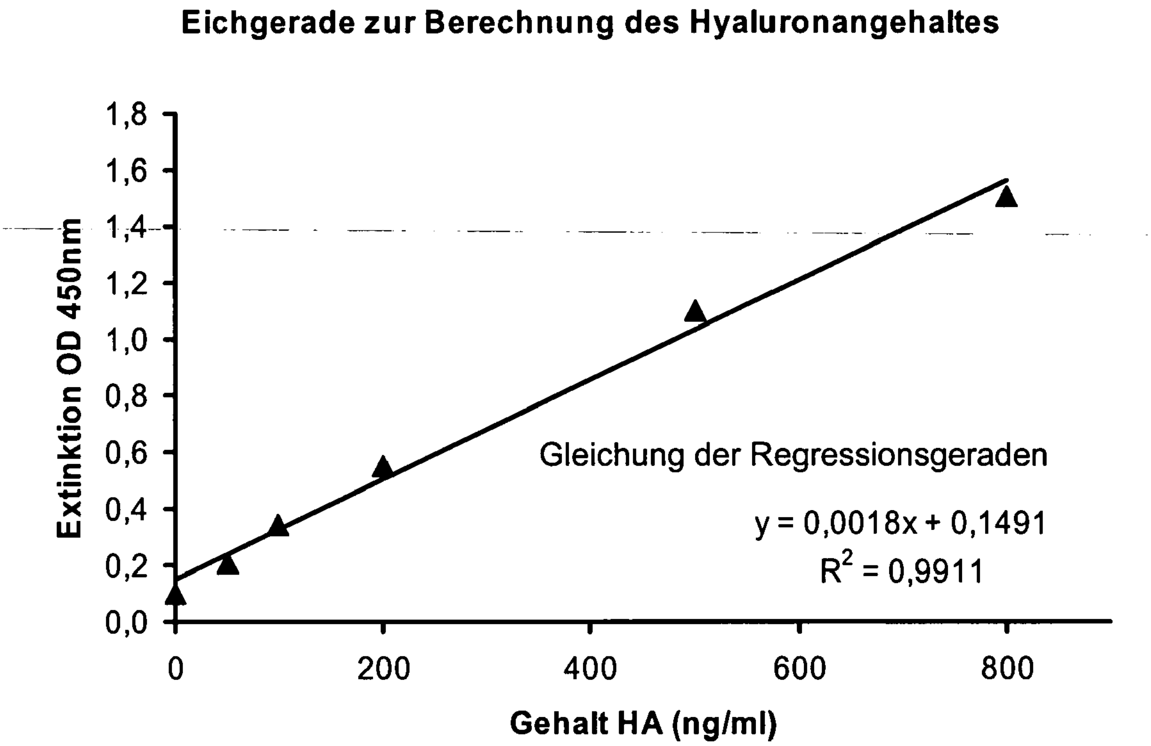
Eichgerade zur Berechnung des Hyaluronangehaltes
1,8
1,6
1,4
1,2
1,0
0,8
0,6
0,4
0,2
0,0
Extinktion OD 450nm
0
200
400
600
800
Gehalt HA (ng/ml)
Gleichung der Regressionsgeraden
y = 0,0018x + 0,1491
R2 = 0,9911

Fig. 1

Europäisches Patentamt

# EUROPÄISCHER RECHERCHENBERICHT

Nummer der Anmeldung
EP 05 09 0277

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,A | WO 2005/012529 A (TOYOBO RESEARCH CENTER CO., LTD; SHIBATANI, SHIGEO; MISAWA, SHUHEI; IH) 10. Februar 2005 (2005-02-10) ----- | | A01H5/00<br>A01H5/08<br>C21N15/82<br>C12N5/14<br>C21N9/04<br>C21N9/06 |
| A | US 2005/202540 A1 (WEIGEL PAUL H ET AL) 15. September 2005 (2005-09-15) ----- | | |
| A,D | GRAVES M V ET AL: "Hyaluronan Synthesis in Virus PBCV-1-Infected Chlorella-like Green Algae" VIROLOGY, ACADEMIC PRESS,ORLANDO, US, Bd. 257, Nr. 1, 25. April 1999 (1999-04-25), Seiten 15-23, XP004440100 ISSN: 0042-6822 ----- | | |
| A,D | SAMAC DEBORAH A ET AL: "Expression of UDP-glucose dehydrogenase reduces cell-wall polysaccharide concentration and increases xylose content in alfalfa stems" APPLIED BIOCHEMISTRY AND BIOTECHNOLOGY, Bd. 113-116, Nr. Spring, April 2004 (2004-04), Seiten 1167-1182, XP009061526 ISSN: 0273-2289 ----- | | RECHERCHIERTE SACHGEBIETE (IPC)<br>C12N<br>A01H |
| A,D | WO 00/11192 A (PIONEER HI-BRED INTERNATIONAL, INC) 2. März 2000 (2000-03-02) ----- | | |

Der vorliegende Recherchenbericht wurde für alle Patentansprüche erstellt

| Recherchenort | Abschlußdatum der Recherche | Prüfer |
|---|---|---|
| München | 15. März 2006 | Puonti-Kaerlas, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 05 09 0277

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-03-2006

| Im Recherchenbericht angeführtes Patentdokument | Datum der Veröffentlichung | Mitglied(er) der Patentfamilie | Datum der Veröffentlichung |
|---|---|---|---|
| WO 2005012529 A | 10-02-2005 | KEINE | |
| US 2005202540 A1 | 15-09-2005 | KEINE | |
| WO 0011192 A | 02-03-2000 | AU 6018399 A | 14-03-2000 |
| | | CA 2341078 A1 | 02-03-2000 |
| | | EP 1108040 A2 | 20-06-2001 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4141973 A
- US 4801539 A
- EP 0694616 A
- US 4782046 A
- US 20030175902 A
- WO 03054163 A
- WO 05012529 A
- WO 9835047 A
- WO 0011192 A
- US 4897349 A
- EP 0144019 A
- US 20030134393 A
- US 20030235893 A
- US 6455304 B
- EP 120516 A
- US 5565347 A
- WO 9506128 A
- EP 0513849 A
- EP 0465875 A
- EP 0292435 A
- US 5019498 A
- US 6444878 B
- WO 04003175 A
- WO 0173087 A
- WO 9307279 A
- US 6607745 B


### In der Beschreibung aufgeführte Nicht-Patentliteratur


- **LAPCIK et al.** *Chemical Reviews,* 1998, vol. 98 (8), 2663-2684
- **BERNHARD GEBAUER.** Inaugural-Dissertation. 1998
- **FRASER et al.** *Journal of Internal Medicine,* 1997, vol. 242, 27-33
- **VOLPI ; MACCARI.** *Biochimie,* 2003, vol. 85, 619-625
- **GRAVES et al.** *Virology,* 1999, vol. 257, 15-23
- **DEANGELIS.** *Science,* 1997, vol. 278, 1800-1803
- **DEANGELIS et al.** *Science,* 1997, vol. 278, 1800-1803
- **LANDSTEIN et al.** *Virology,* 1998, vol. 250, 388-396
- **DEANGELIS.** *CMLS, Cellular and Molecular Life Sciences,* 1999, vol. 56, 670-682
- **OUSKOVA et al.** *Glycobiology,* 2004, vol. 14 (10), 931-938
- **PREHM ; SCHUMACHER.** *Biochemical Pharmacology,* 2004, vol. 68, 1401-1410
- **GOA ; BENFIELD.** *Drugs,* 1994, vol. 47 (3), 536-566
- **TURLEY.** *Adv Drug Delivery Rev,* 1991, vol. 7, 257
- **LAURENT ; FRASER.** *FASEB J.,* 1992, vol. 6, 183
- **STAMENKOVIC ; ARUFFO.** *Methods Enzymol.,* 1993, vol. 245, 195
- **KNUDSON ; KNUDSON.** *FASEB,* 1993, vol. 7, 1233
- **KILIAN, M.** Medical Microbiology. Churchill Livingstone, 2002, 174-188
- **LORENCE et al.** *Plant Physiol,* 2004, vol. 134, 1200-1205
- **REITER.** *Plant Physiol Biochem,* 1998, vol. 36 (1), 167-176
- **H. W. HELDT.** Plant Biochemistry. Academic Press, 2004
- **SEITZ et al.** *Plant Journal,* 2000, vol. 21 (6), 537-546
- **KÄRKÖNEN.** *Plant Biosystems,* 2005, vol. 139 (1), 46-49
- **SAMAC et al.** Applied Biochemistry and Biotechnology. Humana Press, 2004, vol. 113-116, 1167-1182
- Studies on The Role of UDP-Glc-DH in Polysaccharide Biosynthesis. **ROMAN.** Dissertation, Acta Universitatis Upsaliensis. 2004
- **MAYER et al.** *Plant Physiol.,* 1968, vol. 43, 1097-1107
- **DICK et al.** *Eur J Opthalmol.,* 2003, vol. 13 (2), 176-184
- **ITANO et al.** *J. Biol. Chem.,* 2004, vol. 279 (18), 18679-18678
- **HU et al.** *J. Biol. Chem.,* 2004, vol. 279 (29), 29988-29993
- **HUBER ; HARDIN.** *Current Opinion in Plant Biotechnology,* 2004, vol. 7, 318-322

- Bioanalytik, Spektrum akad. Verlag, 1998
- Transgenic Plants. Humana Press, 2004
- **HOEKEMA, IN.** The Binary Plant Vector System Offsetdrukkerij. 1985
- **FRALEY et al.** *Crit. Rev. Plant Sci.,* vol. 4, 1-46
- **BEI AN et al.** *EMBO J.,* 1985, vol. 4, 277-287
- **ROCHA-SOSA et al.** *EMBO J.,* 1989, vol. 8, 29-33
- **CHAN et al.** *Plant Mol. Biol.,* 1993, vol. 22, 491-506
- **HIEI et al.** *Plant J.,* 1994, vol. 6, 271-282
- **DENG et al.** *Science,* 1990, vol. 33, 28-34
- **WILMINK et al.** *Plant Cell Reports,* 1992, vol. 11, 76-80
- **MAY et al.** *Bio/Technology,* 1995, vol. 13, 486-492
- **CONNER ; DOMISSE.** *Int. J. Plant Sci.,* 1992, vol. 153, 550-555
- **RITCHIE et al.** *Transgenic Res.,* 1993, vol. 2, 252-265
- **WAN ; LEMAUX.** *Plant Physiol.,* 1994, vol. 104, 37-48
- **VASIL et al.** *Bio/Technology,* 1993, vol. 11, 1553-1558
- **RITALA et al.** *Plant Mol. Biol.,* 1994, vol. 24, 317-325
- **SPENCER et al.** *Theor. Appl. Genet.,* 1990, vol. 79, 625-631
- **FROMM et al.** *Biotechnology,* 1990, vol. 8, 833-844
- **GORDON-KAMM et al.** *Plant Cell,* 1990, vol. 2, 603-618
- **KOZIEL et al.** *Biotechnology,* 1993, vol. 11, 194-200
- **MOROC et al.** *Theor. Appl. Genet.,* 1990, vol. 80, 721-726
- **RICHARDS et al.** *Plant Cell Reporters,* 2001, vol. 20, 48-54
- **KRENS et al.** *Nature,* 1982, vol. 296, 72-74
- **NEHRA et al.** *Plant J.,* 1994, vol. 5, 285-297
- **BECKER et al.** *Plant Journal,* 1994, vol. 5, 299-307
- **VAN ETTEN ; MEINTS.** *Annu. Rev. Microbiol.,* 1999, vol. 53, 447-494
- **VAN ETTEN et al.** *Arch Virol,* 2002, vol. 147, 1479-1516
- **CAMPBELL et al.** *J. Biol. Chem.,* 1997, vol. 272 (6), 3416-3422
- **SCHILLER et al.** *Biochim. Biophys Acta,* 1973, vol. 293 (1), 1-10
- **BALDUINI et al.** *Biochem. J.,* 1970, vol. 120 (4), 719-724
- **HINTERBERG.** *Plant Physiol. Biochem.,* 2002, vol. 40, 1011-1017
- **ORDMAN ; KIRKWOOD.** *Biochim Biophys Acta,* 1977, vol. 482 (1), 25-32
- **TURNER ; BOTHA.** *Archives of Biochem. Biophys.,* 2002, vol. 407, 209-216
- **ROBERTS et al.** *Plant Physiol.,* 1971, vol. 48, 36-42
- **KORNFELD.** *J. Biol. Chem.,* 1967, vol. 242 (13), 3135-3141
- **GRAACK et al.** *Biochem. J.,* 2001, vol. 360, 401-412
- **DENG et al.** *Metabolic Engeneering,* 2005, vol. 7, 201-214
- **STRASBURGER.** Lehrbuch der Botanik. Spektrum Akad. Verl, 1999
- **SAMBROK et al.** Molecular Cloning, A Laboratory Manual. Cold Spring Harbour Laboratory Press, 2001
- **AUSUBEL et al.** Short Protocols in Molecular Biology. John Wiley & Sons, 2002
- **NAM et al.** *The Plant Cell,* 1989, vol. 1, 699-705
- **LEISTER ; DEAN.** *The Plant Journal,* 1993, vol. 4 (4), 745-750
- **CASTIGLIONI et al.** *Genetics,* 1998, vol. 149, 2039-2056
- **MEKSEM et al.** *Molecular Genetics and Genomics,* 2001, vol. 265, 207-214
- **MEYER et al.** *Molecular and General Genetics,* 1998, vol. 259, 150-160
- **KONIECZNY ; AUSUBEL.** *The Plant Journal,* 1993, vol. 4, 403-410
- **JARVIS et al.** *Plant Molecular Biology,* 1994, vol. 24, 685-687
- **BACHEM et al.** *The Plant Journal,* 1996, vol. 9 (5), 745-753
- **THORNEYCROFT et al.** *Journal of experimental Botany,* 2001, vol. 52 (361), 1593-1601
- **RAMACHANDRAN ; SUNDARESAN.** *Plant Physiology and Biochemistry,* 2001, vol. 39, 234-252
- **WALDEN et al.** *Plant J.,* 1991, 281-288
- **WALDEN et al.** *Plant Mol. Biol.,* 1994, vol. 26, 1521-1528
- **DUTKA-MALEN.** *Biochemie,* 1988, vol. 70 (2), 287-290
- **WATZELE et al.** *J. Biol. Chem.,* 1989, vol. 264, 8753-8758
- **OKI et al.** *Genomics,* 1999, vol. 57 (2), 227-34
- **SAMBROCK et al.** Molecular Cloning, A Laboratory Manual. Cold Spring Harbor Laboratory Press, 1989
- **SAMBROOK et al.** Molecular Cloning, A Laboratory Manual. Cold Spring Harbor Laboratory Press, 1989
- **DE LUCA et al.** *Connective Tissue Research,* 1976, vol. 4, 247-254
- **BAR-PELED.** *Biochem. J.,* 2004, vol. 381, 131-136
- **TURNER ; BOTHA.** *Archives Biochem. Biophys.,* 2002, vol. 407, 209-216

- **THOMPSON et al.** *Nucleic Acids Research,* 1994, vol. 22, 4673-4680
- **STAVOLONE et al.** *Plant Mol. Biol.,* 2003, vol. 53, 703-713
- **ROCHA-SOSA et al.** *EMBO J.,* 1989, vol. 8, 23-29
- **MONTGOMERY et al.** *Plant Cell,* 1993, vol. 5, 1049-1062
- **METHA et al.** *Nature Biotechnol.,* 2002, vol. 20 (6), 613-618
- **MOON ; CALLAHAN.** *J. Experimental Botany,* 2004, vol. 55 (402), 1519-1528
- **STOCKHAUS et al.** *Proc. Natl. Acad. Sci. USA,* 1987, vol. 84, 7943-7947
- **STOCKHAUS et al.** *EMBO J.,* 1989, vol. 8, 2445-2451
- **PEDERSEN et al.** *Cell,* 1982, vol. 29, 1015-1026
- **QUATROCCIO et al.** *Plant Mol. Biol.,* 1990, vol. 15, 81-93
- **LEISY et al.** *Plant Mol. Biol.,* 1990, vol. 14, 41-50
- **ZHENG et al.** *Plant J.,* 1993, vol. 4, 357-366
- **YOSHIHARA et al.** *FEBS Lett.,* 1996, vol. 383, 213-218
- **WERR et al.** *EMBO J.,* 1985, vol. 4, 1373-1380
- **FIEDLER et al.** *Plant Mol. Biol.,* 1993, vol. 22, 669-679
- **BÄUMLEIN et al.** *Mol. Gen. Genet.,* 1991, vol. 225, 459-467
- **MITRA et al.** *Biochem. Biophys Res Commun,* 1994, vol. 204 (1), 187-194
- **MITRA ; HIGGINS.** *Plant Mol Biol,* 1994, vol. 26 (1), 85-93
- **GIELEN et al.** *EMBO J.,* 1989, vol. 8, 23-29
- **CALLIS et al.** *Genes Devel.,* 1987, vol. 1, 1183-1200
- **LUEHRSEN ; WALBOT.** *Mol. Gen. Genet.,* 1991, vol. 225, 81-93
- **RETHMEIER et al.** *Plant Journal,* 1997, vol. 12 (4), 895-899
- **ROSE ; BELIAKOFF.** *Plant Physiol.,* 2000, vol. 122 (2), 535-542
- **VASIL et al.** *Plant Physiol.,* 1989, vol. 91, 1575-1579
- **XU et al.** *Science in China Series C,* 2003, vol. 46 (6), 561-569
- Plant Cell Culture Protocols. Humana Press, 1999
- **RACHEL et al.** *J. Bacteriol.,* 1996, vol. 178 (8), 2320-2327
- **SPICERL et al.** *J. Bacteriol.,* 1998, vol. 273 (39), 25117-25124
- **BEVAN.** *Nucl Acids Res,* 1984, vol. 12, 8711-8721
- **PIETRZAK et al.** *Nucleic Acids Res.,* 1986, vol. 14, 5858
- **HERRERA-ESTRELLA et al.** *Nature,* 1983, vol. 303, 209-213
- **GIELEN et al.** *EMBO Journal,* 1984, vol. 3, 835-846
- **HÖFGEN ; WILLMITZER.** *Plant Science,* 1990, vol. 66, 221-230
- **BECKER.** *Nucleic Acids Res.,* 1990, vol. 18, 203